# EUROPEAN PATENT APPLICATION

(11) **EP 1 001 025 A2**
(43) Date of publication of application: **17.05.2000**
(21) Application number: 99308262.7
(22) Date of filing: 20.10.1999
(51) Int. Cl.: C12N 15/12, C12N 15/62, C07K 14/285, A61K 39/07, G01N 33/68

(54) **Outer membrane proteins from actinobacillus pleuropneumoniae**

(30) Priority: 22.10.1998 US 105285
(71) Applicant: Pfizer Products Inc., Groton, Connecticut 06340 (US)
(72) Inventor: Ankenbauer, Robert Gerard, Pfizer Inc., Groton, Connecticut 06340 (US); Baarsch, Mary Jo, Pfizer Inc., Groton, Connecticut 06340 (US); Campos, Manuel, Pfizer Inc., Groton, Connecticut 06340 (US); Keich, Robin Lee, Pfizer Inc., Groton, Connecticut 06340 (US); Rosey, Everett Lee, Pfizer Inc., Groton, Connecticut 06340 (US); Warren-Stewart, Lynn Marie, Pfizer Inc., Groton, Connecticut 06340 (US); Suiter, Brian Thomas, Pfizer Inc., Groton, Connecticut 06340 (US)
(74) Representative: Simpson, Alison Elizabeth Fraser

(57) **Abstract**

The present invention is directed to five novel, low molecular weight proteins from *Actinobacillus pleuropneumoniae* (APP), which are capable of inducing, or contributing to the induction of, a protective immune response in swine against APP. The present invention is further directed to polynucleotide molecules having nucleotide sequences that encode the proteins, as well as vaccines comprising the proteins or polynucleotide molecules, and methods of making and using the same.

## Description

### 1. FIELD OF THE INVENTION

The present invention is in the field of animal health, and is directed to vaccines that protect swine against *Actinobacillus pleuropneumoniae.* More particularly, the present invention is directed to novel antigenic proteins shared by multiple serotypes of *A*. *pleuropneumoniae,* DNA molecules encoding the proteins, vaccines against APP comprising the proteins, and diagnostic reagents.

### 2. BACKGROUND OF THE INVENTION

*A*. *pleuropneumoniae* (hereinafter referred to as "APP") is a Gram negative coccobacillus recognized as one of the most important swine pneumonic pathogens (Shope, R.E., 1964, J. Exp. Med. 119:357-368; Sebunya, T.N.K. and Saunders, J.R., 1983, J. Am. Vet. Med. Assoc. 182:1331-1337). Twelve different serotypes have been recognized which vary in geographic distribution (Sebunya, T.N.K. and Saunders, J.R., 1983, above; Nielsen, R., 1985, Proc. Am. Assoc. Swine Pract. 18-22; Nielsen, R., 1986, Acta. Vet. Scand. 27:453-455). Immune responses to vaccination against APP have been mainly serotype-specific, suggesting that vaccine-induced immunity is directed to serotype-specific capsular antigens (MacInnes, J.I. and Rosendal, S., 1988, Can. Vet. J. 29:572-574; Fedorka-Cray, P.J., *et al.,* 1994, Comp. Cont. Educ. Pract. Vet. 16:117-125; Nielsen, R., 1979, Nord. Vet. Med. 31:407-413; Rosendal, S., *et al.,* 1986, Vet. Microbiol. 12:229-240).

In contrast, natural immunity to any one serotype seems to confer significant protection from disease caused by other serotypes, suggesting that natural exposure induces cross-reactive immunity to shared antigens (Sebunya, T.N.K. and Saunders, J.R., 1983, above; MacInnes, J.I. and Rosendal, S., 1988, above; Fedorka-Cray, P.J., *et al.,* 1994, above; Nielsen, R., 1979, above; and Rosendal, S., *et al.,* 1986, above).

Virulence factors that might contribute to cross-protection have been proposed as possible vaccine candidates, including exotoxins (Apx) (Nakai, T., *et al.,* 1983, Am. J. Vet. Res. 44:344-347; Frey, J., *et al.,* 1993, J. Gen. Microbiol. 139:1723-1728; Fedorka-Cray, P.J., *et al.,* 1993, Vet. Microbiol. 37:85-100); capsular antigens (Rosendal, S., *et al.,* 1986, above); outer-membrane proteins (OMP) (Denee, H. and Potter, A., 1989, Infect. Immune 57:798-804; Niven, D.F., *et al.,* 1989, Mol. Microbiol. 3:1083-1089; Gonzalez, G., *et al.,* 1990, Mol. Microbiol. 4:1173-1179; Gerlach, G.F., *et al.,* 1992, Infect. Immun. 60:3253-3261); and lipopolysaccharides (LPS) (Fenwick, B.W. and Osborn, B., 1986, Infect. Immun. 54:575-582). However, the patterns of cross-reactivity/cross-protection induced by such components do not cover all twelve APP serotypes. In addition, immunization with isolated individual components or combinations of individual components from APP have so far failed to confer protection from challenge with some heterologous serotypes (unpublished observations). Thus, it can be postulated that the cross-protective responses induced by natural infection are limited to specific serotype clusters.

Alternatively, it is possible that some of the antigens responsible for the cross-protection observed after natural infection have not yet been identified. Most studies regarding APP antigens have focused on the characterization of immunodominant antigens detected in convalescent serum using antibodies. Such an approach does not allow the identification of possible differences between the antibody specificities represented during primary versus secondary responses, nor the identification of dominant specificities at the infection site that are likely to be responsible for protection upon secondary encounter with the pathogen.

It is generally accepted that lymphocytes are educated during primary infections so that when there is secondary exposure to a pathogen the host is better able to prevent disease (MacKay, C.R., 1993, Adv. Immunol. 53:217-240). Memory cells responsible for this activity (antigen-experienced T and B lymphocytes) persist for long periods of time, and are capable of reactivation following an appropriate subsequent encounter with the antigen. In contrast to naive cells, they generally show a faster response time, specialized tissue localization, and more effective antigen recognition and effector functions (MacKay, C.R., 1993, above; Linton, P. and Klinman, N.R., 1992, Sem. Immun. 4:3-9; Meeusen, E.N.T., *et al.,* 1991, Eur. J. Immunol. 21:2269-2272).

During the generation of a secondary response, the frequency of precursor cells capable of responding to the particular antigen is higher than that present during the primary response. Trafficking patterns of memory cell subsets following secondary responses are also different from those of naive cells. Naive cells migrate relatively homogeneously to secondary lymphoid tissues, but they home poorly to non-lymphoid tissues. By contrast, memory cells display heterogeneous trafficking and, in some instances, migration has been shown to be restricted to certain secondary lymphoid tissues and non-lymphoid sites (MacKay, C.R., 1993, above; Gray, D., 1993, Ann. Rev. Immunol. 11:49-77; Picker, L.S., *et al.,* 1993, J. Immunol. 150:1122-1136). Studies in both rodents and sheep have indicated that lymphocytes from the gut preferentially migrate back to the gut, whereas cells draining from the skin or from lymph nodes preferentially migrate back to the skin or lymph nodes (Gray, D., 1993, above; Picker, L.S., *et al.,* 1993, above). Thus, upon secondary encounter with a pathogen, specific effector cells for cell-mediated immunity and antibody secretion can home to infection sites and local lymph nodes more effectively (Meeusen, E.N.T., *et al.,* 1991, above). As a result, infiltrating lymphocytes will rapidly proliferate and their specificities will predominate during early stages of re-infection.

Recovery of local B cells from tissues and draining lymph nodes early after re-infection has allowed some researchers to obtain antibodies with a narrower specificity range (Meeusen, E.N.T. and Brandon, M., 1994, J. Immunol. Meth. 172:71-76). Such antibodies have been successfully used to identify potential protective antigens to several pathogens (Meeusen, E.N.T. and Brandon, M., 1994, above; Meeusen, E.N.T. and Brandon, M., 1994, Eur. J. Immunol. 24:469-474; Bowles, V.M., *et al.,* 1995, Immunol. 84:669-674). The invention disclosed herein below is based on a modification of this approach, in which antibody-secreting cell (ASC) probes were recovered that were associated with local memory responses elicited after homologous and heterologous APP challenge. Antibodies obtained from bronchial lymph node (BLN) cultures after heterologous challenge recognized four previously unrecognized proteins present in all twelve APP serotypes. Partial amino acid sequences for each protein were obtained and used to generate PCR primers that allowed the identification of five novel APP proteins and polynucleotide molecules that encode them.

### 3. SUMMARY OF THE INVENTION

The present invention provides five novel, low molecular weight proteins isolated from APP, which are designated herein, respectively, as "Omp20," "OmpW," "Omp27," "OmpA1," and "OmpA2". These "APP proteins" and the polynucleotide molecules that encode them are useful either as antigenic components in a vaccine to protect swine against APP, or as diagnostic reagents to identify swine that are, or have been, infected with APP, or that have been vaccinated with a vaccine of the present invention.

The amino acid sequence of Omp20 is encoded by the Omp20-encoding ORF of plasmid pER416 which is present in host cells of strain Pz416 (ATCC 98926), and its deduced amino acid sequence is presented as SEQ ID NO:2, which comprises a signal sequence from amino acid residues 1 to 19. The amino acid sequence of OmpW is encoded by the OmpW-encoding ORF of plasmid pER418 which is present in host cells of strain Pz418 (ATCC 98928), and its deduced amino acid sequence is presented as SEQ ID NO:4, which comprises a signal sequence from amino acid residues 1 to 21. The amino acid sequence of Omp27 is encoded by the Omp27-encoding ORF of plasmid pER417 which is present in host cells of strain Pz417 (ATCC 98927), and its deduced amino acid sequence is presented as SEQ ID NO:6, which comprises a signal sequence from amino acid residues 1 to 27. The amino acid sequence of OmpA1 is encoded by the OmpA1-encoding ORF of plasmid pER419 which is present in host cells of strain Pz419 (ATCC 98929), and its deduced amino acid sequence is presented as SEQ ID NO:8, which comprises a signal sequence from amino acid residues 1 to 19. The amino acid sequence of OmpA2 is encoded by the OmpA2-encoding ORF of plasmid pER420 which is present in host cells of strain Pz420 (ATCC 98930), and its deduced amino acid sequence is presented as SEQ ID NO:10, which comprises a signal sequence from amino acid residues 1 to 19. Each of these APP proteins, in substantially purified form, is provided by the present invention.

The present invention further provides substantially purified polypeptides that are homologous to any of the aforementioned APP proteins of the present invention. The present invention further provides peptide fragments of any of the APP proteins or homologous polypeptides of the present invention. The present invention further provides fusion proteins comprising an APP protein, homologous polypeptide, or peptide fragment of the present invention joined to a carrier or fusion partner. The present invention further provides analogs and derivatives of an APP protein, homologous polypeptide, peptide fragment, or fusion protein of the present invention.

The present invention further provides an isolated polynucleotide molecule comprising a nucleotide sequence encoding the APP protein, Omp20, with or without signal sequence. In a preferred embodiment, the isolated Omp20-encoding polynucleotide molecule of the present invention comprises the nucleotide sequence of SEQ ID NO:1 from about nt 329 to about nt 790. In a more preferred embodiment, the isolated Omp20-encoding polynucleotide molecule of the present invention comprises the nucleotide sequence of SEQ ID NO:1 from about nt 272 to about nt 790.

The present invention further provides an isolated polynucleotide molecule comprising a nucleotide sequence encoding the APP protein, OmpW, with or without signal sequence. In a preferred embodiment, the isolated OmpW-encoding polynucleotide molecule of the present invention comprises the nucleotide sequence of SEQ ID NO:3 from about nt 439 to about nt 1023. In a more preferred embodiment, the isolated OmpW-encoding polynucleotide molecule of the present invention comprises the nucleotide sequence of SEQ ID NO:3 from about nt 376 to about nt 1023.

The present invention further provides an isolated polynucleotide molecule comprising a nucleotide sequence encoding the APP protein, Omp27, with or without signal sequence. In a preferred embodiment, the isolated Omp27-encoding polynucleotide molecule of the present invention comprises the nucleotide sequence of SEQ ID NO:5 from about nt 238 to about nt 933. In a more preferred embodiment, the isolated Omp27-encoding polynucleotide molecule of the present invention comprises the nucleotide sequence of SEQ ID NO:5 from about nt 157 to about nt 933.

The present invention further provides an isolated polynucleotide molecule comprising a nucleotide sequence encoding the APP protein, OmpA1, with or without signal sequence. In a preferred embodiment, the isolated OmpA1-encoding polynucleotide molecule of the present invention comprises the nucleotide sequence of SEQ ID NO:7 from about nt 671 to about nt 1708. In a more preferred embodiment, the isolated OmpA1-encoding polynucleotide molecule of the present invention comprises the nucleotide sequence of SEQ ID NO:7 from about nt 614 to about nt 1708.

The present invention further provides an isolated polynucleotide molecule comprising a nucleotide sequence encoding the APP protein, OmpA2, with or without signal sequence. In a preferred embodiment, the isolated OmpA2-encoding polynucleotide molecule of the present invention comprises the nucleotide sequence of SEQ ID NO:9 from about nt 254 to about nt 1306. In a more preferred embodiment, the isolated OmpA2-encoding polynucleotide molecule of the present invention comprises the nucleotide sequence of SEQ ID NO:9 from about nt 197 to about nt 1306.

The present invention further provides an isolated polynucleotide molecule that is homologous to any of the aforementioned polynucleotide molecules of the present invention. The present invention further provides an isolated polynucleotide molecule comprising a nucleotide sequence that encodes a polypeptide that is homologous to any of the APP proteins of the present invention. The present invention further provides an isolated polynucleotide molecule consisting of a nucleotide sequence that is a substantial portion of any of the aforementioned polynucleotide molecules of the present invention. In a non-limiting embodiment, the substantial portion of a polynucleotide molecule of the present invention encodes a peptide fragment of an APP protein or homologous polypeptide of the present invention. The present invention further provides a polynucleotide molecule comprising a nucleotide sequence that encodes a fusion protein comprising an APP protein, homologous polypeptide, or peptide fragment of the present invention joined to a carrier or fusion partner.

The present invention further provides oligonucleotide molecules that are useful as primers for amplifying any of the polynucleotide molecules of the present invention, or as diagnostic reagents. Specific though non-limiting embodiments of such oligonucleotide molecules include oligonucleotide molecules having nucleotide sequences selected from the group consisting of any of SEQ ID NOS:15-47 and 49-93.

The present invention further provides compositions and methods for cloning and expressing any of the polynucleotide molecules of the present invention, including recombinant cloning vectors and recombinant expression vectors comprising a polynucleotide molecule of the present invention, host cells transformed with any of said vectors, and cell lines derived therefrom.

The present invention further provides a recombinantly-expressed APP protein, homologous polypeptide, peptide fragment, or fusion protein encoded by a polynucleotide molecule of the present invention.

The present invention further provides a vaccine for protecting swine against APP, comprising an immunologically effective amount of one or more antigens of the present invention selected from the group consisting of an APP protein, homologous polypeptide, peptide fragment, fusion protein, analog, derivative, or polynucleotide molecule of the present invention capable of inducing, or contributing to the induction of, a protective response against APP in swine; and a veterinarily acceptable carrier or diluent. The vaccine of the present invention can further comprise an adjuvant or other immunomodulatory component. In a non-limiting embodiment, the vaccine of the present invention can be a combination vaccine for protecting swine against APP and, optionally, one or more other diseases or pathological conditions that can afflict swine, which combination vaccine has a first component comprising an immunologically effective amount of one or more antigens of the present invention selected from the group consisting of an APP protein, homologous polypeptide, peptide fragment, fusion protein, analog, derivative, or polynucleotide molecule of the present invention capable of inducing, or contributing to the induction of, a protective response against APP in swine; a second component comprising an immunologically effective amount of a different antigen capable of inducing, or contributing to the induction of, a protective response against a disease or pathological condition that can afflict swine; and a veterinarily acceptable carrier or diluent.

The present invention further provides a method of preparing a vaccine that can protect swine against APP, comprising combining an immunologically effective amount of one or more antigens of the present invention selected from the group consisting of an APP protein, homologous polypeptide, peptide fragment, fusion protein, analog, derivative, or polynucleotide molecule of the present invention capable of inducing, or contributing to the induction of, a protective response against APP in swine, with a veterinarily acceptable carrier or diluent, in a form suitable for administration to swine.

The present invention further provides a method of vaccinating swine against APP, comprising administering a vaccine of the present invention to a pig.

The present invention further provides a vaccine kit for vaccinating swine against APP, comprising a container comprising an immunologically effective amount of one or more antigens of the present invention selected from the group consisting of an APP protein, homologous polypeptide, peptide fragment, fusion protein, analog, derivative, or polynucleotide molecule of the present invention capable of inducing, or contributing to the induction of, a protective response against APP in swine. The kit can further comprise a second container comprising a veterinarily acceptable carrier or diluent.

The present invention further provides antibodies that specifically bind to an APP protein of the present invention.

The present invention further provides diagnostic kits. In a non-limiting embodiment, the diagnostic kit comprises a first container comprising an APP protein, homologous polypeptide, peptide fragment, fusion protein, analog, or derivative of the present invention that will specifically bind to porcine antibodies directed against an APP protein; and a second container comprising a secondary antibody directed against the porcine anti-APP antibodies. The secondary antibody preferably comprises a detectable label. Such a diagnostic kit is useful to detect pigs that currently are, or have previously been, infected with APP, or that have seroconverted as a result of vaccination with a vaccine of the present invention. In a different non-limiting embodiment, the diagnostic kit comprises a first container comprising a primary antibody that binds to an APP protein of the present invention; and a second container comprising a secondary antibody that binds to a different epitope on the APP protein, or that binds to the primary antibody. The secondary antibody preferably comprises a detectable label. In a different non-limiting embodiment, the diagnostic kit comprises a container comprising a polynucleotide molecule or oligonucleotide molecule of the present invention that is useful to specifically amplify an APP-specific polynucleotide molecule of the present invention. These latter two diagnostic kits are useful to detect pigs that are currently infected with APP.

### 4. BRIEF DESCRIPTION OF THE DRAWINGS

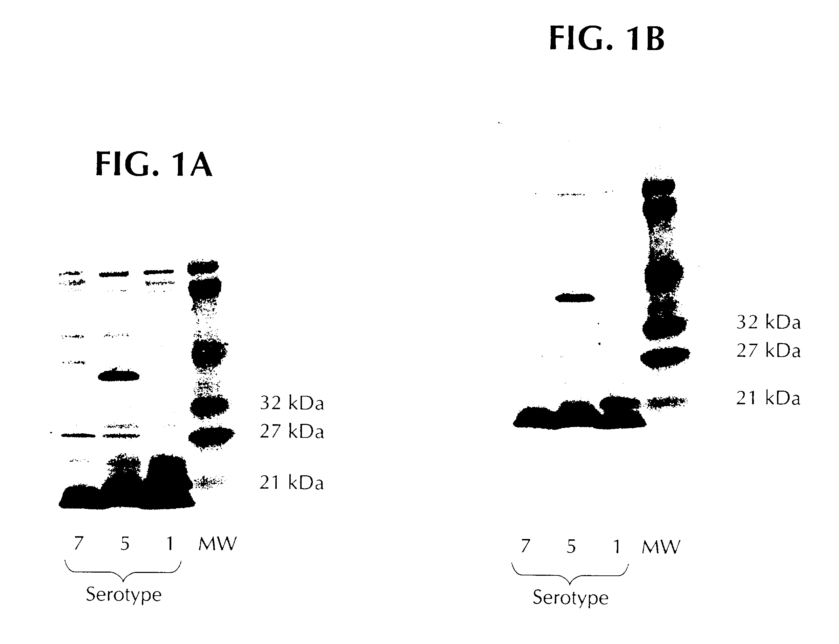

FIGURE 1. Western blot analysis of antibodies present in (a) serum, and (b) bronchial lymph node (BLN) tissue explant supernatants from pig No. 803 challenged with APP serotype5 and heterologously rechallenged with APP serotype-7. All BLN tissue explant supernatants collected after 24 or 48 hr of incubation contained antibodies that specifically recognized APP proteins. The antibodies from the BLN tissue explant supernatants highlighted several low molecular weight proteins present in APP serotypes-1, 5, and 7.

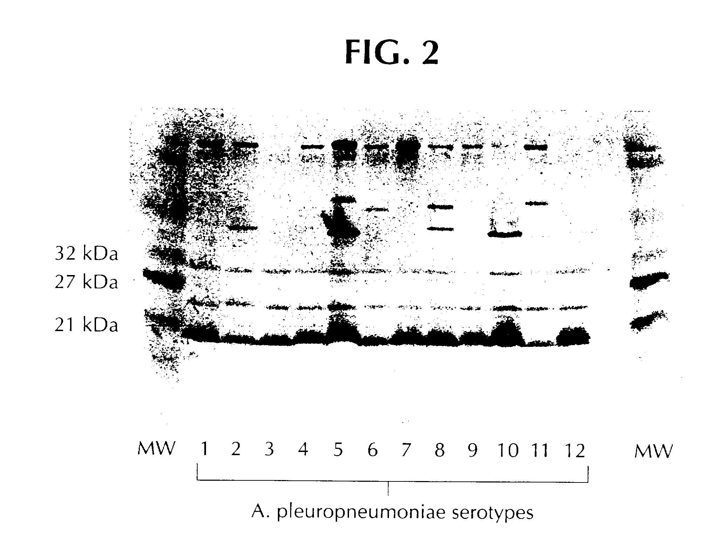

FIGURE 2. Western blot analysis of cross-reactivity of antibodies present in BLN tissue explant supernatants from pig No. 803 against whole bacterial cell antigens from each of the twelve different APP serotypes, demonstrating that at least three of the low molecular weight proteins recognized by antibodies induced by heterologous rechallenge were present in all twelve APP serotypes. Antibodies present in this particular BLN supernatant also recognized other protein bands.

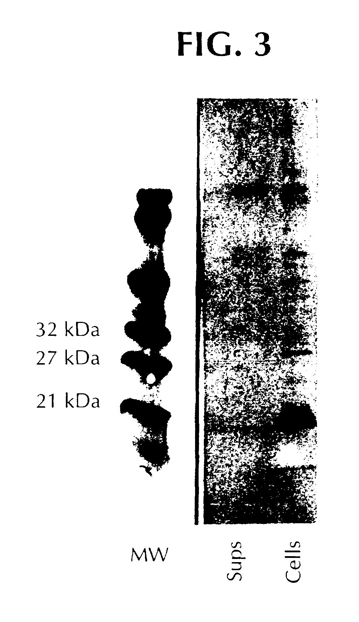

FIGURE 3. Western blot analysis demonstrating that reactivity of antibodies in BLN tissue explant supernatants from pig No. 803 against the low molecular weight proteins is restricted to proteins present in the cell pellets (cells) rather than bacterial cell supernatants (Sups).

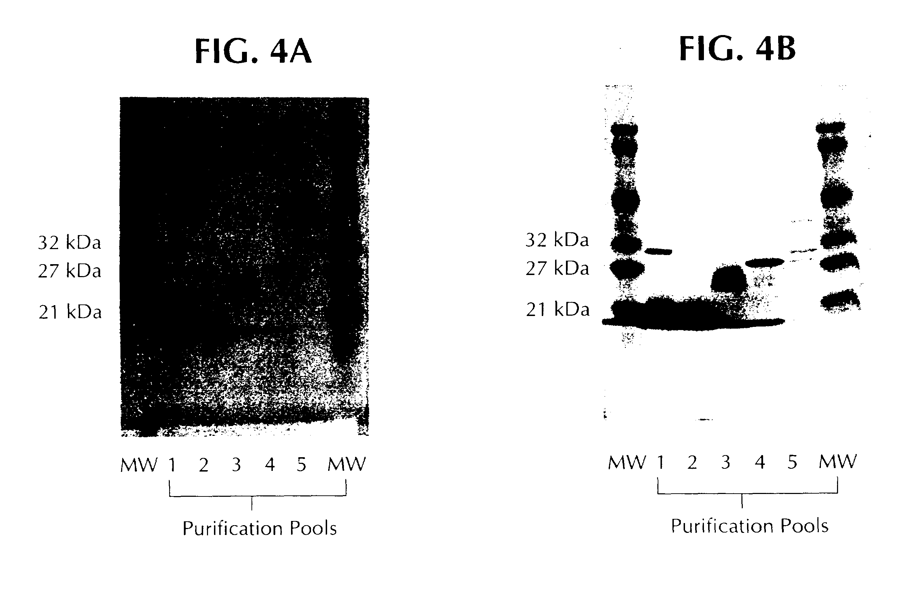

FIGURE 4. Western blot analysis of reactivity of (a) BLN tissue explant supernatant and (b) serum from pig No. 803, against proteins purified from APP serotype-7 by continuous flow electrophoresis. Four protein bands with molecular weights of about 19-20, about 23, about 27, and about 29 kDa, respectively, were identified using this procedure.

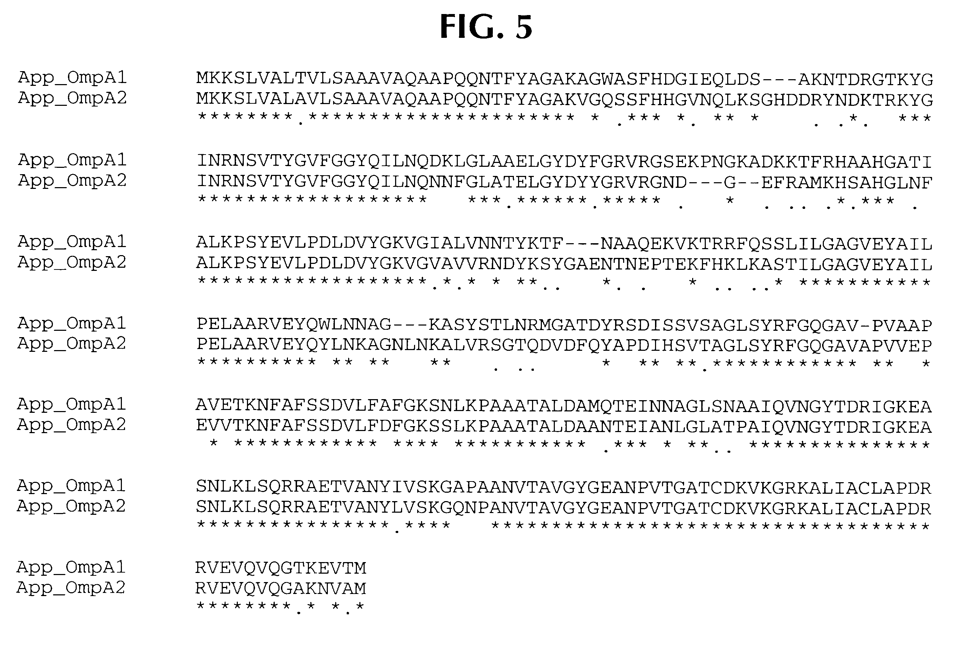

FIGURE 5. Alignment of deduced amino acid sequences of APP OmpA1 and APP OmpA2 proteins. The two proteins share 73.1% amino acid identity.

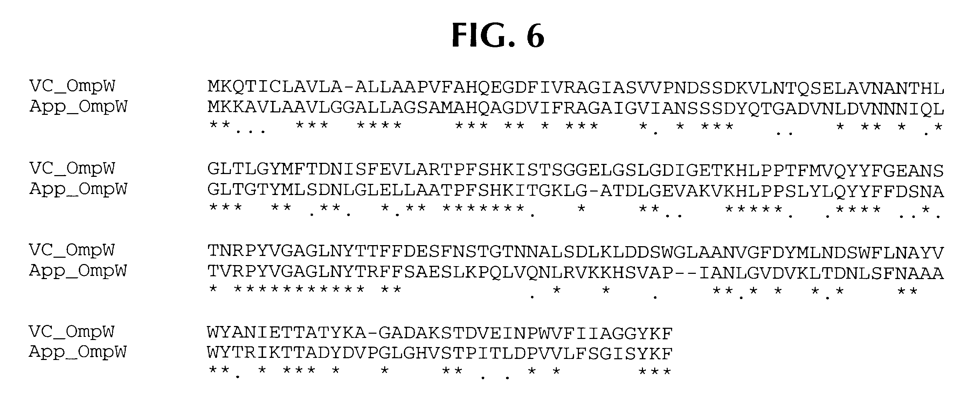

FIGURE 6. Alignment of OmpW protein from *Vibrio cholerae* and OmpW protein from APP. The two proteins share 44.9% amino acid identity.

### 5. DETAILED DESCRIPTION OF THE INVENTION

### 5.1. Novel Proteins Shared By Multiple APP Serotypes

The present invention is based on the discovery of five novel, low molecular weight proteins from APP (referred to hereinafter as "APP proteins"). These APP proteins are designated herein, respectively, as "Omp20," "OmpW," "Omp27," "OmpA1," and "OmpA2."

The amino acid sequence of Omp20 is encoded by the Omp20-encoding ORF of plasmid pER416 which is present in host cells of strain Pz416 (ATCC 98926). The deduced amino acid sequence of Omp20 is presented as SEQ ID NO:2. The first 19 amino acids of the protein shown in SEQ ID NO:2 represent a signal sequence, and the present invention encompasses both an Omp20 protein having only amino acid residues 20 to 172 of SEQ ID NO:2 (*i.e.,* lacking the signal sequence), and an Omp20 protein having the sequence of SEQ ID NO:2 (*i.e.,* including the signal sequence). The present invention thus provides a substantially purified protein comprising the amino acid sequence of about amino acid residue 20 to about amino acid residue 172 of SEQ ID NO:2. The present invention further provides a substantially purified protein comprising the amino acid sequence of SEQ ID NO:2.

The amino acid sequence of OmpW is encoded by the OmpW-encoding ORF of plasmid pER418 which is present in host cells of strain Pz418 (ATCC 98928). The deduced amino acid sequence of OmpW is presented as SEQ ID NO:4. The first 21 amino acids of the protein shown in SEQ ID NO:4 represent a signal sequence, and the present invention encompasses both an OmpW protein having only amino acid residues 22 to 215 of SEQ ID NO:4 (*i.e.,* lacking the signal sequence), and an OmpW protein having the sequence of SEQ ID NO:4 (*i.e.,* including the signal sequence). The present invention thus provides a substantially purified protein comprising the amino acid sequence of about amino acid residue 22 to about amino acid residue 215 of SEQ ID NO:4. The present invention further provides a substantially purified protein comprising the amino acid sequence of SEQ ID NO:4.

The amino acid sequence of Omp27 is encoded by the Omp27-encoding ORF of plasmid pER417 which is present in host cells of strain Pz417 (ATCC 98927). The deduced amino acid sequence of Omp27 is presented as SEQ ID NO:6. The first 27 amino acids of the protein shown in SEQ ID NO:6 represent a signal sequence, and the present invention encompasses both an Omp27 protein having only amino acid residues 28 to 258 of SEQ ID NO:6 (*i.e.,* lacking the signal sequence), and an Omp27 protein having the sequence of SEQ ID NO:6 (*i.e.,* including the signal sequence). The present invention thus provides a substantially purified protein comprising the amino acid sequence of about amino acid residue 28 to about amino acid residue 258 of SEQ ID NO:6. The present invention further provides a substantially purified protein comprising the amino acid sequence of SEQ ID NO:6.

The amino acid sequence of OmpA1 is encoded by the OmpA1-encoding ORF of plasmid pER419 which is present in host cells of strain Pz419 (ATCC 98929). The deduced amino acid sequence of OmpA1 is presented as SEQ ID NO:8. The first 19 amino acids of the protein shown in SEQ ID NO:8 represent a signal sequence, and the present invention encompasses both an OmpA1 protein having only amino acid residues 20 to 364 of SEQ ID NO:8 (*i.e.,* lacking the signal sequence), and an OmpA1 protein having the sequence of SEQ ID NO:8 (*i.e.*, including the signal sequence). The present invention thus provides a substantially purified protein comprising the amino acid sequence of about amino acid residue 20 to about amino acid residue 364 of SEQ ID NO:8. The present invention further provides a substantially purified protein comprising the amino acid sequence of SEQ ID NO:8.

The amino acid sequence of OmpA2 is encoded by the OmpA2-encoding ORF of plasmid pER420 which is present in host cells of strain Pz420 (ATCC 98930). The deduced amino acid sequence of OmpA2 is presented as SEQ ID NO:10. The first 19 amino acids of the protein shown in SEQ ID NO:10 represent a signal sequence, and the present invention encompasses both an OmpA2 protein having only amino acid residues 20 to 369 of SEQ ID NO:10 (*i.e.,* lacking the signal sequence), and an OmpA2 protein having the sequence of SEQ ID NO:10 (*i.e.,* including the signal sequence). The present invention thus provides a substantially purified protein comprising the amino acid sequence of about amino acid residue 20 to about amino acid residue 369 of SEQ ID NO:10. The present invention further provides a substantially purified protein comprising the amino acid sequence of SEQ ID NO:10.

The APP proteins of the present invention, *i.e.,* Omp20, OmpW, Omp27, OmpA1, and OmpA2, have molecular weights of about 19-20, about 23, about 27, about 29 and about 29 kDa, respectively, as based on their electrophoretic mobility; and about 20, about 23, about 27, about 35 and about 35 kDa, respectively, as based on their deduced amino acid sequences without signal sequences.

The present invention further provides polypeptides that are homologous to an APP protein of the present invention. As used herein to refer to polypeptides, the term "homologous" refers to a polypeptide otherwise having an amino acid sequence selected from the group of amino acid sequences consisting of SEQ ID NOS: 2, 4, 6, 8, and 10, or those same amino acid sequences but without their native signal sequences, in which one or more amino acid residues have been conservatively substituted with a different amino acid residue, wherein the homologous polypeptide has an amino acid sequence that has about 70%, more preferably about 80%, and most preferably about 90% sequence identity, as determined by any standard amino acid sequence analysis algorithm (such as one of the BlastP algorithms of GENBANK), to a polypeptide having an amino acid sequence selected from the group of amino acid sequences consisting of SEQ ID NOS: 2, 4, 6, 8, and 10, and wherein the resulting homologous polypeptide is useful in practicing the present invention. Conservative amino acid substitutions are well-known in the art. Rules for making such substitutions include those described by Dayhof, M.D., 1978, Nat. Biomed. Res. Found., Washington, D.C., Vol. 5, Sup. 3, among others. More specifically, conservative amino acid substitutions are those that generally take place within a family of amino acids that are related in acidity, polarity, or bulkiness of their side chains. Genetically encoded amino acids are generally divided into four groups: (1) acidic = aspartate, glutamate; (2) basic = lysine, arginine, histidine; (3) non-polar = alanine, valine, leucine, isoleucine, proline, phenylalanine, methionine, tryptophan; and (4) uncharged polar = glycine, asparagine, glutamine, cysteine, serine, threonine, tyrosine. Phenylalanine, tryptophan and tyrosine are also jointly classified as aromatic amino acids. One or more replacements within any particular group, *e.g.*, of a leucine with an isoleucine or valine, or of an aspartate with a glutamate, or of a threonine with a serine, or of any other amino acid residue with a structurally related amino acid residue, *e.g.*, an amino acid residue with similar acidity, polarity, bulkiness of side chain, or with similarity in some combination thereof, will generally have an insignificant effect on the function or immunogenicity of the polypeptide.

As used herein, a polypeptide is "useful in practicing the present invention" where the polypeptide: (a) is immunogenic, *i.e.,* can be used in a vaccine composition, either alone to induce a protective response in swine against APP, or in combination with other antigens of the present invention to contribute to the induction of a protective response in swine against APP; or (b) can be used to induce the production of APP-specific antibodies when administered to a member of a mammalian species, which antibodies are useful as diagnostic reagents; or (c) can be used as a diagnostic reagent to detect the presence of anti-APP antibodies in a blood or serum sample from a pig resulting either from infection with APP or vaccination with a vaccine of the present invention.

The present invention further provides peptide fragments of an APP protein or homologous polypeptide of the present invention. As used herein, a "peptide fragment" means a polypeptide consisting of less than the complete amino acid sequence of the corresponding full-length APP protein, either with or without signal sequence, or homologous polypeptide thereof, but comprising a sub-sequence of at least about 10, more preferably at least about 20, and most preferably at least about 30 amino acid residues of the amino acid sequence thereof, and that is useful in practicing the present invention, as usefulness is defined above for polypeptides. A peptide fragment of the present invention can comprise more than one sub-sequence of a full-length APP protein or homologous polypeptide of the present invention. For example, two or more different sub-sequences from the full-length APP protein or homologous polypeptide can be brought together and made contiguous to each other in the peptide fragment where they were non-contiguous in the APP protein or homologous polypeptide. In a preferred embodiment, a peptide fragment of the present invention comprises one or more sub-sequences representing one or more epitopes of the APP protein or homologous polypeptide, or multiple copies of an epitope, against which antibodies can be raised.

In a non-limiting embodiment, the present invention provides a peptide fragment of an APP protein of the present invention, which peptide fragment comprises the native signal sequence of the APP protein. In a preferred embodiment, the peptide fragment consists of an amino acid sequence selected from the group consisting of about amino acid residue 1 to about amino acid residue 19 of SEQ ID NO:2 (Omp20), about amino acid residue 1 to about amino acid residue 21 of SEQ ID NO:4 (OmpW), about amino acid residue 1 to about amino acid residue 27 of SEQ ID NO:6 (Omp27), about amino acid residue 1 to about amino acid residue 19 of SEQ ID NO:8 (OmpA1), and about amino acid residue 1 to about amino acid residue 19 of SEQ ID NO:10 (OmpA1). Such signal sequences, and the polynucleotide molecules that encode them, are useful for a variety of purposes, including to direct the cellular trafficking of recombinant proteins expressed in APP or other bacterial host cells, or as diagnostic probes for detecting an APP-specific polynucleotide molecule in a fluid or tissue sample from an infected animal.

The present invention further provides full-length APP proteins or homologous polypeptides in which sub-sequences thereof are arranged in a different relative order to each other compared to that found in the native molecule so as to increase, alter, or otherwise improve the antigenicity of the polypeptide.

As used herein, the terms "antigen," "antigenic," and the like, refer to a molecule containing one or more epitopes that stimulate a host's immune system to make a humoral and/or cellular antigen-specific response. The term is also used interchangeably with "immunogen." As used herein, the term "epitope" or "epitopic region" refers to a site on an antigen or hapten to which a specific antibody molecule binds. The term is also used interchangeably with "antigenic determinant."

The present invention further provides fusion proteins comprising an APP protein partner with or without its native signal sequence, a homologous polypeptide thereof, or a peptide fragment of the present invention, joined to a carrier or fusion partner, which fusion proteins are useful in practicing the present invention, as usefulness is defined above for polypeptides. See Section 5.4.1 below for examples of fusion partners. Fusion proteins are useful for a variety of reasons, including to increase the stability of recombinantly-expressed APP proteins, as antigenic components in an APP vaccine, to raise antisera against the particular APP protein partner, to study the biochemical properties of the APP protein partner, to engineer APP proteins with different or enhanced antigenic properties, to serve as diagnostic reagents, or to aid in the identification or purification of the expressed APP protein partner as described, *e.g.,* in Section 5.4.1 below.

Fusion proteins of the present invention can be further engineered using standard techniques to contain specific protease cleavage sites so that the particular APP protein partner can be released from the carrier or fusion partner by treatment with a specific protease. For example, a fusion protein of the present invention can comprise a thrombin or factor Xa cleavage site, among others.

The present invention further provides analogs and derivatives of an APP protein, homologous polypeptide, peptide fragment or fusion protein of the present invention, where such analogs and derivatives are useful in practicing the present invention, as usefulness is defined above for polypeptides. Manipulations that result in the production of analogs can be carried out either at the gene level or at the protein level, or both, to improve or otherwise alter the biological or immunological characteristics of the particular polypeptide from which the analog is prepared. For example, at the gene level, a cloned DNA molecule encoding an APP protein of the present invention can be modified by one or more known strategies to encode an analog of that protein. Such modifications include, but are not limited to, endonuclease digestion, and mutations that create or destroy translation, initiation or termination sequences, or that create variations in the coding region, or a combination thereof. Such techniques are described, among other places, in Maniatis *et al.,* 1989, Molecular Cloning, A Laboratory Manual, Cold Spring Harbor Laboratory Press, Cold Spring Harbor, NY; Ausubel *et al.,* 1989, Current Protocols In Molecular Biology, Greene Publishing Associates & Wiley Interscience, NY; Sambrook *et al.,* 1989, Molecular Cloning: A Laboratory Manual, 2d ed., Cold Spring Harbor Laboratory Press, Cold Spring Harbor, NY; Innis *et al.* (eds), 1995, PCR Strategies, Academic Press, Inc., San Diego; and Erlich (ed), 1992, PCR Technology, Oxford University Press, New York, all of which are incorporated herein by reference.

Alternatively or additionally, an analog of the present invention can be prepared by modification of an APP protein or other polypeptide of the present invention at the protein level. One or more chemical modifications of the protein can be carried out using known techniques, including but not limited to one or more of the following: substitution of one or more L-amino acids of the protein with corresponding D-amino acids, amino acid analogs, or amino acid mimics, so as to produce, *e.g.*, carbazates or tertiary centers; or specific chemical modification, such as proteolytic cleavage with, *e.g.*, trypsin, chymotrypsin, papain or V8 protease, or treatment with NaBH₄ or cyanogen bromide, or acetylation, formylation, oxidation or reduction, *etc.*

An APP protein or other polypeptide of the present invention can be derivatized by conjugation thereto of one or more chemical groups, including but not limited to acetyl groups, sulfur bridging groups, glycosyl groups, lipids, and phosphates, and/or a second APP protein or other polypeptide of the present invention, or another protein, such as, *e.g.,* serum albumin, keyhole limpet hemocyanin, or commercially activated BSA, or a polyamino acid (*e.g.,* polylysine), or a polysaccharide, (*e.g.,* sepharose, agarose, or modified or unmodified celluloses), among others. Such conjugation is preferably by covalent linkage at amino acid side chains and/or at the N-terminus or C-terminus of the APP protein. Methods for carrying out such conjugation reactions are well-known in the field of protein chemistry.

Derivatives useful in practicing the claimed invention also include those in which a water-soluble polymer, such as, *e.g.,* polyethylene glycol, is conjugated to an APP protein or other polypeptide of the present invention, or to an analog thereof, thereby providing additional desirable properties while retaining, at least in part, or improving the immunogenicity of the APP protein. These additional desirable properties include, *e.g.,* increased solubility in aqueous solutions, increased stability in storage, increased resistance to proteolytic degradation, and increased *in vivo* half-life. Water-soluble polymers suitable for conjugation to an APP protein or other polypeptide of the present invention include but are not limited to polyethylene glycol homopolymers, polypropylene glycol homopolymers, copolymers of ethylene glycol with propylene glycol, wherein said homopolymers and copolymers are unsubstituted or substituted at one end with an alkyl group, polyoxyethylated polyols, polyvinyl alcohol, polysaccharides, polyvinyl ethyl ethers, and α,β-poly[2-hydroxyethyl]-DL-aspartamide. Polyethylene glycol is particularly preferred. Methods for making water-soluble polymer conjugates of polypeptides are known in the art and are described in, among other places, U.S. Patent 3,788,948; U.S. Patent 3,960,830; U.S. Patent 4,002,531; U.S. Patent 4,055,635; U.S. Patent 4,179,337; U.S. Patent 4,261,973; U.S. Patent 4,412,989; U.S. Patent 4,414,147; U.S. Patent 4,415,665; U.S. Patent 4,609,546; U.S. Patent 4,732,863; U.S. Patent 4,745,180; European Patent (EP) 152,847; EP 98,110; and Japanese Patent (JP) 5,792,435, which patents are incorporated herein by reference.

All subsequent references to "APP proteins" and the like are intended to include the APP proteins, homologous polypeptides, peptide fragments, fusion proteins, analogs, and derivatives of the present invention, as these terms are defined above, unless otherwise indicated.

### 5.2. Polynucleotide Molecules Encoding Novel APP Proteins

The present invention further provides isolated polynucleotide molecules comprising a nucleotide sequence encoding an APP protein. As used herein, the terms "polynucleotide molecule," "polynucleotide sequence," "coding sequence," "open-reading frame (ORF)," and the like, are intended to refer to both DNA and RNA molecules, which can either be single-stranded or double-stranded; that can include one or more prokaryotic sequences, cDNA sequences, genomic DNA sequences (including exons and introns), or chemically synthesized DNA and RNA sequences; and that can include both sense and anti-sense strands. As used herein, the term "ORF" refers to the minimal nucleotide sequence required to encode a particular APP protein of the present invention without any intervening termination codons. The boundaries of the polynucleotide coding sequence are generally determined by the presence of a start codon at the 5' (amino) terminus and a translation stop codon at the 3' (carboxy) terminus.

Production and manipulation of the polynucleotide molecules and oligonucleotide molecules disclosed herein below are within the skill in the art and can be carried out according to recombinant techniques described, among other places, in Maniatis *et al.,* 1989, above; Ausubel *et al.,* 1989, above; Sambrook *et al.,* 1989, above; Innis *et al.,* 1995, above; and Erlich, 1992, above.

### 5.2.1. Polynucleotide Molecules Encoding Omp20

References herein below to nucleotide sequences from SEQ ID NO:1, and to selected and substantial portions thereof, are intended to also refer to the corresponding Omp20-related nucleotide sequences of plasmid pER416 which is present in host cells of strain Pz416 (ATCC 98926), unless otherwise indicated. In addition, references herein below to the amino acid sequences shown in SEQ ID NO:2, and to peptide fragments thereof, are intended to also refer to the corresponding amino acid sequences encoded by the Omp20-related nucleotide sequence of plasmid pER416, unless otherwise indicated.

The present invention provides an isolated polynucleotide molecule comprising a nucleotide sequence encoding the APP protein, Omp20, with or without signal sequence. In a preferred embodiment, the isolated Omp20-encoding polynucleotide molecule of the present invention comprises the nucleotide sequence of SEQ ID NO:1 from about nt 329 to about nt 790. In a more preferred embodiment, the isolated Omp20-encoding polynucleotide molecule of the present invention comprises the nucleotide sequence of SEQ ID NO:1 from about nt 272 to about nt 790. In a non-limiting embodiment, the isolated Omp20-encoding polynucleotide molecule of the present invention comprises the nucleotide sequence of SEQ ID NO:1.

The present invention further provides an isolated polynucleotide molecule that is homologous to an APP Omp20-encoding polynucleotide molecule of the present invention. The term "homologous" when used to refer to an Omp20-encoding polynucleotide molecule means a polynucleotide molecule having a nucleotide sequence: (a) that encodes the same amino acid sequence as the nucleotide sequence of SEQ ID NO:1 from nt 329 to nt 790, but that includes one or more silent changes to the nucleotide sequence according to the degeneracy of the genetic code; or (b) that hybridizes to the complement of a polynucleotide molecule having a nucleotide sequence that encodes amino acid residues 20 to 172 of SEQ ID NO:2 under moderately stringent conditions, *i.e.,* hybridization to filter-bound DNA in 0.5 M NaHPO₄, 7% sodium dodecyl sulfate (SDS), 1 mM EDTA at 65°C, and washing in 0.2xSSC/0.1% SDS at 42°C (see Ausubel *et al.* (eds.), 1989, Current Protocols in Molecular Biology, Vol. I, Green Publishing Associates, Inc., and John Wiley & Sons, Inc., New York, at p. 2.10.3), and that is useful in practicing the present invention. In a preferred embodiment, the homologous polynucleotide molecule hybridizes to the complement of a polynucleotide molecule having a nucleotide sequence that encodes amino acid residues 20 to 172 of SEQ ID NO:2 under highly stringent conditions, *i.e.,* hybridization to filter-bound DNA in 0.5 M NaHPO₄, 7% SDS, 1 mM EDTA at 65°C, and washing in 0.1xSSC/0.1% SDS at 68°C (Ausubel *et al.,* 1989, above), and is useful in practicing the present invention. In a more preferred embodiment, the homologous polynucleotide molecule hybridizes under highly stringent conditions to the complement of a polynucleotide molecule consisting of the nucleotide sequence of SEQ ID NO:1 from nt 329 to nt 790, and is useful in practicing the present invention.

As used herein, a polynucleotide molecule is "useful in practicing the present invention" where: (a) the polynucleotide molecule encodes a polypeptide which can be used in a vaccine composition either to induce by itself, or to contribute in combination with one or more other antigens to the induction of, a protective response in swine against APP; or (b) the polynucleotide molecule can be used directly in a DNA vaccine composition to induce by itself, or to contribute in combination with one or more other polynucleotide molecules or one or more other antigens to the induction of, a protective response in swine against APP; or (c) the polynucleotide molecule encodes a polypeptide that can be used to induce the production of APP-specific antibodies when administered to a member of a mammalian species, which antibodies are useful as diagnostic reagents; or (d) the polynucleotide molecule encodes a polypeptide that can be used as a diagnostic reagent to detect the presence of APP-specific antibodies in a blood or serum sample from a pig; or (e) the polynucleotide molecule can be used as a diagnostic reagent to detect the presence of an APP-specific polynucleotide molecule in a fluid or tissue sample from an APP-infected pig.

The present invention further provides an isolated polynucleotide molecule comprising a nucleotide sequence that encodes a polypeptide that is homologous to the Omp20 protein of the present invention, as "homologous polypeptides" are defined above in Section 5.1.

The present invention further provides a polynucleotide molecule consisting of a nucleotide sequence that is a substantial portion of any of the aforementioned Omp20-related polynucleotide molecules of the present invention. As used herein, a "substantial portion" of an Omp20-related polynucleotide molecules means a polynucleotide molecule consisting of less than the complete nucleotide sequence of the particular full-length Omp20-related polynucleotide molecule, but comprising at least about 10%, and more preferably at least about 20%, of the nucleotide sequence of the particular full-length Omp20-related polynucleotide molecule, and that is useful in practicing the present invention, as "usefulness" is defined above for polynucleotide molecules. In a non-limiting embodiment, the substantial portion of the Omp20-related polynucleotide molecule encodes a peptide fragment of any of the aforementioned Omp20-related proteins or polypeptides of the present invention, as the term "peptide fragment" is defined above.

The present invention further provides a polynucleotide molecule comprising a nucleotide sequence which encodes the native Omp20 signal sequence from about amino acid residue 1 to about amino acid residue 19 of SEQ ID NO:2. In a preferred though non-limiting embodiment, the Omp20 signal sequence-encoding polynucleotide molecule comprises from about nt 272 to about nt 328 of SEQ ID NO:1.

The present invention further provides a polynucleotide molecule comprising a nucleotide sequence that encodes a fusion protein comprising the Omp20 protein, homologous polypeptide, or peptide fragment, fused to a carrier or fusion partner.

### 5.2.2. Polynucleotide Molecules Encoding OmpW

References herein below to nucleotide sequences from SEQ ID NO:3, and to selected and substantial portions thereof, are intended to also refer to the corresponding OmpW-related nucleotide sequences of plasmid pER418 which is present in host cells of strain Pz418 (ATCC 98928), unless otherwise indicated. In addition, references herein below to the amino acid sequences shown in SEQ ID NO:4, and to peptide fragments thereof, are intended to also refer to the corresponding amino acid sequences encoded by the OmpW-related nucleotide sequence of plasmid pER418, unless otherwise indicated.

The present invention provides an isolated polynucleotide molecule comprising a nucleotide sequence encoding the APP protein, OmpW, with or without signal sequence. In a preferred embodiment, the isolated OmpW-encoding polynucleotide molecule of the present invention comprises the nucleotide sequence of SEQ ID NO:3 from about nt 439 to about nt 1023. In a more preferred embodiment, the isolated OmpW-encoding polynucleotide molecule of the present invention comprises the nucleotide sequence of SEQ ID NO:3 from about nt 376 to about nt 1023. In a non-limiting embodiment, the isolated OmpW-encoding polynucleotide molecule of the present invention comprises the nucleotide sequence of SEQ ID NO:3.

The present invention further provides an isolated polynucleotide molecule that is homologous to an APP OmpW-encoding polynucleotide molecule of the present invention. The term "homologous" when used to refer to an OmpW-encoding polynucleotide molecule means a polynucleotide molecule having a nucleotide sequence: (a) that encodes the same amino acid sequence as the nucleotide sequence of SEQ ID NO:3 from nt 439 to nt 1023, but that includes one or more silent changes to the nucleotide sequence according to the degeneracy of the genetic code; or (b) that hybridizes to the complement of a polynucleotide molecule having a nucleotide sequence that encodes amino acid residues 22 to 215 of SEQ ID NO:4 under moderately stringent conditions, *i.e.,* hybridization to filter-bound DNA in 0.5 M NaHPO₄, 7% SDS, 1 mM EDTA at 65°C, and washing in 0.2xSSC/0.1% SDS at 42°C (Ausubel *et al.,* 1989, above), and that is useful in practicing the present invention, as "usefulness" is defined above for polynucleotide molecules. In a preferred embodiment, the homologous polynucleotide molecule hybridizes to the complement of a polynucleotide molecule having a nucleotide sequence that encodes amino acid residues 22 to 215 of SEQ ID NO:4 under highly stringent conditions, *i.e.,* hybridization to filter-bound DNA in 0.5 M NaHPO₄, 7% SDS, 1 mM EDTA at 65°C, and washing in 0.1xSSC/0.1% SDS at 68°C (Ausubel *et al.,* 1989, above), and is useful in practicing the present invention. In a more preferred embodiment, the homologous polynucleotide molecule hybridizes under highly stringent conditions to the complement of a polynucleotide molecule consisting of the nucleotide sequence of SEQ ID NO:3 from nt 439 to nt 1023, and is useful in practicing the present invention.

The present invention further provides an isolated polynucleotide molecule comprising a nucleotide sequence that encodes a polypeptide that is homologous to the OmpW protein of the present invention, as "homologous polypeptide" is defined above.

The present invention further provides a polynucleotide molecule consisting of a nucleotide sequence that is a substantial portion of any of the aforementioned OmpW-related polynucleotide molecules of the present invention. As used herein, a "substantial portion" of an OmpW-related polynucleotide molecules means a polynucleotide molecule consisting of less than the complete nucleotide sequence of the particular full-length OmpW-related polynucleotide molecule, but comprising at least about 10%, and more preferably at least about 20%, of the nucleotide sequence of the particular full-length OmpW-related polynucleotide molecule, and that is useful in practicing the present invention, as "usefulness" is defined above for polynucleotide molecules. In a non-limiting embodiment, the substantial portion of the OmpW-related polynucleotide molecule encodes a peptide fragment of any of the aforementioned OmpW-related proteins or polypeptides of the present invention, as the term "peptide fragment" is defined above.

The present invention further provides a polynucleotide molecule comprising a nucleotide sequence which encodes the native OmpW signal sequence from about amino acid residue 1 to about amino acid residue 21 of SEQ ID NO:4. In a preferred though non-limiting embodiment, the OmpW signal sequence-encoding polynucleotide molecule comprises from about nt 376 to about nt 438 of SEQ ID NO:3.

The present invention further provides a polynucleotide molecule comprising a nucleotide sequence that encodes a fusion protein comprising the OmpW protein, homologous polypeptide, or peptide fragment, fused to a carrier or fusion partner.

### 5.2.3. Polynucleotide Molecules Encoding Omp27

References herein below to nucleotide sequences from SEQ ID NO:5, and to selected and substantial portions thereof, are intended to also refer to the corresponding Omp27-related nucleotide sequences of plasmid pER417 which is present in host cells of strain Pz417 (ATCC 98927), unless otherwise indicated. In addition, references herein below to the amino acid sequences shown in SEQ ID NO:6, and to peptide fragments thereof, are intended to also refer to the corresponding amino acid sequences encoded by the Omp27-related nucleotide sequence of plasmid pER417, unless otherwise indicated.

The present invention provides an isolated polynucleotide molecule comprising a nucleotide sequence encoding the APP protein, Omp27, with or without signal sequence. In a preferred embodiment, the isolated Omp27-encoding polynucleotide molecule of the present invention comprises the nucleotide sequence of SEQ ID NO:5 from about nt 238 to about nt 933. In a more preferred embodiment, the isolated Omp27-encoding polynucleotide molecule of the present invention comprises the nucleotide sequence of SEQ ID NO:5 from about nt 157 to about nt 933. In a non-limiting embodiment, the isolated Omp27-encoding polynucleotide molecule of the present invention comprises the nucleotide sequence of SEQ ID NO:5.

The present invention further provides an isolated polynucleotide molecule that is homologous to an APP Omp27-encoding polynucleotide molecule of the present invention. The term "homologous" when used to refer to an Omp27-encoding polynucleotide molecule means a polynucleotide molecule having a nucleotide sequence: (a) that encodes the same amino acid sequence as the nucleotide sequence of SEQ ID NO:5 from nt 238 to nt 933, but that includes one or more silent changes to the nucleotide sequence according to the degeneracy of the genetic code; or (b) that hybridizes to the complement of a polynucleotide molecule having a nucleotide sequence that encodes amino acid residues 28 to 258 of SEQ ID NO:6 under moderately stringent conditions, *i.e.,* hybridization to filter-bound DNA in 0.5 M NaHPO₄, 7% SDS, 1 mM EDTA at 65°C, and washing in 0.2xSSC/0.1% SDS at 42°C (Ausubel *et al.,* 1989, above), and that is useful in practicing the present invention, as "usefulness" is defined above for polynucleotide molecules. In a preferred embodiment, the homologous polynucleotide molecule hybridizes to the complement of a polynucleotide molecule having a nucleotide sequence that encodes amino acid residues 28 to 258 of SEQ ID NO:6 under highly stringent conditions, *i.e.,* hybridization to filter-bound DNA in 0.5 M NaHPO₄, 7% SDS, 1 mM EDTA at 65°C, and washing in 0.1xSSC/0.1% SDS at 68°C (Ausubel *et al.,* 1989, above), and is useful in practicing the present invention. In a more preferred embodiment, the homologous polynucleotide molecule hybridizes under highly stringent conditions to the complement of a polynucleotide molecule consisting of the nucleotide sequence of SEQ ID NO:5 from nt 238 to nt 933, and is useful in practicing the present invention.

The present invention further provides an isolated polynucleotide molecule comprising a nucleotide sequence that encodes a polypeptide that is homologous to the Omp27 protein of the present invention, as "homologous polypeptide" is defined above.

The present invention further provides a polynucleotide molecule consisting of a nucleotide sequence that is a substantial portion of any of the aforementioned Omp27-related polynucleotide molecules of the present invention. As used herein, a "substantial portion" of an Omp27-related polynucleotide molecules means a polynucleotide molecule consisting of less than the complete nucleotide sequence of the particular full-length Omp27-related polynucleotide molecule, but comprising at least about 10%, and more preferably at least about 20%, of the nucleotide sequence of the particular full-length Omp27-related polynucleotide molecule, and that is useful in practicing the present invention, as "usefulness" is defined above for polynucleotide molecules. In a non-limiting embodiment, the substantial portion of the Omp27-related polynucleotide molecule encodes a peptide fragment of any of the aforementioned Omp27-related proteins or polypeptides of the present invention, as the term "peptide fragment" is defined above.

The present invention further provides a polynucleotide molecule comprising a nucleotide sequence which encodes the native Omp27 signal sequence from about amino acid residue 1 to about amino acid residue 27 of SEQ ID NO:6. In a preferred though non-limiting embodiment, the Omp27 signal sequence-encoding polynucleotide molecule comprises from about nt 157 to about nt 237 of SEQ ID NO:5.

The present invention further provides a polynucleotide molecule comprising a nucleotide sequence that encodes a fusion protein comprising the Omp27 protein, homologous polypeptide, or peptide fragment, fused to a carrier or fusion partner.

### 5.2.4. Polynucleotide Molecules Encoding OmpA1

References herein below to nucleotide sequences from SEQ ID NO:7, and to selected and substantial portions thereof, are intended to also refer to the corresponding OmpA1-related nucleotide sequences of plasmid pER419 which is present in host cells of strain Pz419 (ATCC 98929), unless otherwise indicated. In addition, references herein below to the amino acid sequences shown in SEQ ID NO:8, and to peptide fragments thereof, are intended to also refer to the corresponding amino acid sequences encoded by the OmpA1-related nucleotide sequence of plasmid pER419, unless otherwise indicated.

The present invention provides an isolated polynucleotide molecule comprising a nucleotide sequence encoding the APP protein, OmpA1, with or without signal sequence. In a preferred embodiment, the isolated OmpA1-encoding polynucleotide molecule of the present invention comprises the nucleotide sequence of SEQ ID NO:7 from about nt 671 to about nt 1708. In a more preferred embodiment, the isolated OmpA1-encoding polynucleotide molecule of the present invention comprises the nucleotide sequence of SEQ ID NO:7 from about nt 614 to about nt 1708. In a non-limiting embodiment, the isolated OmpA1-encoding polynucleotide molecule of the present invention comprises the nucleotide sequence of SEQ ID NO:7.

The present invention further provides an isolated polynucleotide molecule that is homologous to an APP OmpA1-encoding polynucleotide molecule of the present invention. The term "homologous" when used to refer to an OmpA1-encoding polynucleotide molecule means a polynucleotide molecule having a nucleotide sequence: (a) that encodes the same amino acid sequence as the nucleotide sequence of SEQ ID NO:7 from nt 671 to nt 1708, but that includes one or more silent changes to the nucleotide sequence according to the degeneracy of the genetic code; or (b) that hybridizes to the complement of a polynucleotide molecule having a nucleotide sequence that encodes amino acid residues 20 to 364 of SEQ ID NO:8 under moderately stringent conditions, *i.e.*, hybridization to filter-bound DNA in 0.5 M NaHPO₄, 7% SDS, 1 mM EDTA at 65°C, and washing in 0.2xSSC/0.1% SDS at 42°C (Ausubel *et al.,* 1989, above), and that is useful in practicing the present invention, as "usefulness" is defined above for polynucleotide molecules. In a preferred embodiment, the homologous polynucleotide molecule hybridizes to the complement of a polynucleotide molecule having a nucleotide sequence that encodes amino acid residues 20 to 364 of SEQ ID NO:8 under highly stringent conditions, *i.e.,* hybridization to filter-bound DNA in 0.5 M NaHPO₄, 7% SDS, 1 mM EDTA at 65°C, and washing in 0.1xSSC/0.1% SDS at 68°C (Ausubel *et al.,* 1989, above), and is useful in practicing the present invention. In a more preferred embodiment, the homologous polynucleotide molecule hybridizes under highly stringent conditions to the complement of a polynucleotide molecule consisting of the nucleotide sequence of SEQ ID NO:7 from nt 671 to nt 1708, and is useful in practicing the present invention.

The present invention further provides an isolated polynucleotide molecule comprising a nucleotide sequence that encodes a polypeptide that is homologous to the OmpA1 protein of the present invention, as "homologous polypeptide" is defined above.

The present invention further provides a polynucleotide molecule consisting of a nucleotide sequence that is a substantial portion of any of the aforementioned OmpA1-related polynucleotide molecules of the present invention. As used herein, a "substantial portion" of an OmpA1-related polynucleotide molecules means a polynucleotide molecule consisting of less than the complete nucleotide sequence of the particular full-length OmpA1-related polynucleotide molecule, but comprising at least about 10%, and more preferably at least about 20%, of the nucleotide sequence of the particular full-length OmpA1-related polynucleotide molecule, and that is useful in practicing the present invention, as "usefulness" is defined above for polynucleotide molecules. In a non-limiting embodiment, the substantial portion of the OmpA1-related polynucleotide molecule encodes a peptide fragment of any of the aforementioned OmpA1-related polypeptides of the present invention, as the term "peptide fragment" is defined above.

The present invention further provides a polynucleotide molecule comprising a nucleotide sequence which encodes the native OmpA1 signal sequence from about amino acid residue 1 to about amino acid residue 19 of SEQ ID NO:8. In a preferred though non-limiting embodiment, the OmpA1 signal sequence-encoding polynucleotide molecule comprises from about nt 614 to about nt 670 of SEQ ID NO:7.

The present invention further provides a polynucleotide molecule comprising a nucleotide sequence that encodes a fusion protein comprising the OmpA1 protein, homologous polypeptide, or peptide fragment, fused to a carrier or fusion partner.

### 5.2.5. Polynucleotide Molecules Encoding OmpA2

References herein below to nucleotide sequences from SEQ ID NO:9, and to selected and substantial portions thereof, are intended to also refer to the corresponding OmpA2-related nucleotide sequences of plasmid pER420 which is present in host cells of strain Pz420 (ATCC 98930), unless otherwise indicated. In addition, references herein below to the amino acid sequences shown in SEQ ID NO:10, and to peptide fragments thereof, are intended to also refer to the corresponding amino acid sequences encoded by the OmpA2-related nucleotide sequence of plasmid pER420, unless otherwise indicated.

The present invention provides an isolated polynucleotide molecule comprising a nucleotide sequence encoding the APP protein, OmpA2, with or without signal sequence. In a preferred embodiment, the isolated OmpA2-encoding polynucleotide molecule of the present invention comprises the nucleotide sequence of SEQ ID NO:9 from about nt 254 to about nt 1306. In a more preferred embodiment, the isolated OmpA2-encoding polynucleotide molecule of the present invention comprises the nucleotide sequence of SEQ ID NO:9 from about nt 197 to about nt 1306. In a non-limiting embodiment, the isolated OmpA2-encoding polynucleotide molecule of the present invention comprises the nucleotide sequence of SEQ ID NO:9.

The present invention further provides an isolated polynucleotide molecule that is homologous to an APP OmpA2-encoding polynucleotide molecule of the present invention. The term "homologous" when used to refer to an OmpA2-encoding polynucleotide molecule means a polynucleotide molecule having a nucleotide sequence: (a) that encodes the same amino acid sequence as the nucleotide sequence of SEQ ID NO:9 from nt 254 to nt 1306, but that includes one or more silent changes to the nucleotide sequence according to the degeneracy of the genetic code; or (b) that hybridizes to the complement of a polynucleotide molecule having a nucleotide sequence that encodes amino acid residues 20 to 369 of SEQ ID NO:10 under moderately stringent conditions, *i.e.,* hybridization to filter-bound DNA in 0.5 M NaHPO₄, 7% SDS, 1 mM EDTA at 65°C, and washing in 0.2xSSC/0.1% SDS at 42°C (Ausubel *et al.,* 1989, above), and that is useful in practicing the present invention, as "usefulness" is defined above for polynucleotide molecules. In a preferred embodiment, the homologous polynucleotide molecule hybridizes to the complement of a polynucleotide molecule having a nucleotide sequence that encodes amino acid residues 20 to 369 of SEQ ID NO:10 under highly stringent conditions, *i.e.,* hybridization to filter-bound DNA in 0.5 M NaHPO₄, 7% SDS, 1 mM EDTA at 65°C, and washing in 0.1xSSC/0.1% SDS at 68°C (Ausubel *et al.,* 1989, above), and is useful in practicing the present invention. In a more preferred embodiment, the homologous polynucleotide molecule hybridizes under highly stringent conditions to the complement of a polynucleotide molecule consisting of the nucleotide sequence of SEQ ID NO:9 from nt 254 to nt 1306, and is useful in practicing the present invention.

The present invention further provides an isolated polynucleotide molecule comprising a nucleotide sequence that encodes a polypeptide that is homologous to the OmpA2 protein of the present invention, as "homologous polypeptide" is defined above.

The present invention further provides a polynucleotide molecule consisting of a nucleotide sequence that is a substantial portion of any of the aforementioned OmpA2-related polynucleotide molecules of the present invention. As used herein, a "substantial portion" of an OmpA2-related polynucleotide molecules means a polynucleotide molecule consisting of less than the complete nucleotide sequence of the particular full-length OmpA2-related polynucleotide molecule, but comprising at least about 10%, and more preferably at least about 20%, of the nucleotide sequence of the particular full-length OmpA2-related polynucleotide molecule, and that is useful in practicing the present invention, as "usefulness" is defined above for polynucleotide molecules. In a non-limiting embodiment, the substantial portion of the OmpA2-related polynucleotide molecule encodes a peptide fragment of any of the aforementioned OmpA2-related polypeptides of the present invention, as the term "peptide fragment" is defined above.

The present invention further provides a polynucleotide molecule comprising a nucleotide sequence which encodes the native OmpA2 signal sequence from about amino acid residue 1 to about amino acid residue 19 of SEQ ID NO:10. In a preferred though non-limiting embodiment, the OmpA2 signal sequence-encoding polynucleotide molecule comprises from about nt 197 to about nt 253 of SEQ ID NO:9.

The present invention further provides a polynucleotide molecule comprising a nucleotide sequence that encodes a fusion protein comprising the OmpA2 protein, homologous polypeptide, or peptide fragment, fused to a carrier or fusion partner.

### 5.3. Oligonucleotide Molecules

The present invention further provides oligonucleotide molecules that hybridize to any of the aforementioned polynucleotide molecules of the present invention, or that hybridize to a polynucleotide molecule having a nucleotide sequence that is the complement of any of the aforementioned polynucleotide molecules of the present invention. Such oligonucleotide molecules are preferably at least about 10 to 15 nucleotides in length, but can extend up to the length of any sub-sequence of SEQ ID NOS:1, 3, 5, 7 or 9, or homologous polynucleotide molecule thereof, and can hybridize to one or more of the aforementioned polynucleotide molecules under highly stringent conditions. For shorter oligonucleotide molecules, an example of highly stringent conditions includes washing in 6xSSC/0.5% sodium pyrophosphate at about 37°C for ∼14-base oligos, at about 48°C for ∼17-base oligos, at about 55°C for ∼20-base oligos, and at about 60°C for ∼23-base oligos. For longer oligonucleotide molecules (*i.e.,* greater than about 100 nts), examples of highly stringent conditions are provided in Section 5.2 above. Other appropriate hybridization conditions can be determined and adjusted as known in the art, depending upon the particular oligonucleotide and polynucleotide molecules utilized.

In a preferred embodiment, an oligonucleotide molecule of the present invention hybridizes under highly stringent conditions to a polynucleotide molecule consisting of a nucleotide sequence selected from SEQ ID NOS:1, 3, 5, 7, or 9, or to a polynucleotide molecule consisting of a nucleotide sequence that is the complement of a nucleotide sequence selected from SEQ ID NOS:1, 3, 5, 7, or 9.

In a non-limiting embodiment, an oligonucleotide molecule of the present invention comprises a nucleotide sequence selected from the group consisting of SEQ ID NOS:15-47 and 49-93 and the complements of said sequences. In a non-limiting embodiment, the oligonucleotide molecule consists of a nucleotide sequence selected from the group consisting of SEQ ID NOS:15-47 and 49-93 and the complements of said sequences.

The oligonucleotide molecules of the present invention are useful for a variety of purposes, including as primers in amplification of an APP protein-encoding polynucleotide molecule for use, *e.g.*, in differential disease diagnosis, or to encode or act as antisense molecules useful in gene regulation. Amplification can be used to detect the presence of a polynucleotide molecule encoding an APP protein in a tissue or fluid sample, *e.g.*, in mucous or bronchial fluid, from an infected animal. The production of a specific amplification product can help support a diagnosis of APP bacterial infection, while lack of an amplified product can indicate a lack of such infection. The oligonucleotide molecules disclosed herein can also be used to isolate homologous genes from other species or strains of *Actinobacillus,* or from other bacteria.

Amplification can be carried out using suitably designed oligonucleotide molecules in conjunction with standard techniques, such as the polymerase chain reaction (PCR), although other amplification techniques known in the art, *e.g.*, the ligase chain reaction, can be used. For example, for PCR, a mixture comprising suitably designed primers, a template comprising the nucleotide sequence to be amplified, and appropriate PCR enzymes and buffers as known in the art, is prepared and processed according to standard protocols to amplify a specific APP-related polynucleotide sequence of the template. Methods for conducting PCR are described, among other places, in Innis *et al.* (eds), 1995, above; and Erlich (ed), 1992, above.

### 5.4. Recombinant Expression Systems

### 5.4.1. Cloning And Expression Vectors

The present invention further provides compositions for cloning and expressing any of the polynucleotide molecules of the present invention, including recombinant cloning vectors and recombinant expression vectors comprising a polynucleotide molecule of the present invention, host cells transformed with any of said vectors, and cell lines derived therefrom. Recombinant vectors of the present invention, particularly expression vectors, are preferably constructed so that the coding sequence of the polynucleotide molecule (referred to hereinafter as the "APP coding sequence") is in operative association with one or more regulatory elements necessary for transcription and translation of the APP coding sequence to produce a polypeptide.

As used herein, the term "regulatory element" includes but is not limited to nucleotide sequences that encode inducible and non-inducible promoters, enhancers, operators and other elements known in the art that serve to drive and/or regulate expression of polynucleotide coding sequences. Also, as used herein, the APP coding sequence is in "operative association" with one or more regulatory elements where the regulatory elements effectively regulate and allow for the transcription of the coding sequence or the translation of its mRNA, or both.

Methods are well-known in the art for constructing recombinant vectors containing particular coding sequences in operative association with appropriate regulatory elements, including *in vitro* recombinant techniques, synthetic techniques, and *in vivo* genetic recombination. See, *e.g.,* the techniques described in Maniatis *et al.,* 1989, above; Ausubel *et al.,* 1989, above; Sambrook *et al.,* 1989, above; Innis *et al.,* 1995, above; and Erlich, 1992, above.

A variety of expression vectors are known in the art that can be utilized to express any of the APP coding sequences of the present invention, including recombinant bacteriophage DNA, plasmid DNA, and cosmid DNA expression vectors containing an APP coding sequence. Typical prokaryotic expression vector plasmids that can be engineered to contain an APP coding sequence of the present invention include pUC8, pUC9, pBR322 and pBR329 (Biorad Laboratories, Richmond, CA), pPL and pKK223 (Pharmacia, Piscataway, NJ), pQE50 (Qiagen, Chatsworth, CA), and pGEX series plasmids (Pharmacia), among many others. Typical eukaryotic expression vectors that can be engineered to contain an APP coding sequence of the present invention include an ecdysone-inducible mammalian expression system (Invitrogen, Carlsbad, CA), cytomegalovirus promoter-enhancer-based systems (Promega, Madison, WI; Stratagene, La Jolla, CA; Invitrogen), baculovirus-based expression systems (Promega), and plant-based expression systems, among others.

The regulatory elements of these and other vectors can vary in their strength and specificities. Depending on the host/vector system utilized, any of a number of suitable transcription and translation elements can be used. For instance, when cloning in mammalian cell systems, promoters isolated from the genome of mammalian cells, *e.g.*, mouse metallothionein promoter, or from viruses that grow in these cells, *e.g.*, vaccinia virus 7.5K promoter or Moloney murine sarcoma virus long terminal repeat, can be used. Promoters obtained by recombinant DNA or synthetic techniques can also be used to provide for transcription of the inserted coding sequence. In addition, expression from certain promoters can be elevated in the presence of particular inducers, *e.g.*, zinc and cadmium ions for metallothionein promoters. Non-limiting examples of transcriptional regulatory regions or promoters include for bacteria, the β-gal promoter, the T7 promoter, the TAC promoter, λ left and right promoters, trp and lac promoters, trp-lac fusion promoters, *etc.;* for yeast, glycolytic enzyme promoters, such as ADH-I and II promoters, GPK promoter, PGI promoter, TRP promoter, *etc.;* and for mammalian cells, SV40 early and late promoters, adenovirus major late promoters, among others.

Specific initiation signals are also required for sufficient translation of inserted coding sequences. These signals typically include an ATG initiation codon and adjacent sequences. In cases where the APP coding sequence of the present invention including its own initiation codon and adjacent sequences are inserted into the appropriate expression vector, no additional translation control signals are needed. However, in cases where only a portion of an APP coding sequence is inserted, exogenous translational control signals, including the ATG initiation codon, can be required. These exogenous translational control signals and initiation codons can be obtained from a variety of sources, both natural and synthetic. Furthermore, the initiation codon must be in phase with the reading frame of the coding regions to ensure in-frame translation of the entire insert.

Expression vectors can also be constructed that will express a fusion protein comprising any of the APP-related polypeptides of the present invention fused to a carrier or fusion partner. Such fusion proteins can be used for a variety of purposes, such as to increase the stability of a recombinantly-expressed APP protein, to raise antisera against an APP protein, to study the biochemical properties of an APP protein, to engineer an APP protein exhibiting altered immunological properties, or to aid in the identification or purification of a recombinantly-expressed APP protein. Possible fusion protein expression vectors include but are not limited to vectors incorporating sequences that encode a protective peptide, such as that described below in Section 8.2, as well as β-galactosidase and trpE fusions, maltose-binding protein fusions, glutathione-S-transferase (GST) fusions and polyhistidine fusions (carrier regions). Methods are well-known in the art that can be used to construct expression vectors encoding these and other fusion proteins.

Fusion proteins can be useful to aid in purification of the expressed protein. In non-limiting embodiments, *e.g.*, an APP protein-maltose-binding fusion protein can be purified using amylose resin; an APP protein-GST fusion protein can be purified using glutathione-agarose beads; and an APP protein-polyhistidine fusion protein can be purified using divalent nickel resin. Alternatively, antibodies against a carrier protein or peptide can be used for affinity chromatography purification of the fusion protein. For example, a nucleotide sequence coding for the target epitope of a monoclonal antibody can be engineered into the expression vector in operative association with the regulatory elements and situated so that the expressed epitope is fused to an APP protein of the present invention. In a non-limiting embodiment, a nucleotide sequence coding for the FLAG™ epitope tag (International Biotechnologies Inc.), which is a hydrophilic marker peptide, can be inserted by standard techniques into the expression vector at a point corresponding to the amino or carboxyl terminus of the APP protein. The expressed polypeptide-FLAG™ epitope fusion product can then be detected and affinity-purified using commercially available anti-FLAG™ antibodies.

The expression vector of the present invention can also be engineered to contain polylinker sequences that encode specific protease cleavage sites so that the expressed APP protein can be released from the carrier region or fusion partner by treatment with a specific protease. For example, the fusion protein vector can include a nucleotide sequence encoding a thrombin or factor Xa cleavage site, among others.

A signal sequence upstream from and in reading frame with the APP coding sequence can be engineered into the expression vector by known methods to direct the trafficking and secretion of the expressed APP polypeptide. Non-limiting examples of signal sequences include those which are native to the APP proteins of the present invention as disclosed herein, as well as signal sequences from α-factor, immunoglobulins, outer membrane proteins, penicillinase, and T-cell receptors, among others.

To aid in the selection of host cells transformed or transfected with a recombinant vector of the present invention, the vector can be engineered to further comprise a coding sequence for a reporter gene product or other selectable marker. Such a coding sequence is preferably in operative association with the regulatory elements, as described above. Reporter genes that are useful in practicing the invention are well-known in the art and include those encoding chloramphenicol acetyltransferase (CAT), green fluorescent protein, firefly luciferase, and human growth hormone, among others. Nucleotide sequences encoding selectable markers are well-known in the art, and include those that encode gene products conferring resistance to antibiotics or anti-metabolites, or that supply an auxotrophic requirement. Examples of such sequences include those that encode thymidine kinase activity, or resistance to methotrexate, ampicillin, kanamycin, chloramphenicol, zeocin, tetracycline, and carbenicillin, among many others.

In specific though non-limiting embodiments, the present invention provides the following plasmid cloning vectors, constructed as described below in Section 11, which are present in host cells that have been deposited with the American Type Culture Collection (ATCC): plasmid pER416, which is present in host cells of strain Pz416 (ATCC 98926), and which plasmid comprises the ORF of *omp20;* plasmid pER418, which is present in host cells of strain Pz418 (ATCC 98928), and which plasmid comprises the ORF of *ompW*; plasmid pER417, which is present in host cells of strain Pz417 (ATCC 98927), and which plasmid comprises the ORF of *omp27;* plasmid pER419, which is present in host cells of strain Pz419 (ATCC 98929), and which plasmid comprises the ORF of *ompA1;* and plasmid pER420, which is present in host cells of strain Pz420 (ATCC 98930), and which plasmid comprises the ORF of *ompA*2.

### 5.4.2. Transformation Of Host Cells

The present invention further provides host cells transformed with a polynucleotide molecule or recombinant vector of the invention, and cell lines derived therefrom. Host cells useful in practicing the present invention can either be prokaryotic or eukaryotic. Such transformed host cells include but are not limited to microorganisms, such as bacterial cells transformed with recombinant bacteriophage DNA, plasmid DNA or cosmid DNA expression vectors; or yeast cells transformed with recombinant expression vectors; or animal cells, such as insect cells infected with recombinant virus expression vectors, *e.g.*, baculovirus, or mammalian cells infected with recombinant virus expression vectors, *e.g.*, adenovirus or vaccinia virus, among others.

Bacterial cells are generally preferred as host cells. A strain of *E. coli* can typically be used, such as, *e.g.*, strain DH5α, available from Gibco BRL, Life Technologies (Gaithersburg, MD), or *E. coli* strain LW14, as described below. Eukaryotic host cells, including yeast cells, and mammalian cells, such as from a mouse, hamster, pig, cow, monkey, or human cell line, can also be used effectively. Examples of eukaryotic host cells that can be used to express the recombinant protein of the invention include Chinese hamster ovary (CHO) cells (*e.g.*, ATCC Accession No. CCL61) and NIH Swiss mouse embryo cells NIH/3T3 (*e.g.,* ATCC Accession No. CRL 1658).

A recombinant vector of the invention is preferably transformed or transfected into one or more host cells of a substantially homogeneous culture of cells. The vector is generally introduced into host cells in accordance with known techniques such as, *e.g.*, by calcium phosphate precipitation, calcium chloride treatment, microinjection, electroporation, transfection by contact with a recombined virus, liposome-mediated transfection, DEAE-dextran transfection, transduction, conjugation, or microprojectile bombardment. Selection of transformants can be conducted by standard procedures, such as by selecting for cells expressing a selectable marker, *e.g.*, antibiotic resistance, associated with the recombinant vector.

Once the vector is introduced into the host cell, the integration and maintenance of the APP coding sequence either in the host cell genome or episomally can be confirmed by standard techniques, *e.g.*, by Southern hybridization analysis, restriction enzyme analysis, PCR analysis, including reverse transcriptase PCR (rt-PCR), or by immunological assay to detect the expected protein product. Host cells containing and/or expressing the APP coding sequence can be identified by any of at least four general approaches, which are well-known in the art, including: (i) DNA-DNA, DNA-RNA, or RNA-antisense RNA hybridization; (ii) detecting the presence of "marker" gene functions; (iii) assessing the level of transcription as measured by the expression of APP-specific mRNA transcripts in the host cell; and (iv) detecting the presence of mature APP protein product as measured, *e.g.*, by immunoassay.

### 5.4.3. Expression Of Recombinant Polypeptides

Once the APP coding sequence has been stably introduced into an appropriate host cell, the transformed host cell is clonally propagated, and the resulting cells are grown under conditions conducive to the maximum production of the APP protein. Such conditions typically include growing such cells to high density. Where the expression vector comprises an inducible promoter, appropriate induction conditions such as, *e.g.,* temperature shift, exhaustion of nutrients, addition of gratuitous inducers (*e.g.,* analogs of carbohydrates, such as isopropyl-β-D-thiogalactopyranoside (IPTG)), accumulation of excess metabolic by-products, or the like, are employed as needed to induce expression.

Where the recombinantly-expressed APP protein is retained inside the host cells, the cells are harvested and lysed, and the APP protein is substantially purified or isolated from the lysate under extraction conditions known in the art to minimize protein degradation such as, *e.g.*, at 4°C, or in the presence of protease inhibitors, or both. Where the recombinantly-expressed APP protein is secreted from the host cells, the exhausted nutrient medium can simply be collected and the APP polypeptide substantially purified or isolated therefrom.

The recombinantly-expressed APP protein can be partially or substantially purified or isolated from cell lysates or culture medium, as appropriate, using standard methods, including but not limited to any combination of the following methods: ammonium sulfate precipitation, size fractionation, ion exchange chromatography, HPLC, density centrifugation, and affinity chromatography. Increasing purity of the APP polypeptide of the present invention can be determined as based, *e.g.*, on size, or reactivity with an antibody specific to the APP polypeptide, or by the presence of a fusion tag. For use in practicing the present invention, *e.g.*, in a vaccine composition, the APP protein can be used in an unpurified state as secreted into the culture fluid, or as present in host cells or in a cell lysate, or in substantially purified or isolated form. As used herein, an APP protein is "substantially purified" where the protein constitutes more than about 20 wt% of the protein in a particular preparation. Also, as used herein, an APP protein is "isolated" where the protein constitutes at least about 80 wt% of the protein in a particular preparation.

The present invention thus provides a method for preparing an APP protein, homologous polypeptide, peptide fragment or fusion protein of the present invention, comprising culturing a host cell transformed with a recombinant expression vector, said recombinant expression vector comprising a polynucleotide molecule comprising a nucleotide sequence encoding: (a) an APP protein comprising the amino acid sequence of SEQ ID NO:2, 4, 6, 8, or 10, either with or without its native signal sequence; or (b) a polypeptide that is homologous to the APP protein of (a); or (c) a peptide fragment of the APP protein of (a) or homologous polypeptide of (b); or (d) a fusion protein comprising the APP protein of (a), homologous polypeptide of (b), or peptide fragment of (c), fused to a fusion partner; which polynucleotide molecule is in operative association with one or more regulatory elements that control expression of the polynucleotide molecule in the host cell, under conditions conducive to the production of the APP protein, homologous polypeptide, peptide fragment or fusion protein, and recovering the APP protein, homologous polypeptide, peptide fragment or fusion protein from the cell culture.

Once an APP protein of the present invention has been obtained in sufficient purity, it can be characterized by standard methods, including by SDS-PAGE, size exclusion chromatography, amino acid sequence analysis, serological reactivity, *etc*. The amino acid sequence of the APP protein can be determined using standard peptide sequencing techniques. The APP protein can be further characterized using hydrophilicity analysis (see, *e.g.*, Hopp and Woods, 1981, Proc. Natl. Acad. Sci. *USA* 78:3824), or analogous software algorithms, to identify hydrophobic and hydrophilic regions. Structural analysis can be carried out to identify regions of the APP protein that assume specific secondary structures. Biophysical methods such as X-ray crystallography (Engstrom, 1974, Biochem. Exp. Biol. 11: 7-13), computer modelling (Fletterick and Zoller (eds), 1986, in: Current Communications in Molecular Biology, Cold Spring Harbor Laboratory, Cold Spring Harbor, NY), nuclear magnetic resonance (NMR), and mass spectrometry can also be used to characterize the protein. Information obtained from these studies can be used, *e.g.*, to design more effective vaccine compositions, or to select vaccines comprising only specific portions of the APP protein.

An APP protein that is useful in practicing the present invention is a polypeptide that: (a) is immunogenic, *i.e.*, capable of inducing by itself, or capable of contributing in combination with other APP proteins or other APP-related antigens to the induction of, a protective response against APP when administered to swine; or (b) is capable of inducing the production of anti-APP antibodies when administered to a member of a mammalian species; or (c) can be used as a diagnostic reagent to detect the presence of anti-APP antibodies in a blood or serum sample from a pig resulting from infection with APP or from vaccination with a vaccine of the present invention. Such a protein, once prepared, can be identified using routine screening procedures known in the art. For example, the ability to induce, or contribute to the induction of, a protective immune response against APP can be identified by administering the APP protein, either alone or in combination with other APP proteins or other APP-related antigens, respectively, to a pig, and testing for the resulting induction of APP-neutralizing antibodies, or for the resulting ability of the vaccinated animal to resist subsequent challenge with APP compared to an unvaccinated control. The ability to induce the production of APP-specific antibodies can be identified by administering the APP protein to a model animal, such as a mouse, pig, sheep, goat, horse, cow, *etc.*, and testing the animal's serum for the presence of APP-specific antibodies using standard techniques. The ability to use the APP protein as a diagnostic reagent can be determined by exposing the APP protein to a blood or serum sample of an animal previously or currently infected with APP, or previously vaccinated with a vaccine of the present invention, and detecting the binding to the APP protein of APP-specific antibodies from the sample using standard techniques, such as with an ELISA assay.

### 5.5. APP Vaccines

The present invention further provides a vaccine for protecting swine against APP, comprising an immunologically effective amount of one or more of the following: (a) an APP protein comprising an amino acid sequence selected from the group consisting of SEQ ID NOS:2, 4, 6, 8 or 10, either with or without its native signal sequence; (b) a polypeptide that is homologous to the APP protein of (a); (c) a peptide fragment consisting of a sub-sequence of the APP protein of (a) or the homologous polypeptide of (b); (d) a fusion protein comprising the APP protein of (a), homologous polypeptide of (b), or peptide fragment of (c), fused to a fusion partner; (e) an analog or derivative of the APP protein of (a), homologous polypeptide of (b), peptide fragment of (c), or fusion protein of (d); or (f) a polynucleotide molecule comprising a nucleotide sequence encoding the APP protein of (a), homologous polypeptide of (b), peptide fragment of (c), fusion protein of (d), or analog or derivative of (e); which APP protein, homologous polypeptide, peptide fragment, fusion protein, analog, derivative or polynucleotide molecule, can induce by itself, or in combination with one or more other such antigens contribute to the induction of, a protective response against APP in swine; and a veterinarily acceptable carrier. As used herein, the term "immunologically effective amount" refers to that amount of antigen that is capable of inducing, or contributing to the induction of, a protective immune response in swine against one or more serotypes of APP after either a single administration or after multiple administrations.

The phrase "capable of inducing a protective immune response" is used broadly herein to include the induction of, or increase in, any immune-based response in the pig in response to vaccination, including either an antibody or cell-mediated immune response, or both, that serves to protect the vaccinated animal against APP. The terms "protective immune response", "protect", and the like, as used herein, are not limited to absolute prevention of APP-associated swine pneumonia or absolute prevention of infection of pigs by APP, but are intended to also refer to any reduction in the degree or rate of infection by the pathogen, or any reduction in the severity of the disease or in any symptom or condition resulting from infection with the pathogen, including, *e.g.*, any detectable decrease in lung pathology, as compared to that occurring in an unvaccinated, infected control animal.

Vaccine compositions of the present invention can be formulated following accepted convention using standard buffers, carriers, stabilizers, diluents, preservatives, and solubilizers, and can also be formulated to facilitate sustained release. Diluents can include water, saline, dextrose, ethanol, glycerol, and the like. Additives for isotonicity can include sodium chloride, dextrose, mannitol, sorbitol, and lactose, among others. Stabilizers include albumin, among others.

Adjuvants can optionally be employed in the vaccine. Non-limiting examples of adjuvants include the RIBI adjuvant system (Ribi Inc.), alum, aluminum hydroxide gel, oil-in-water emulsions, water-in-oil emulsions such as, *e.g.*, Freund's complete and incomplete adjuvants, Block co polymer (CytRx, Atlanta GA), SAF-M (Chiron, Emeryville CA), AMPHIGEN® adjuvant, saponin, Quil A, QS-21 (Cambridge Biotech Inc., Cambridge MA), or other saponin fractions, SEAM-1, monophosphoryl lipid A, Avridine lipid-amine adjuvant, heat-labile enterotoxin from *E*. *coli* (recombinant or otherwise), cholera toxin, or muramyl dipeptide, among many others. The vaccine can further comprise one or more other immunomodulatory agents such as, *e.g.*, interleukins, interferons, or other cytokines.

Suitable veterinarily acceptable vaccine vehicles, carriers, and additives are known, or will be apparent to those skilled in the art; see, *e.g.*, Remington's Pharmaceutical Science, 18th Ed., 1990, Mack Publishing, which is incorporated herein by reference. The vaccine can be stored in solution, or alternatively in lyophilized form to be reconstituted with a sterile diluent solution prior to administration.

The present invention further provides vaccine formulations for the sustained release of the antigen. Examples of such sustained release formulations include the antigen in combination with composites of biocompatible polymers, such as, *e.g.*, poly(lactic acid), poly(lactic-co-glycolic acid), methylcellulose, hyaluronic acid, collagen and the like. The structure, selection and use of degradable polymers in drug delivery vehicles have been reviewed in several publications, including A. Domb *et al.,* 1992, Polymers for Advanced Technologies 3: 279-292, which is incorporated herein by reference. Additional guidance in selecting and using polymers in pharmaceutical formulations can be found in the text by M. Chasin and R. Langer (eds), 1990, "Biodegradable Polymers as Drug Delivery Systems" in: Drugs and the Pharmaceutical Sciences, Vol. 45, M. Dekker, NY, which is also incorporated herein by reference. Alternatively, or additionally, the antigen can be microencapsulated to improve administration and efficacy. Methods for microencapsulating antigens are well-known in the art, and include techniques described, among other places, in U.S. Patent 3,137,631; U.S. Patent 3,959,457; U.S. Patent 4,205,060; U.S. Patent 4,606,940; U.S. Patent 4,744,933; U.S. Patent 5,132,117; and International Patent Publication WO 95/28227, all of which are incorporated herein by reference.

Liposomes and liposome derivatives (*e.g.*, cochleates, vesicles) can also be used to provide for the sustained release of the antigen. Details regarding how to make and use liposomal formulations can be found, among other places, in U.S. Patent 4,016,100; U.S. Patent 4,452,747; U.S. Patent 4,921,706; U.S. Patent 4,927,637; U.S. Patent 4,944,948; U.S. Patent 5,008,050; and U.S. Patent 5,009,956, all of which are incorporated herein by reference.

In a non-limiting embodiment, the vaccine of the present invention can be a combination vaccine for protecting swine against APP and, optionally, one or more other diseases or pathological conditions that can afflict swine, which combination vaccine comprises a first component comprising an immunologically effective amount of an antigen of the present invention selected from the group consisting of an APP protein, homologous polypeptide, peptide fragment, fusion protein, analog, derivative, or polynucleotide molecule of the present invention which is capable of inducing, or contributing to the induction of, a protective response against APP in swine; a second component comprising an immunologically effective amount of an antigen that is different from the antigen in the first component, and which is capable of inducing, or contributing to the induction of, a protective response against a disease or pathological condition that can afflict swine; and a veterinarily acceptable carrier or diluent.

The second component of the combination vaccine is selected based on its ability to induce, or to contribute to the induction of, a protective response against either APP or another pathogen, disease, or pathological condition which afflicts swine, as known in the art. Any immunogenic composition now known or to be determined in the future to be useful in a vaccine composition for swine can be used in the second component of the combination vaccine. Such immunogenic compositions include but are not limited to those that provide protection against *Actinobacillus suis, Pasteurella multocida, Salmonella cholerasuis, Streptococcus suis, Erysipelothrix rhusiopathiae, Leptospira sp., Staphylococcus hyicus, Haemophilus parasuis, Bordetella bronchiseptica, Mycoplasma hyopneumoniae, Lawsonia intracellularis, Escherichia coli*, porcine reproductive and respiratory syndrome virus, swine influenza virus, transmissible gastroenteritis virus, porcine parvovirus, encephalomyocarditis virus, coronavirus, pseudorabies virus, and circovirus. In a non-limiting embodiment, the combination vaccine comprises a combination of components including one or more APP proteins of the present invention, and one or more other APP bacterial components such as APXI, ApxII, and OmIA.

The antigen comprising the second component can optionally be covalently linked to the antigen of the first component to produce a chimeric molecule. In a non-limiting embodiment, the antigen of the second component comprises a hapten, the immunogenicity of which is detectably increased by conjugation to the antigen of the first component. Chimeric molecules comprising covalently-linked antigens of the first and second components of the combination vaccine can be synthesized using one or more techniques known in the art. For example, a chimeric molecule can be produced synthetically using a commercially available peptide synthesizer utilizing standard chemical synthetic processes (see, *e.g.,* Merrifield, 1985, Science 232:341-347). Alternatively, the separate antigens can be separately synthesized and then linked together by the use of chemical linking groups, as known in the art. Alternatively, a chimeric molecule can be produced using recombinant DNA technology whereby, *e.g.,* separate polynucleotide molecules having sequences encoding the different antigens of the chimeric molecule are spliced together in-frame and expressed in a suitable transformed host cell for subsequent isolation of the chimeric fusion polypeptide. Where the vaccine of the invention comprises a polynucleotide molecule rather than a polypeptide, the spliced polynucleotide molecule can itself be used in the vaccine composition. Ample guidance for carrying out such recombinant techniques is provided, among other places, in Maniatis *et al.,* 1989, above; Ausubel *et al.,* 1989, above; Sambrook *et al.,* 1989, above; Innis *et al.,* 1995, above; and Erlich, 1992, above.

The present invention further provides a method of preparing a vaccine for protecting swine against APP, comprising combining an immunologically effective amount of one or more antigens of the present invention selected from the group consisting of an APP protein, homologous polypeptide, peptide fragment, fusion protein, analog, derivative, or polynucleotide molecule of the present invention which is capable of inducing, or contributing to the induction of, a protective response against APP in swine, with a veterinarily acceptable carrier or diluent, in a form suitable for administration to swine.

The present invention further provides a method of vaccinating swine against APP, comprising administering a vaccine of the present invention to a pig. The amount of antigen administered depends upon such factors as the age, weight, health and general physical characteristics of the animal being vaccinated, as well as the particular vaccine composition to be administered. Determination of the optimum dosage for each parameter can be made using routine methods in view, *e.g.*, of empirical studies. The amount of APP protein administered will preferably range from about 0.1 µg to about 10 mg of polypeptide, more preferably from about 10 µg to about 1 mg, and most preferably from about 25 µg to about 0.1 mg. For a DNA vaccine, the amount of a polynucleotide molecule will preferably range from about 0.05 µg to about 500 mg, more preferably from about 0.5 µg to about 50 mg. In addition, the typical dose volume of the vaccine will range from about 0.5 ml to about 5 ml per dose per animal.

Animals can be vaccinated at any appropriate time, including within 1 week after birth, or at weaning age, or just prior to or at the time of breeding, or at the time that APP infection first begins to appear in one or more members of an animal population. Supplemental administrations, or boosters, may be required to achieve full protection. Methods for determining whether adequate immune protection has been achieved in an animal are well-known in the art, and include, *e.g.,* determining seroconversion.

The vaccine can be administered by any appropriate route such as, *e.g.*, by oral, intranasal, intramuscular, intra-lymph node, intradermal, intraperitoneal, subcutaneous, rectal or vaginal administration, or by a combination of routes. The skilled artisan will readily be able to formulate the vaccine composition according to the route chosen.

The present invention further provides a vaccine kit for vaccinating swine against infection or disease caused by APP, comprising a first container comprising an immunologically effective amount of one or more antigens of the present invention selected from the group consisting of an APP protein, homologous polypeptide, peptide fragment, fusion protein, analog, derivative, or polynucleotide molecule of the present invention which is capable of inducing, or contributing to the induction of, a protective response against APP in swine. The kit can optionally further comprise a second container comprising a veterinarily acceptable carrier or diluent. The vaccine composition can be stored in the first container either in solution or in lyophilized form to be reconstituted using the carrier or diluent of the second container.

### 5.6. Anti-APP Antibodies

The present invention further provides isolated antibodies that bind to an APP protein of the present invention. Such antibodies are useful for a variety of purposes including, *e.g.*, as affinity reagents to purify the APP protein, or to detect the presence of the APP protein in a cell, tissue or fluid sample collected from an APP-infected animal, *e.g.*, by use of an ELISA or Western blot assay, or as a therapeutic agent to prevent, control or treat APP infection.

Antibodies against an APP protein of the present invention can be raised according to known methods by administering an appropriate antigen of the present invention to a host animal selected from pigs, cows, horses, rabbits, goats, sheep, and mice, among others. Various adjuvants, such as those described above, can be used to enhance antibody production. Antibodies of the present invention can either be polyclonal or monoclonal. Polyclonal antibodies can be prepared and isolated from the serum of immunized animals and tested for anti-APP protein specificity using standard techniques. Alternatively, monoclonal antibodies against an APP protein can be prepared and isolated using any technique that provides for the production of antibody molecules by continuous cell lines in culture. These include but are not limited to the hybridoma technique originally described by Kohler and Milstein (Nature, 1975, 256: 495-497); the human B-cell hybridoma technique (Kosbor *et al.,* 1983, Immunology Today 4:72; Cote *et al.,* 1983, Proc. Natl. Acad. Sci. *USA* 80: 2026-2030); and the EBV-hybridoma technique (Cole *et al.,* 1985, Monoclonal Antibodies and Cancer Therapy, Alan R. Liss, Inc., pp. 77-96). Alternatively, techniques described for the production of single chain antibodies (see, *e.g.*, U.S. Patent 4,946,778) can be adapted to produce APP protein-specific single chain antibodies.

Also encompassed within the scope of the present invention are antibody fragments that contain specific binding sites for an APP protein of the present invention. Such fragments include but are not limited to F(ab')₂ fragments, which can be generated by pepsin digestion of an intact antibody molecule, and Fab fragments, which can be generated by reducing the disulfide bridges of the F(ab')₂ fragments. Alternatively, Fab and/or scFv expression libraries can be constructed (see, *e.g.,* Huse *et al.,* 1989, Science 246: 1275-1281) to allow rapid identification of fragments having the desired specificity to an APP protein of the present invention.

Techniques for the production and isolation of monoclonal antibodies and antibody fragments are well-known in the art, and are additionally described, among other places, in Harlow and Lane, 1988, Antibodies: A Laboratory Manual, Cold Spring Harbor Laboratory, and in J. W. Goding, 1986, Monoclonal Antibodies: Principles and Practice, Academic Press, London. All of the above-cited publications are incorporated herein by reference.

### 5.7. Diagnostic Kits

The present invention further provides diagnostic kits. In a non-limiting embodiment, the diagnostic kit of the present invention comprises a first container comprising an APP protein, homologous polypeptide, peptide fragment, fusion protein, analog, or derivative of the present invention that can specifically bind to antibodies directed against the APP protein; and a second container comprising a secondary antibody directed against porcine antibodies. The secondary antibody preferably comprises a detectable label. Such a diagnostic kit is useful to detect pigs that currently are, or have previously been, infected with APP, or that have seroconverted as a result of vaccination with a vaccine of the present invention.

In an alternative embodiment, the present invention provides a diagnostic kit comprising a first container comprising a primary antibody that binds to an APP protein; and a second container comprising a secondary antibody that binds to a different epitope on the APP protein, or that is directed against the primary antibody. The secondary antibody preferably comprises a detectable label. In an alternative embodiment, the diagnostic kit comprises a container comprising a polynucleotide molecule or oligonucleotide molecule of the present invention that can specifically hybridize to, or amplify, an APP-specific polynucleotide molecule. These latter two diagnostic kits are useful to detect pigs that are currently infected with APP.

### 5.8. Anti-Sense Oligonucleotides And Ribozymes

The present invention further provides oligonucleotide molecules that include anti-sense oligonucleotides, phosphorothioates and ribozymes that function to bind to, degrade and/or inhibit the translation of an APP protein-encoding mRNA.

Anti-sense oligonucleotides, including anti-sense RNA molecules and anti-sense DNA molecules, act to directly block the translation of mRNA by binding to targeted mRNA and thereby preventing protein translation. For example, antisense oligonucleotides of at least about 15 bases and complementary to unique regions of the mRNA transcript sequence encoding an APP protein can be synthesized, *e.g.,* by conventional phosphodiester techniques.

Ribozymes are enzymatic RNA molecules capable of catalyzing the specific cleavage of RNA. The mechanism of ribozyme action involves sequence specific hybridization of the ribozyme molecule to complementary target RNA, followed by endonucleolytic cleavage. Engineered hammerhead motif ribozyme molecules that specifically and efficiently catalyze endonucleolytic cleavage of APP protein mRNA sequences are also within the scope of the invention.

Specific ribozyme cleavage sites within any potential RNA target are initially identified by scanning the target molecule for ribozyme cleavage sites which include the following sequences, GUA, GUU, and GUC. Once identified, short RNA sequences of between about 15 and 20 ribonucleotides corresponding to the region of the target gene containing the cleavage site can be evaluated for predicted structural features, such as secondary structure, that can render the oligonucleotide sequence unsuitable. The suitability of candidate targets can also be evaluated by testing their accessibility to hybridization with complementary oligonucleotides, using, *e.g.*, ribonuclease protection assays.

Both the anti-sense oligonucleotides and ribozymes of the invention can be prepared by known methods. These include techniques for chemical synthesis such as, *e.g.*, by solid phase phosphoramadite chemical synthesis. Alternatively, anti-sense RNA molecules can be generated by *in vitro* or *in vivo* transcription of DNA sequences encoding the RNA molecule. Such DNA sequences can be incorporated into a wide variety of vectors which incorporate suitable RNA polymerase promoters such as the T7 or SP6 polymerase promoters.

Various modifications to the oligonucleotides of the invention can be introduced as a means of increasing intracellular stability and half-life. Possible modifications include but are not limited to the addition of flanking sequences of ribonucleotides or deoxyribonucleotides to the 5' and/or 3' ends of the molecule, or the use of phosphorothioate or 2'-O-methyl rather than phosphodiesterase linkages within the oligonucleotide backbone.

The following examples are illustrative only, and are not intended to limit the scope of the present invention.

### 6. EXAMPLE: IDENTIFICATION OF NOVEL APP PROTEINS

Results of the following experiment demonstrate the specificities of local antibody responses induced when pigs that were previously challenged with APP serotype-5 were heterologously rechallenged with APP serotype-7. The antibody specificities were used to identify previously unrecognized APP proteins, three of which (Omp20, OmpW, Omp27) were shown by Western blot analysis to be present in all twelve APP serotypes. The two additional novel proteins (OmpA1, OmpA2) were identified following protein fraction isolation and concentration (see Section 6.1.6, below).

### 6.1. Materials And Methods

### 6.1.1. Bacterial Challenge

The APP serotype-5 culture (strain K-17) used to prepare porcine challenge material was obtained from Dr. R. A. Schultz, Avoca, IA, USA. The APP serotype-7 culture (strain WF-83) used to prepare porcine challenge material was obtained from Dr. E. Jones, Swedeland, PA, USA.

Clinically healthy 7-8 week old cross-bred pigs were obtained from a herd in Nebraska with no previous history of APP infection, and were housed in isolation facilities at Pfizer Animal Health, Lincoln, NE, according to IACUC guidelines. Animals were examined by a veterinarian to determine their health status prior to initiation of the study. Following a 2-week acclimatization period, pigs were anesthetized using a combination of 100 mg/ml of Telazol, 50 mg/ml of Xylazine, and 50 mg/ml of Ketamine administered at a rate of approximately 1 ml/50 lb of body weight, and intranasally inoculated with 2.6 X 10⁶ cfu of APP serotype-5. Seventy-eight days after primary challenge, six of the surviving pigs, which had demonstrated clinical APP disease but had recovered, were again anesthetized as described above. The first and second of these 6 pigs were intranasally rechallenged with 1 x 10⁷ cfu of APP serotype-5 (homologous rechallenge); the third and fourth of these pigs were intranasally rechallenged with 1 x 10⁸ cfu of APP serotype-7 (heterologous rechallenge); and the fifth and sixth pigs were intranasally inoculated with bacterial growth medium only (control). All challenge inoculums were administered in 1 ml volumes (0.5 ml/nostril).

All pigs were sacrificed 48 hrs post-rechallenge, their serum and organs were collected, and tissue pieces from the organs were cultured *in vitro* for 24 or 48 hr, as described in Sections 6.1.2 and 6.1.3 below. Antibody-containing supernatants from the tissue explant cultures were used to compare the memory antibody profile elicited by heterologous rechallenge to that elicited by homologous rechallenge or single challenge (control). The specificity of antibodies produced by the tissue explants *in vitro* was determined by Western blot analysis against a panel of whole bacterial cell preparations representing all 12 APP serotypes. This analysis established the presence of an antibody profile following heterologous rechallenge that was distinct from the antibody profile following homologous rechallenge or single challenge (control).

APP serotype-1 (strain 4074), serotype-2 (strain 4226), serotype-3 (strain 1421), serotype-4 (strain M62), serotype-5 (strain K-17), serotype-6 (strain Femo FE 171D), serotype-7 (strain WF-83), serotype-8 (strain 405), serotype-9 (strain CVJ-13261), serotype-10 (strain 13039), serotype-11 (strain 56153), and serotype-12 (strain 8329) reference cultures used to prepare material for Western blots were obtained from Dr. B. Fenwick, Kansas State University, Manhattan, KS, USA.

### 6.1.2. Tissue Collection

Serum samples were obtained from pigs prior to rechallenge, and again prior to necropsy (48 hr post-rechallenge). Pigs were euthanized 48 hr post-rechallenge with an overdose of intravenous pentobarbital. Lungs were removed and examined for characteristic gross lesions attributable to APP infection, and a complete gross examination was done of all major organs. Tissue samples of lung, lymph nodes (mesenteric, popliteal, and bronchial), Peyers patches, and tonsils were collected, washed with 70% ethanol, and rinsed 3x in RPMI transport media (RPMI medium (Gibco/BRL, Grand Island, NY) supplemented with 10 mM HEPES, 5% FBS, 50 U/ml of penicillin and 50 µg/ml of streptomycin). After washing, tissues were placed in 50 ml centrifuge tubes containing 10 ml of transport media and placed on ice until processed in the laboratory (within 3 hr of collection). Additional tissues samples were frozen in liquid nitrogen for future mRNA isolation and immunohistochemistry, or fixed in formalin for histopathology. A sample of lung tissue was also submitted to the University of Nebraska-Lincoln diagnostic laboratory for bacterial identification.

### 6.1.3. Tissue Explant Cultures

Tissues were placed in individual Petri dishes containing approximately 5 ml of transport media. Small tissue pieces of approximately 2 x 2 mm were cut off from the original sample with a scalpel blade and/or scissors and placed in individual wells of 12- or 24-well plates (Costar, Cambridge, MA) containing 2 ml of wash media (Ca²⁺ and Mg²⁺ free Hank's balanced salt solution (HBSS) supplemented with 10mM HEPES and 50 µg/ml of gentamicin). The tissue pieces were rinsed in the wash media and transferred to another well containing wash media. This washing/rinsing operation was repeated four times, and the tissue pieces were then transferred to wells containing RPMI media supplemented with 10% FBS, 10 mM HEPES, 2 mM glutamine, 50 µg/ml gentamicin, 62 µg/ml amphotericin B, 40 µg/ml sodium desoxycholate, 50 U/ml penicillin and 50 µg/ml of streptomycin. Plates were incubated at 38.5° C for 24 or 48 hr in a humidified chamber having 5% CO₂. After incubation, supernatant fluids were removed and frozen at -70°C.

### 6.1.4. Western Blot Analysis

The specificity of recovered antibodies in the tissue explant supernatants was examined by Western blot analysis as follows. Representative isolates of each of the 12 APP serotypes were each grown separately to generate whole bacterial cell antigen to test the supernatants. Each strain was cultured (1% seed) in minimal medium-3 (MM3) (1.8% Bacterin HP medium, 1.7% lactic acid, 0.3% glycerol, 0.05M HEPES, 0.011 M L-glutamic acid (monosodium salt), 5 x 10⁻⁵ M nicotinamide, and 0.2% casamino acids) supplemented with 10 µg/ml of β-nicotinamide adenine dinucleotide (β-NAD), for 5-6 hr at 37°C and 180 rpm until OD₅₆₀ of ∼0.5-0.6. The cells were pelleted by centrifugation at 12,000 x g for 10 min, the medium was reserved for analysis, and the pellet was resuspended in 5 ml Dulbecco's phosphate buffered saline (DPBS). Prior to the protein assay, the resuspended pellet was frozen at -20°C and then thawed in order to lyse any intact bacterial cells. The protein concentration of each preparation was determined using a BCA Protein Assay Reagent Kit (Pierce, Rockford, IL). APP antigen preparations (5 µg/lane) were loaded onto a 4-20% Tris-glycine gel (Novex, San Diego, CA), and proteins were separated by electrophoresis at rm temp with a constant current of 20 mA.

Separated proteins were transferred to ProBlot™ membranes (Applied Biosystems, Foster City, CA) using a semi-dry graphite electroblotter (Milliblot, Millipore, Seattle, WA). Transfer was performed at rm temp for 30 min at a constant current of 200 mA. After transfer was complete, membranes were blocked by incubating overnight at rm temp with Buffer A (50 mM Tris HCI, 150 mM NaCI, pH 7.4 and 5% (w/v) nonfat dried milk). The blocking buffer was then decanted and replaced with either serum (1:100 dilution) or tissue explant culture supernatants (1:3 dilution) in Buffer A, and the membranes were incubated for 1 hr at rm temp, followed by a 10 min wash in Buffer B (Buffer A containing 0.2% (v/v) Triton X-100) and two 10 min washes in Buffer A. After washing was complete, the membranes were incubated for 1 hr at rm temp with phosphatase-conjugated goat anti-swine antibodies (Kirkegaard & Perry Laboratories, Gaithersburg, MD) diluted 1:1000 in Buffer A. The membranes were then washed in Buffer A for 10 min, and incubated for 15 min with 5-bromo-4-chloro-3-indolyl-phosphate/nitroblue tetrazolium (BCIP/NBT) substrate system (Kirkegaard & Perry Laboratories).

### 6.1.5. Preparation Of APP Serotype-7 Membranes

An aliquot of APP serotype-7 was seeded (1%) into MM3 supplemented with 10 µg/ml β-NAD and cultured overnight at 37°C (180 rpm). A portion of the overnight culture was inoculated into fresh medium (bacterial inoculum was 3% of total volume) and incubated for 5-6 hr or to a culture density of 274 Klett units. The cells were pelleted by centrifugation at 4,500 rpm for 40 min at 10°C, the supernatant was removed, and the pellet was resuspended in 5 ml of 50 mM Tris-HCI, pH 8.0, with sufficient PMSF (phenyl methylsulfonyl fluoride) to result in a final concentration of 1 mM PMSF. Bacterial cells were lysed using a French Press under 16,000 lb/in² in a 40K PSI pressure cell (Sim Aminco., Rochester, NY). The broken cells were centrifuged at 1,000 x g for 15 min to remove large bacterial debris. Crude total membranes were collected by centrifugation at 45,000 rpm for 60 min at 18°C. The supernatant was discarded, the pellet was resuspended in 50 mM Tris-HCI, pH 8.0, and protein was determined using the Bradford Standard Protein Assay.

To 15 mg of crude membrane in a 3 ml volume was added 30 µl of 100 mM PMSF and 750 µl of 2.5% sarkosyl, and the entire volume was mixed thoroughly. After a 30 min incubation on ice, the membranes were pelleted by centrifugation at 200,000 x g for 15 min at 10°C. The supernatant was removed from the pelleted membrane fraction and the pellet was resuspended in 3 ml of 50 mM Tris-HCI/100 mM NaCI, pH 8.0. This membrane preparation, which represented the APP serotype-7 membrane antigen, was then stored at -20°C.

### 6.1.6. Membrane Protein Fractionation and Purification

Purification of APP proteins for N-terminal sequencing was achieved through continuous-elution SDS-PAGE using a BioRad Model 491 Prep Cell (BioRad, Richmond, CA). A 10 ml volume (4.5 mg total protein) of APP serotype-7 membrane protein fraction was mixed with an equal volume of non-reducing sample buffer (125 mM Tris-HCL, pH 6.8, 4% SDS, 20% glycerol, and 0.1% bromphenol blue). The protein-buffer mixture was boiled for 5 min and applied to a 3% stacking/15% separation SDS-polyacrylamide gel. Samples were electrophoresed at 20 mA constant current (initial voltage 175-250 V, final voltage 200-300 V) for 72 hr. Approximately 800 x 5 ml fractions were collected at a flow rate of 1 ml/min throughout the run, and analyzed for protein content by spectrophotometry at A₂₈₀. Every 10th fraction was analyzed by SDS-PAGE and silver staining (Bio-Rad, Richmond, CA). Fractions which putatively contained the same protein, as determined by molecular weight, were pooled and stored at 4°C. Pooled samples were desalted and concentrated in preparation for N-terminal sequencing. Desalting was performed by applying aliquots of pooled sample to a Presto™ desalting column (Pierce, Rockford, IL) with a 10 ml bed volume. Three ml aliquots of each protein pool were applied to separate columns and eluted in ddH₂O in 10 x 2 ml fractions. This was repeated 10 times for each protein pool until 30 ml had been desalted. As determined by Western blot analysis, the majority of the desalted protein was found in fraction No. 2. Therefore, the second fraction from each of the 10 elutions were pooled for each individual protein. The resulting 20 ml samples were lyophilized and resuspended in 0.5 ml ddH₂O for N-terminal sequencing. N-terminal sequences were obtained at the Pfizer Central Research Molecular Sciences Sequence Facility.

### 6.2. Results

### 6.2.1. Clinical Signs And Pathological Findings After Rechallenge

Pigs did not show any signs of clinical disease after either homologous (serotype-5) or heterologous (serotype-7) rechallenge. Pathological examination confirmed that the animals had not developed lung lesions that would be consistent with acute APP infection following rechallenge. However, bronchial lymph nodes of the animals rechallenged with serotype-7 were hemorrhagic and enlarged compared to those from animals homologously rechallenged with serotype-5 or from control animals.

### 6.2.2. Specificity Of Antibodies Elicited by Rechallenge

The specificity of antibodies present in serum and tissue explant supernatants was assessed by Western blot analysis as described above. All tissue-derived supernatants collected after 24 or 48 hr incubation contained antibodies that specifically recognized APP proteins. In general, the reactivity against serotype-5 antigens was greater than the reactivity against antigens of serotype-7 or serotype-1. However, the reactivity of most tissue-derived supernatants was less intense and narrower in spectrum than the reactivity of serum (FIGURE 1a). In general, the reactivity of tissue explant supernatants had no particular pattern. Nevertheless, the pattern of Western blot reactivity of tissue explant supernatants from one specific animal (No. 803, heterologously rechallenged with serotype-7) was strong, and highlighted several low molecular weight proteins present in APP serotypes-1, -5, and -7 (FIGURE 1b). This supernatant was used as the antibody source to further characterize the degree of cross-reactivity of the secondary (memory) antibody response elicited by heterologous rechallenge with APP serotype-7 in pigs that had been challenged initially with APP serotype-5.

The degree of cross-reactivity of the antibodies in the BLN tissue explant supernatants from pig No. 803 was assessed by Western blot analysis using whole bacterial cell antigens prepared from each of the twelve different APP serotypes. This analysis showed that three of the low molecular weight proteins recognized by the antibodies were present in all twelve serotypes (FIGURE 2). Antibodies present in this BLN tissue explant supernatant also recognized other protein bands. A high molecular weight band present in serotypes-1, 2, 4, 5, 6, 7, 8, and 9, which may correspond to the exotoxin, Apx II (see Nakai, 1983, above), and another protein band present in serotypes 2, 5, 8 and 10, represented the second most cross-reactive patterns. Western blot analysis of APP cell pellets and supernatants revealed that reactivity of antibodies in the BLN tissue explant supernatants to the low molecular weight proteins was restricted to proteins present in the cell pellets (FIGURE 3), indicating that the proteins are associated with the bacterial cell and are not secreted.

### 6.2.3. Proteins Recognized By Cross-Reactive Antibodies

The low molecular weight proteins were purified as described above, yielding partially purified preparations containing the protein of interest as identified by Western blot analysis using: (a) BLN tissue explant supernatant fluids from pig No. 803; or (b) serum from pig No. 803 (FIGURE 4). Upon fractionation of membrane proteins, four protein bands with molecular weights of about 19-20, about 23, about 27, and about 29 kDa, respectively, were identified using this procedure.

N-terminal sequence analysis of the proteins in the four bands yielded a primary sequence and tentative residues (in parentheses) as shown in TABLE 1, below, and designated therein as "Pep-1" (SEQ ID NO:11), "Pep-2" (SEQ ID NO:12), "Pep-3" (SEQ ID NO:13), and "Pep-4" (SEQ ID NO:14). Occasional secondary signals were observed and were probably due to the presence of minor contaminants (data not shown). The partial N-terminal sequences shown in TABLE 1 were used to design probes and primers to obtain the primary DNA sequences encoding the cross-reactive APP proteins. Sequence homology comparisons suggested that the four proteins recognized by the dominant local antibody response elicited after heterologous rechallenge have not previously been described for APP.

**TABLE 1**

| **N-Terminal Amino acid Sequences of Low Molecular Weight APP Proteins** | | | |
|---|---|---|---|
| **Peptide (SEQ ID NO)** | **Approx. Mol. Wt (kDa)** | **Protein Name** | **Sequence**^{**1**} **(NH**_{**2**} **to COOH)** |
| Pep-1 (11) | 19-20 | Omp20 | Ala-Pro-Val-Gly-Asn-Thr-Phe-Thr-Gly-Val-(Lys)-Val-(Tyr)-Val-Asp-Leu-Thr-Xaa-Val-Ala |
| Pep-2 (12) | 23 | OmpW | His-Gln-Ala-Gly-Asp-Val-Ile-Phe-Arg-Ala-Gly-Ala-Ile-Gly-Val-Ile-Ala-Asn-Ser-Ser-Ser-Asp-Tyr-(Gln)-Thr-(Gln)-Ala-Asp-Val-(Asn/Val)-Leu-Asp-Val-Asn-Asn |
| Pep-3 (13) | 27 | Omp27 | Ala-Glu-lle-Gly-Leu-Gly-(Gly)-Ala-Arg-Glu-(Ser)-(Ser)-Ile-Tyr-Tyr-(Ser)-Lys-His-Lys-Val-Ala-Thr-Asn-Pro-Phe-Leu-Ala-Leu-Asp-Leu |
| Pep-4 (14) | 29 | OmpA | Ala-(Asp/Glu)-Pro-Glu-Asn-Thr-Phe-Tyr-Pro-Gly-Ala-Lys-Val-Xaa-Xaa-(Ser)-Xaa-(Phe)-(His) |

| | | | |
|---|---|---|---|
| ¹Xaa indicates that the amino acid residue at the particular position could not be determined. | | | |

### 7. EXAMPLE: MOLECULAR CLONING OF DNA ENCODING THE APP PROTEINS

### 7.1. Isolation Of Chromosomal DNA And Construction Of Genomic Libraries

Genomic DNA from each of the twelve APP serotypes was separately isolated by either the hexadecyltrimethyl ammonium bromide (CTAB)-proteinase K method (Ausubel *et al.,* 1988, Curr. Protocols Mol. Biol. Wiley Interscience, NY), or the DNA Isolator Genomic DNA Isolation Reagent (Genosys Biotechnologies, Inc., The Woodlands, Texas). The APP DNA was dissolved in TE buffer (10 mM Tris-HCI, pH 8.0, 1 mM EDTA) at < 1 µg/ml and quantitated by UV spectrophotometry.

To facilitate cloning of the APP gene sequences encoding Omp20, OmpW, Omp27, and OmpA, several genomic libraries were constructed. These libraries were specifically modified by ligation of a known sequence (Vectorette II™, Genosys Biotechnologies, Inc., The Woodlands, TX) to the 5' and 3' ends of restricted DNA fragments essentially as recommended by the supplier. Thus, Vectorette libraries were constructed by separately digesting two µg chromosomal DNA FROM APP 7-1 (serotype 7, passage 1) with restriction endonuclease *Bam*HI, *Bg*/II, *Hin*dIII, *Eco*RI, *Dra*I or *Hpa*I at 37°C overnight. The reaction was then spiked with additional fresh restriction enzyme and adjusted to 2 mM ATP, 2 mM DTT final concentration. Vectorette tailing was carried out by addition of T4 DNA Ligase (400 U) plus 3 pMol of the appropriate compatible Vectorette linker (*Bam*HI Vectorette: *Bam*HI, *Bg*/II; *Hin*dIII Vectorette: *Hin*dIII; *Eco*RI Vectorette: *Eco*RI; Blunt Vectorette: *Dra*I, *Hpa*I*).* The mixture was incubated for three cycles at 20°C, 60 min; 37°C, 30 min to complete the tailing reaction, and then adjusted to 200 µl with dH₂0 and stored at -20°C.

### 7.2. Molecular Cloning Of omp20

Screening of the Vectorette libraries was carried out to obtain DNA fragments encoding Omp20 and flanking regions. Degenerate oligonucleotide ER49 (SEQ ID NO: 39) was designed based on the N-terminal amino acid sequence of this protein (TABLE 1, Pep-1 (SEQ ID NO:11), aa 1-9).

For PCR amplification of a fragment of the *omp20* gene, oligonucleotide ER49 (SEQ ID NO: 39) was used in combination with a Vectorette specific primer, ER70 (SEQ ID NO: 48) in 50 µl reactions containing 1x PCR Buffer II (Perkin Elmer), 1.5 mM MgCl₂, 200 µM each deoxy-NTP, 100 pMol each primer, and 2.5 U AmpliTaq Gold (Perkin Elmer) thermostable polymerase. Multiple single reactions were performed with 5 µl of the Vectorette libraries as DNA template. Amplification was carried out as follows: denaturation (95°C, 9 min); 35 cycles of denaturation (95°C, 30 sec), annealing (55°C, 1 min), and polymerization (72°C, 3 min); followed by a final extension (72°C, 7 min).

The amplified products were visualized by separation on a 1.2% agarose gel (Sigma). A 433-bp product resulted from amplification of the *Eco*RI Vectorette library. The fragment was cloned into pGEM®-T Easy PCR cloning vector (Promega, Madison, WI) and sequenced. Analysis of the sequence confirmed the identity of the fragment as partially encoding Omp20 based on the N-terminal amino acid sequence (Pep-1).

Based on the newly identified sequence of this partial gene, specific primers ER67 (SEQ ID NO: 46) and ER68 (SEQ ID NO: 47) were designed to obtain additional 5' and 3' flanking sequences by a second round of screening the Vectorette libraries by PCR amplification as described above. Screening of *Eco*RI, *Hin*dIII, *Dra*l, and *Hpa*l Vectorette libraries by PCR, employing ER68 (SEQ ID NO: 47) plus ER70 (SEQ ID NO: 48), resulted in successful amplification of an approximately 600-bp fragment from the *Dra*l Vectorette library. This fragment was sequenced to determine the 3' end of the *omp20* gene. Since no unique products were observed during screening of these libraries using ER67 (SEQ ID NO: 46) plus ER70 (SEQ ID NO: 48), additional specific primers ER71 (SEQ ID NO: 49), ER72 (SEQ ID NO: 50), ER76 (SEQ ID NO:52), and ER77 (SEQ ID NO:53) were designed to obtain DNA fragments located 5' of the ER49 binding site. Such "genome walking" by amplification from numerous Vectorette DNA libraries was reiterated until the outer boundaries of the ORF, *i.e.,* translational start and stop codons as well as flanking nucleotide sequences, were characterized. Generally, PCR products were sequenced directly or cloned into pGEM®-T Easy PCR cloning vector prior to sequence analysis.

### 7.3. Molecular Cloning Of omp27

Screening of the Vectorette libraries for *omp27* was carried out essentially as described above for *omp20,* except that degenerate oligonucleotide ER50 (SEQ ID NO:40), which was designed based on the N-terminal amino acid sequence of the purified Omp27 protein (TABLE 1, Pep-3 (SEQ ID NO:13), aa 13-24), was used. Following PCR amplification of Vectorette libraries as described above, a 152-bp fragment was confirmed to encode a portion of Omp27 based on the N-terminal amino acid sequence of Pep-3 (SEQ ID NO:13). A second round of genome walking using *Bam*HI, *Bg*/II*, Hin*dIII, *Eco*RI, *Hpa*I, and *Dra*I Vectorette libraries and specific primers ER88 (SEQ ID NO:64), and ER89 (SEQ ID NO:65) resulted in PCR amplification of an approximately 2 kb fragment from the *Dra*I library, and an approximately 1.5 kb fragment from the *Hin*dIII library. These DNA fragments were cloned into pGEM®-T Easy PCR cloning vector, which allowed for derivation of the nucleotide sequence 5' and 3' of the *omp27* gene, respectively.

### 7.4. Molecular Cloning Of ompA1 And ompA2

The N-terminal amino acid sequence obtained from the purified 29 kDa protein (TABLE 1, Pep-4 (SEQ ID NO:14)) was used to design degenerate N-terminal primers RA22 (SEQ ID NO: 73) and RA34 (SEQ ID NO:77) for use in direct PCR amplification of the gene encoding the 29 kDa protein from APP chromosomal DNA. The sequence of Pep-4 was analyzed using a BlastP computer program comparison (National Center for Biotechnology Information) against the GenBank protein database (Altschul *et al.,* 1990, J. Mol. Biol. 215:403-10.), and was found to exhibit homology with OmpA proteins of several different eubacteria, such as *Pasteurella multocida, P. haemolytica, Haemophilus ducreyi, H. somnus, H. influenzae, Actinobacillus actinomycetemcomitans, E. coli, Shigella,* and *Salmonella.* N-terminal sequences of the OmpA-related proteins from the Pasteurellaceae were aligned to produce the consensus sequence Ala-Pro-Gln-Ala/Glu-Asn-Thr-Phe-Tyr-Ala/Val-Gly-Ala-Lys-Ala (SEQ ID NO:94). These alignments were analyzed and used to design several additional N-terminal degenerate primers. Oligonucleotide primers RA53 (SEQ ID NO:81), RA54 (SEQ ID NO:82), RA55 (SEQ ID NO:83), RA56 (SEQ ID NO:84), and RA57 (SEQ ID NO:85) overlap each other, and were designed to bind to the region encoding aa 4-11, aa 5-12, aa 3-10, aa 1-8, and aa 1-7, respectively, of this consensus peptide.

Other degenerate oligonucleotide primers were designed following alignment of the C-terminal regions of the OmpA-related proteins. This alignment indicated a highly conserved region near the C-terminus which included the amino acid sequence Cys-Leu-Ala-Pro-Asp-Arg-Arg-Val-Glu-Ile (SEQ ID NO:95). Both RA49 (SEQ ID NO:78) and RA50 (SEQ ID NO:79) reverse primers were designed to bind to the negative DNA strand in this region of OmpA. These reverse primers were applied in a two-dimensional matrix in which RA49 (SEQ ID NO:78) and RA50 (SEQ ID NO:79) were each combined pairwise with RA22 (SEQ ID NO:73), RA34 (SEQ ID NO:77), RA53 (SEQ ID NO:81), RA54 (SEQ ID NO:82), RA55 (SEQ ID NO:83), RA56 (SEQ ID NO:84), and RA57 (SEQ ID NO:85) in HotStart 50™ tubes (Molecular Bioproducts Inc., San Diego, CA) with combined Klen*Taq*1 and *Pfu* polymerases. The following references describe "hot start" methods: D'Aquila *et al.,* 1991, Nucl. Acids Res. 19:3749; and Horton *et al.,* 1994, Biotechniques 16:42-43. The cycling program for PCR was a variant of "touchdown PCR" protocols and was carried out as follows: denaturation (94°C, 5 min); 30 cycles of denaturation (94°C, 30 sec), annealing (59°C, 30 sec initial cycle, then -0.1°C per additional cycle), and polymerization (72°C, 1 min); followed by a final extension (72°C, 15 min). The following references describe "touchdown PCR" protocols: Roux, 1994, BioTechniques, 16:812-814; and Hecker and Roux, 1996, BioTechniques 20:478-485.

Among the PCR products generated, an approximately 950-bp band was produced from reactions with either RA49 (SEQ ID NO:78) or RA50 (SEQ ID NO:79), when each was combined pairwise with the forward primers RA34 (SEQ ID NO:77), RA53 (SEQ ID NO:81), RA56 (SEQ ID NO:84), or RA57 (SEQ ID NO:85). These DNA fragments were cloned into pGEM®-T Easy PCR cloning vector and sequenced. Analysis of these sequenced fragments indicated the existence of two different variant sequences. Products derived from RA49 (SEQ ID NO:78)/RA57 (SEQ ID NO:85), and RA50 (SEQ ID NO:79)/RA56 (SEQ ID NO:84), represented variant A1, and the partially encoded protein was designated as "OmpA1." Products derived from RA50 (SEQ ID NO:79)/RA34 (SEQ ID NO:77), and RA50 (SEQ ID NO:79)/RA53 (SEQ ID NO:81) represented variant A2, and the partially encoded protein was designated as "OmpA2."

The two similar yet distinct *ompA* partial DNA sequences were expanded to include the entire ORFs and flanking 5' and 3' sequences through genome walking by application of the previously described Vectorette libraries. Alignment of the partial *ompA1* and *ompA2* DNA sequences allowed the design of specific oligonucleotide primers capable of differentiating these closely related gene sequences. Outward facing, differentiating primers specific for the 5' or 3' regions of *ompA1, i.e.,* ER55 (SEQ ID NO:41), ER58 (SEQ ID NO:42), and for *ompA2, i.e.,* ER59 (SEQ ID NO:43), ER62 (SEQ ID NO:44), respectively, were used to probe Vectorette libraries as described above. Unique fragments of approximately 1100, 400, 450, and 280-bp were obtained from an *Eco*RI Vectorette library when probed with ER70 (SEQ ID NO:48) plus either ER55 (SEQ ID NO:41), ER58 (SEQ ID NO:42), ER59 (SEQ ID NO:43), or ER62 (SEQ ID NO:44), respectively. Sequence analysis of the resulting fragments allowed determination of the endpoints and flanking regions of both *ompA1* and *ompA2* ORFs.

### 7.5. Molecular Cloning Of ompW

The N-terminal amino acid sequence obtained from the purified 23 kDa protein (TABLE 1, Pep-2 (SEQ ID NO:12)) was used to design RA20 (SEQ ID NO:72), a degenerate oligonucleotide primer corresponding to amino acids 1-8 of Pep-2.

Purified APP DNA was used as a template in a variant of "gene walking PCR" methods. The single PCR primer RA20 (SEQ ID NO:72) was used in 'hot start' reactions with combined Klen*Taq*1 (Ab Peptides, Inc, St. Louis, MO.) and *Pfu* (Stratagene, Inc., La Jolla, CA) polymerases. The cycling program for PCR was a variant of "touchdown PCR" protocols and was carried out as follows: denaturation (94°C, 5 min); 40 cycles of denaturation (94°C, 1 min), annealing (63°C, 2 min initial cycle, then -0.2°C and -2 sec per additional cycle), and polymerization (72°C, 1.5 min); followed by a final extension (72°C, 10 min).

Among the numerous PCR products generated, an approximately 220-bp product was obtained and cloned into pGEM®-T Easy PCR cloning vector. Sequence analysis of this plasmid insert confirmed that the cloned PCR product encoded amino acids corresponding to a portion of the 23 kDa protein based on the N-terminal amino acid sequence of Pep-2 (SEQ ID NO:12).

Based on this newly identified sequence, a specific primer, RA23 (SEQ ID NO:74), was generated for the amplification of downstream sequences. Genomic mini-libraries of APP serotype-7 DNA were constructed by limited digestions with *Taq*^{α}I or *Hin*P1 I, which both create 5'-CG overhangs. This DNA was ligated into a *Bsp*DI-cut pUC21 or pUC128 vector and transformed into *E. coli* DH5α to yield the genomic libraries.

Mini-preps of these plasmid-borne genomic libraries were used as templates in "gene walking PCR" using "hot start" methods. The specific primer RA23 (SEQ ID NO:74), along with vector-specific M13 Forward and M13 Reverse sequencing primers, was used with combined Klen*Taq* and *Pfu* polymerases. The cycling program for PCR was carried out as follows: denaturation (94°C, 5 min); 32 cycles of denaturation (94°C, 30 sec), annealing (63°C, 30 sec initial cycle, then -0.2°C per cycle), and polymerization (72°C, 30 sec); followed by a final extension (72°C, 7 min).

Among the numerous PCR products generated, a 0.8 kb product and a 1.4 kb product were cloned into pGEM®-T Easy PCR cloning vector and sequenced. Analysis and alignments of the resulting sequences along with that obtained above yielded the sequence of the mature protein. In order to obtain the sequence of the 5' flanking region of the *ompW gene,* specific primers RA24 (SEQ ID NO:75) and RA26 (SEQ ID NO:76) were used to probe numerous Vectorette libraries, as described above. Amplification was carried out as follows: denaturation (95°C, 9 min); 40 cycles of denaturation (95°C, 30 sec), annealing (60°C, 1 min), and polymerization (72°C, 3 min); followed by a final extension (72°C, 7 min). Specific 600 and 700-bp products resulted from probing the *Hpa*l and *Dra*l Vectorette libraries, respectively. The 700-bp product was directly sequenced to obtain the nucleotide sequence encompassing the 5'-flanking region, and encoded the N-terminus of the 23 kDa protein. Due to partial similarity between the predicted APP 23 kDa protein and the predicted *Vibrio cholerae* OmpW protein, the APP gene fragment was designated as "*ompW*."

### 7.6. Molecular Analysis Of Genes Encoding APP Proteins

### 7.6.1. Specific PCR Amplification Of DNA

Results from the cloning and preliminary sequencing of the novel APP proteins, as described above, were used to design oligonucleotide primers for the specific amplification of the intact *omp20, omp27, ompA1, ompA2* and *ompW* genes directly from APP serotype-7 chromosomal DNA. This approach was preferred based on the desire to eliminate the introduction of sequencing artifacts due to possible mutations arising during the cloning of gene fragments in *E. coli.* Accordingly, oligonucleotides which flank the above intact APP genes were used to specifically amplify those regions from chromosomal DNA. The 5' and 3' primer pairs utilized for each gene amplification were as follows: for *omp20,* the primers were ER80 (SEQ ID NO:56) and ER81 (SEQ ID NO:57); for *omp27,* the primers were ER95 (SEQ ID NO:69) and ER96 (SEQ ID NO:70); for *ompA1,* the primers were ER84 (SEQ ID NO:60) and ER86 (SEQ ID NO:62); for *ompA2,* the primers were ER87 (SEQ ID NO:63) and ER66 (SEQ ID NO:45); and for *ompW,* the primers were ER82 (SEQ ID NO:58) and ER83 (SEQ ID NO:59). PCR reactions were carried out in triplicate and contained 260 ng purified chromosomal DNA, 1x PC2 buffer (Ab Peptides, Inc.), 200 µM each dNTP, 100 pMol each primer, 7.5 U Klen*Taq*1 and 0.15 U cloned *Pfu* thermostable polymerases in a 100 µl final sample volume. Conditions for amplification consisted of denaturation (94°C, 5 min) followed by 30 cycles of denaturation (95°C, 30 sec), annealing (65°C, 30 sec), and polymerization (72°C, 2 min). A final extension (72°C, 7 min) completed the amplification of the target intact gene region. Following amplification, each of the triplicate samples were pooled and the specific product was purified by agarose gel electrophoresis and extraction with spin chromatography (QlAquick™, QIAGEN Inc., Santa Clarita, CA) prior to direct sequence analysis using DyeDeoxy termination reactions on an ABI automated DNA sequencer (Applied Biosystems, Foster City, CA).

Synthetic oligonucleotide primers were used to sequence both DNA strands of the amplified products from APP serotype-7. The primers employed for sequencing the APP protein genes are presented below in TABLE 2.

The nucleotide sequence of the ORF of *omp20 is* presented in SEQ ID NO:1 from nt 272 to 790. The nucleotide sequence of the ORF of *ompW* is presented in SEQ ID NO:3 from nt 376 to 1023. The nucleotide sequence of the ORF of *omp27 is* presented in SEQ ID NO:5 from nt 157 to 933. The nucleotide sequence of the ORF of *ompA1* is presented in SEQ ID NO:7 from nt 614 to 1708. The nucleotide sequence of the ORF of *ompA2* is presented in SEQ ID NO:9 from nt 197 to 1306.

**TABLE 2**

| **APP Gene** | **Primer** | **(SEQ ID NO)** |
|---|---|---|
| *omp20* | ER79 | (55) |
| | ER80 | (56) |
| | ER81 | (57) |
| | AP19.1 | (15) |
| | AP19.2 | (16) |
| | AP19.3 | (17) |
| | AP19.4 | (18) |
| | AP19.5 | (19) |
| *omp27* | ER88 | (64) |
| | ER89 | (65) |
| | ER91 | (66) |
| | ER94 | (68) |
| | ER95 | (69) |
| | ER96 | (70) |
| *ompA1* | ER84 | (60) |
| | ER85 | (61) |
| | ER86 | (62) |
| | AP21.1 | (24) |
| | AP21.2 | (25) |
| | AP21.3 | (26) |
| | AP21.4 | (27) |
| | AP21.5 | (28) |
| | AP21.6 | (29) |
| | AP21.7 | (30) |
| | AP21.8 | (31) |
| | AP21.9 | (32) |
| *ompA2* | ER66 | (45) |
| | ER87 | (63) |
| | AP22.1 | (33) |
| | AP22.2 | (34) |
| | AP22.3 | (35) |
| | AP22.4 | (36) |
| | AP22.5 | (37) |
| | AP22.6 | (38) |
| *ompW* | ER82 | (58) |
| | ER83 | (59) |
| | AP20.1 | (20) |
| | AP20.2 | (21) |
| | AP20.3 | (22) |
| | AP20.4 | (23) |

### 7.6.2. Similarity Of APP Serotype-7 OmpA1 And OmpA2 Proteins

The amino acid sequences of the OmpA1 protein (SEQ ID NO:8) and the OmpA2 protein (SEQ ID NO:10) were deduced from SEQ ID NOS:7 and 9, respectively, and were aligned to compare their similarity. The deduced OmpA1 protein is 364 amino acids in length, which is 5 amino acids shorter than the deduced OmpA2 protein. The alignment of the APP proteins shown in FIGURE 5 indicates that the two proteins share 73.1% (270/369) amino acid identity.

### 7.6.3. Comparison Of APP Serotype-7 OmpW Protein To Vibrio cholerae OmpW

The amino acid sequence (SEQ ID NO:4) deduced from the nucleotide sequence (SEQ ID NO:3) of the ORF encoding the 23 kDa APP OmpW protein was determined to be most similar to the OmpW protein described for *Vibrio cholerae* (Jalajakumari, M. B., *et al.,* 1990, Nucleic Acids Res. 18(8):2180). The amino acid sequences of these two proteins were aligned using the Clustal W (ver 1.4) multiple sequence alignment algorithm (Thompson, J. D., *et al.,* 1994, Nucleic Acids Res., 22:4673-4680). This comparison indicated that the APP OmpW and *V. cholerae* OmpW proteins, which are 215 and 216 residues in length, respectively, share 44.9% (97/216) amino acid identity. The aligned proteins are shown in FIGURE 6.

### 7.7. Southern Blot Hybridizations

The conservation of DNA sequences encoding the Omp20, OmpW, Omp27, OmpA1 and OmpA2 proteins among different APP serotypes was determined by performing Southern blot hybridization analysis using each of the 5 different coding sequences as probes against APP DNA from the different serotypes. Probes were generated with a PCR DIG™ Probe Synthesis Kit (Boehringer Mannheim, Inc., Indianapolis, Indiana) according to manufacturer's instructions. For example, the *ompW* probe was constructed in the following manner. A PCR product encompassing the *ompW* coding sequence was generated using *ompW* specific primers and APP serotype-7 DNA. APP serotype-7 genomic DNA (0.2 µg), 1 µM MW3 primer (SEQ ID NO:71), 1 µM primer RA52 (SEQ ID NO:80), 7.5 U KlenTaq1 polymerase (Ab Peptides, Inc.), 0.075 U *Pfu* polymerase (Stratagene), 1x KlenTaq1 buffer, and 0.2 mM dNTPs were combined in a 50 µl volume. PCR was carried out as follows: denaturation (94°C, 5 min); 35 cycles of denaturation (94°C, 30 sec), annealing (58°C, 30 sec), and polymerization (72°C, 1 min); and final extension (72°C for 7 min). The ∼650-bp *ompW* PCR product was purified following agarose gel electrophoresis using a JETsorb™ kit (GENOMED, Inc., Research Triangle Park, NC). The purified DNA was quantitated using a Low Mass DNA Ladder™ mass standard (GIBCO/BRL, Gaithersburg, MD). A digoxigenin-labeled probe was generated by PCR amplification of 24 pg of the *ompW* PCR product, produced as described above, using a PCR DIG™ Probe Synthesis Kit according to manufacturer's instructions, and the PCR-generated probe was stored unpurified at -20°C.

*Eco*RI-digested APP genomic DNA (1.5 µg) obtained from each of APP serotypes 1, 2, 5, 7, 8 and 9 was separated by agarose gel electrophoresis. The DNA profiles were transferred to Hybond-N 0.45 µm nylon membrane (Amersham, Inc., Cleveland, OH) using alkaline transfer with a Turboblotter™ Kit (Schleicher & Schuell, Inc., Keene, New Hampshire), according to manufacturer's instructions. The DNA was covalently bound to the membrane by UV irradiation using a Stratalinker™ UV Cross-Linker (Stratagene) at the auto-crosslink setting (120 mJ/cm²). The blots were allowed to dry and were stored at rm temp.

Nylon DNA blots were incubated in the presence of probe to allow hybridization for detection of probe sequences in multiple APP serotypes. Blots were pre-hybridized for 2.5 hr at 68°C using an excess (0.2 ml/cm²) of 1x Prehybridization Solution (GIBCO/BRL). Probe-hybridization solution was prepared by adding 5.4 µl of the unpurified digoxigenin-labeled probe to 500 µl of 1x Hybridization Solution (GIBCO/BRL) and boiling at 100°C for 10 min. The probe was cooled to 0°C for 1 min, and then added to sufficient 1x Hybridization Solution to give a total of 0.025 ml/cm² of blot. Blots were hybridized in this probe-hybridization solution mixture at 68°C for 16 hours. Stringency washes were carried out on blots as follows: (i) 2 washes with an excess (0.2 ml/cm²) of 2x SSC/0.1% SDS at 25°C for 5 min; (ii) 2 washes with an excess (0.2 ml/cm²) of 0.1x SSC/0.1% SDS at 68°C for 15 min. Blots were then developed using a chemiluminescence method with a DIG™ High Prime DNA Labeling and Detection Starter Kit II and a DIG™ Wash and Block Buffer Set (Boehringer Mannheim, Inc., Indianapolis, Indiana), according to manufacturer's instructions. Developed blots were exposed to X-ray film for varying lengths of time to detect hybridizing bands.

Probes generated as above against *omp20, ompW, omp27, ompA1* and *ompA2* sequences hybridized with DNA in all the APP serotypes tested (serotypes 1, 2, 5, 7, 8 and 9). The sizes of the *Eco*RI bands detected were identical across all serotypes for *ompA1* and *ompA2,* but were not conserved for *omp20, ompW*, and *omp27.*

The size of the *Eco*RI fragments hybridized by the *omp20* probe in each serotype was as follows: serotypes-1, 2, 7 and 9 gave a 5.8 kb fragment; serotype-5 gave a 6.1 kb fragment; serotype-8 gave a 5.0 kb fragment.

The size of the *Eco*RI fragments hybridized by the *ompW* probe in each serotype was as follows: serotype-1 gave a 1.15 kb fragment; serotype-2 gave a 1.1 kb fragment; serotype-5 gave a 1.0 kb fragment; serotype-7 gave a 0.9 kb fragment; serotype-8 gave a 1.05 kb fragment; and serotype-9 gave a 1.2 kb fragment.

The size of the *Eco*RI fragments hybridized by the *omp27* probe in each serotype was as follows: serotypes-1, 2 and 9 gave an approximately 9.5 kb fragment; serotypes-5, 7 and 8 gave an approximately 10.5 kb fragment.

The size of the *Eco*RI fragments hybridized by the *ompA1* probe was 2.3 kb in all serotypes. Weaker hybridizing fragments of 0.55 kb and 0.85 kb were also detected.

The size of the *Eco*RI fragments hybridized by the *ompA2* probe was 0.85 kb in all serotypes. Weaker hybridizing fragments of 0.55 kb and 2.3 kb were also detected.

### 8. EXAMPLE: EXPRESSION OF RECOMBINANT APP PROTEINS

### 8.1. Host Strain

The *E*. *coli* host used for recombinant protein expression was *E. coli* LW14. The genotype of this strain is λ⁻ IN(*rrnD-rrnE)*1 *galE::*Tn*10 λcl*857*ΔH1 bio.* This strain was provided by SmithKline Beecham Pharmaceuticals, King of Prussia, PA, USA, and contains the temperature-sensitive λ repressor λ*cl*857 which inhibits expression from λ promoters at 30°C. At 42°C, the repressor is inactivated and expression from λ promoters is enabled, yielding high-level transcription and protein synthesis. *E. coli* LW14 was propagated at 30°C.

### 8.2. Plasmid Expression Vectors

The expression vector used for recombinant protein synthesis was pEA181, alternatively designated pEA181KanRBS3. This vector is 6.766 kb in size, encodes kanamycin resistance (*kan*), and contains the strong λ promoter *p*_{L}. The vector contains an *Nde*l site just downstream of an optimized ribosome-binding site; the presence of this *Nde*l site allows for the precise placement of the Met start codon of a protein for optimal expression. The vector also encodes an NS1 leader fusion protein to enable enhanced expression of poorly expressed proteins. This vector was provided by SmithKline Beecham Pharmaceuticals (see also U.S. Patent 4,925,799, and Rosenberg *et al.,* 1983, Meth. Enzymol. 101:123-138).

The coding sequences of each of the five APP proteins were amplified by PCR using 5' specific primers designed to yield an *Nde*l site (CATATG) overlapping the Met start codon (ATG), and 3' specific primers designed for cloning into the 3' *Xba*l site in pEA181. The respective PCR products were initially cloned into the pGEM®-T Easy PCR cloning vector (Promega Corp), and transformed into commercially available competent *E. coli* DH5α (MAX Efficiency DH5α Competent Cells, GIBCO/BRL). The PCR products were excised from these plasmid clones as *Nde*l-*Xba*l or *Nde*l-*Spe*l fragments and cloned into *Nde*l/*Xba*l cut pEA181. Due to the presence of the strong λ promoter *p*_{L}, pEA181 derivatives could be transformed only into *E. co/i* LW14 which repressed expression from the vector by the activity of the temperature-sensitive lambda repressor λ*cl*857. Transformation and propagation of transformants bearing pEA181 and its derivatives were done at 30°C. *E. coli* LW14 was made competent by the method of Hanahan for the preparation of frozen competent cells (Hanahan, 1985, In: DNA Cloning: A Practical Approach (Glover, D., ed.) Vol 1, pp. 109-135, IRL Press, Oxford, England).

Due to difficulties with expression of mature OmpW in *E. coli* LW14, a leader peptide allowing enhanced protein synthesis was employed. This leader peptide, termed a "protective peptide" or "pp", protects recombinant proteins from proteolytic degradation, as based upon information from Sung *et al.,* 1986, Proc. Natl. Acad. Sci. USA 83:561-565; Sung *et al.,* 1987, Meth. Enzymol. 153:385-389; and U.S. Patent 5,460,954, which references are incorporated herein by reference. The protective peptide, which consists of the amino acid sequence Met-Asn-Thr-Thr-Thr-Thr-Thr-Thr-Ser-Arg (SEQ ID NO:96), was fused to the N-terminus of each of the APP proteins by designing PCR primers to contain the protective peptide coding sequence upstream from the APP coding sequence. Amplification of the separate APP coding sequences with such primers generated sequences encoding the N-terminal protective peptide fused to the first amino acid in the mature (*i.e.,* lacking the native signal sequence) APP protein. An *Nde*l site was positioned at the Met codon in the protective peptide so that it could be ligated into the *Nde*l site of pEA181. The primer pairs for the amplification of the protective peptide-APP protein coding sequences were as follows: for *ompW,* MW3 (SEQ ID NO:71) and RA52 (SEQ ID NO:80); for *ompA1,* RA78 (SEQ ID NO:88) and RA71 (SEQ ID NO:87); for *ompA2,* RA78 (SEQ ID NO:88) and RA69 (SEQ ID NO:86); for *omp20,* ER78 (SEQ ID NO:54) and ER73 (SEQ ID NO:51); and for *omp27,* ER92 (SEQ ID NO: 67) and ER94 (SEQ ID NO: 68).

### 8.3. Expression Of Recombinant Proteins

The *E. coli* LW14 transformants bearing pEA181 derivatives encoding protective peptide fusions with the respective mature APP proteins OmpA1, OmpA2, Omp20, OmpW and Omp27, were propagated overnight at 30°C in LB Km⁵⁰ (Luria Broth with 50 µg/ml kanamycin sulfate). The cultures were diluted 1:100 into 2X YT Km⁵⁰ medium (1.6% tryptone, 1% yeast extract, 0.5% NaCI, 1.25 mM NaOH, containing 50 µg/ml kanamycin sulfate) and were grown at 30°C until A₆₀₀ was 0.8 to 1.0. The cultures were then shifted to 42°C in a water bath incubator and incubated for 3 to 4 hr.

Wet cells of *E*. *coli* LW14 transformants from a 5 liter fermentation grown in 2X YT Km⁵⁰ medium, and expressing either pp-OmpA1, pp-OmpA2, or pp-OmpW, were harvested by centrifugation. The cells were suspended in 0.1M Tris-HCl, pH 8.0, and lysed in a high pressure homogenizer. Inclusion bodies were collected by centrifugation (12,000 RCF, 30 min), and washed once or twice with 2x RIPA/TET which was in a 5:4 ratio. 2x RIPA is 20 mM Tris (pH 7.4), 0.3 M NaCI, 2.0% sodium deoxycholate, and 2% (v/v) Igepal CA-630™ (Sigma). TET is 0.1 M Tris (pH 8.0), 50 mM EDTA, and 2% (v/v) Triton X-100. The inclusion bodies were dissolved in 5 M guanidine-hydrochloride, adjusted to >1.4 mg/ml protein in 2.5 M guanidine-hydrochloride, and filter-sterilized (0.2 µm). This preparation was used for the vaccination trials as described below.

Wet cells of *E. coli* LW14 transformants from a 5 liter fermentation grown in 2X YT Km⁵⁰ medium, and expressing pp-Omp20, were harvested by centrifugation. The cells were suspended in 25% sucrose - 50 mM Tris-HCI, pH 8.0 with lysozyme (cells dispersed in 0.5 ml sucrose buffer for every 50 ml culture; for each ml of sucrose buffer, 0.125 ml lysozyme solution at 10 mg/ml was added), and sonicated. Inclusion bodies were collected by centrifugation (12,000 RCF, 30 min), washed with 2x RIPA/TET as above, collected again by centrifugation, and washed with 0.1M glycine and Zwittergent 3-14 (Calbiochem) at pH 11. The pH was adjusted to 7.0, and the inclusion bodies were collected by centrifugation (12,000 RCF, 30 min), dissolved in 3.5 M guanidine hydrochloride (final protein concentration, 6.36 mg/ml ), and filter-sterilized (0.2 µm). This preparation was used for the vaccination trials as described below.

Wet cells of *E. coli* LW14 transformants from a 1600 ml flask culture grown in 2X YT Km⁵⁰ medium, as described above, and expressing pp-Omp27, were harvested by centrifugation. The cells were suspended in 25% sucrose - 50 mM Tris-HCI, pH 8.0, with lysozyme, as above, sonicated, washed with 2x RIPA/TET as above, and collected by centrifugation. The inclusion bodies were collected by centrifugation (12,000 RCF, 30 min), dissolved in 5 M guanidine hydrochloride (final protein concentration, 2.46 mg/ml), and filter-sterilized (0.2 µm). This preparation was used for the vaccination trials as described below.

### 9. EXAMPLE: IMMUNOLOGICAL CHARACTERIZATION OF RECOMBINANT APP PROTEINS

### 9.1. Materials And Methods

### 9.1.1. Preparation And Quantification Of APP Whole Cell Antigens For Western Blot

Whole bacterial cell antigens were prepared as described above in Section 6.1.4, except that HP growth medium was substituted for the MM3 medium and the cells were suspended in 10 ml of DPBS instead of 5 ml of DPBS. The protein concentration of each preparation was determined using a BCA Protein Assay kit (Pierce). In brief, each sample was diluted 1/10, 1/20, 1/40, and 1/80 in sterile deionized, distilled water (ddH₂O). BSA (protein standard) was diluted to concentrations ranging from 200 to 800 µg/ml. A 20 µl volume of sample or standard was added to triplicate wells in a 96-well microtitre plate, and 200 µl of Reagent B diluted 1/50 in Reagent A was added to each well. The plate was incubated at 37°C for 30 min. Sample absorbance was determined at 560 nm. The protein concentration for each sample was calculated by extrapolation using the BSA standard curve.

### 9.1.2. Antibodies

The secondary antibodies used for Western blots included alkaline phosphatase-conjugated goat anti-porcine IgG (H+L) and goat anti-mouse IgG (H+L) (Kirkegaard and Perry Laboratories). These antibodies were used to visualize APP protein-specific antibody in serum or supernatant samples by Western blot analysis. Both antibodies were diluted 1/1000 in dilution buffer (PBS, 0.05% Tween 20, 5% skim milk powder) prior to use.

### 9.1.3. Vaccination Protocol

Recombinantly-expressed protein preparations, prepared as described above in Section 8.3 were diluted to 80 µg/ml in DPBS, and then combined 1:1 with 2x concentrated SEAM-1 adjuvant (80 µg/ml Quil A, 16 µg/ml cholesterol, 5% squalene, 1% Span 85, 0.1% vitamin A acetate, 0.1% ethanol, and 0.01% thimerosol). Male CF1 mice were injected s.c. with 0.25 ml of protein/adjuvant preparation equivalent to 10 µg recombinant protein, 10 µg Quil A and 2 µg of cholesterol. Negative (adjuvant) control groups received 0.25 ml of DPBS mixed 1:1 with adjuvant. The mice were vaccinated a second time with the same protein preparation at 20-22 days post-primary vaccination. Two weeks after the second vaccination, animals were anesthetized with CO₂ and bled by either the brachial artery or through cardiac puncture. The serum was separated from each blood sample, and serum pools from mice within the same group were stored at -20°C.

### 9.1.4. Western Blot Analysis

A volume of APP whole bacterial cell lysate (from APP serotype 1, 2, 5, 7, or 9; prepared as described above in Section 9.1.1) corresponding to 10 µg of protein was mixed with water to a final volume of 10 µl, and 2 µl of 5x reducing sample buffer (Pierce) was added. In a similar manner, an aliquot of recombinant protein (see Section 8.3 above) (protein load was variable) was also prepared. Samples were heated for 5 min at 100°C, and the entire volume was loaded into separate wells of a 15-well, 1.5 mm thick, 14% Tris-glycine gel (Novex). Prestained, broad-range molecular weight markers (5 µl/well) (BioRad) were also included in each gel. Separated proteins in selected gels were stained with Coomassie Blue.

Proteins separated by SDS-PAGE were transferred to PVDF membranes (BioRad) at 200 mA constant current for 1.5 hr. The blots were either: (i) incubated directly in blocking buffer (5% skim milk powder and 0.05% Tween 20 in PBS); or (ii) dried at rm temp, stored frozen at -20°C until needed, then rehydrated in methanol, rinsed in water, and subsequently incubated in blocking buffer. Membranes were incubated in blocking buffer (also used as dilution buffer) overnight with gentle agitation. The blocking buffer was removed, and diluted serum or supernatant sample was added to the membrane, followed by a 1 hr incubation at rm temp. After removal of the test sample, membranes were washed 3 times for 5-10 min each time with PBST (PBS with 0.05% Tween 20). Alkaline phosphatase-conjugated anti-murine or anti-porcine IgG (H+L) antibodies were diluted, added to washed membrane, and incubated for 1 hr at rm temp. The membranes were washed with PBST, and the substrate BCIP/NBT (Kirkegaard and Perry Laboratories) was added to the membranes and incubated with gentle agitation until a suitable color reaction developed. The membranes were then rinsed with water to stop the reaction, and dried at rm temp.

### 9.2. Results

The antigenic characteristics of the novel APP proteins were determined using the following three methods. The first method used pig antibody probes, *i.e.*, convalescent pig sera or ASC supernatants, obtained from animals experimentally infected with either APP serotype-1 or serotype-5, or serotype-5 followed by rechallenge with serotype-7, as immunological probes in Western blots containing the recombinantly-expressed APP proteins (TABLE 3). The second method used sera from mice immunized with the recombinantly-expressed APP proteins to probe Western blots containing APP antigens (whole bacterial cell pellets) (TABLE 4). The third method used sera from mice immunized with the recombinantly-expressed APP proteins to probe Western blots containing the recombinantly-expressed APP proteins (TABLE 5). The results of each method are described below.

### 9.2.1. Recognition Of Recombinant APP Proteins By Pig Antibody Probes Generated Against APP Serotypes

Antibody probes (sera or ASC supernatants) were obtained from pigs following experimental challenge with either APP serotype-1, or serotype-5, or serotype-5 followed by rechallenge with serotype-7. The sera were used to originally identify the novel APP proteins (FIGURES 1-4). TABLE 3 summarizes the reactivity of the antibody probes with the recombinantly-expressed APP proteins by Western blotting. ASC probes generated following challenge with serotype-5 and rechallenge with serotype-7 recognized OmpW, OmpA1, OmpA2, and Omp20 recombinant proteins. The ASC probes did not react immunologically with recombinant Omp27. In contrast, sera derived from animals that were challenged with serotype-1, or serotype-5, or serotype-5 followed by rechallenge with serotype-7, only recognized OmpA1 and OmpA2 recombinant proteins. An ASC probe obtained from a non-challenged control pig (No. 780) did not react with any of the recombinantly-expressed proteins. However, an additional non-challenged control pig (No. 779) reacted with all recombinantly-expressed proteins. In addition, serum from a non-challenged control pig (No. 1421) reacted with OmpA1 and OmpA2. These latter two animals were suspected of having been subclinically infected with APP.

### 9.2.2. Recognition Of APP Proteins By Mouse Antisera Generated Against The Recombinant APP Proteins

Antisera from mice immunized with the recombinantly-expressed APP proteins were used to probe Western blots containing APP antigens from bacterial cell pellets. Results are summarized in TABLE 4. Mice immunized with recombinant pp-OmpW, or pp-OmpA1, or pp-OmpA2 produced serum antibodies that recognized specific bands consistent with the predicted molecular weights of the particular APP protein. However, sera from mice immunized with either recombinant Omp20 or Omp27 did not react specifically with the particular native protein in any serotype tested.

### 9.2.3. Recognition Of Recombinant APP Proteins By Mouse Antisera Generated Against The Recombinant APP Proteins

Antisera from mice immunized with the recombinantly-expressed novel proteins were used to probe Western blots containing the recombinant APP proteins. Results are summarized in TABLE 5. Sera from mice immunized with recombinant OmpW, either as a GST or pp fusion protein, recognized recombinant OmpW, OmpA1, and OmpA2 proteins. Sera from mice immunized with either recombinant OmpA1 or recombinant OmpA2 reacted strongly with both OmpA1 and OmpA2 immunogens. Sera from mice immunized with recombinant Omp20 reacted strongly with recombinant Omp20, and to a lesser degree with recombinant OmpA1 and OmpA2. In contrast, sera from mice vaccinated with recombinant Omp27 did not recognize recombinant Omp27, but did react with recombinant OmpA1 and OmpA2. Sera from control mice vaccinated with PBS reacted very weakly with recombinant OmpA1 and OmpA2, and did not recognize recombinant OmpW, Omp20 or Omp27.

In summary, pigs that had been experimentally infected with APP produced local antibodies (ASC probes) that recognized OmpW, OmpA1, OmpA2, and Omp20 recombinant proteins, whereas serum antibodies only reacted with recombinant OmpA1 and OmpA2, as demonstrated by Western blotting (TABLE 3). Serum thus appears to be much more restricted in terms of immunological reactivity than the ASC probes. Neither serum nor ASC probes recognized recombinantly-expressed Omp27. It is possible that Omp27 is not recognized in a Western blot assay due to the denaturing conditions of the assay.

Immunological characterization of recombinant OmpW, OmpA1, and OmpA2 indicates that these proteins can induce serum antibodies that recognize the native proteins (based upon the predicted molecular weight) found in serotypes 1, 2, 5, 7, 8 and 9 (TABLE 4), as well as recognize the recombinantly-derived forms of these proteins (TABLE 5), and where recombinant Omp20 was also recognized.

Sera from mice immunized with recombinant Omp20 or Omp27 were used to probe Western blots containing whole bacterial cell antigens derived from in-vitro grown APP serotypes 1, 2, 5, 7, 8, and 9 (TABLE 4). These sera did not recognize bands consistent with their native form in any of the APP serotypes examined. It is possible that Omp20 and Omp27 represent antigens of APP that are only expressed *in vivo,* and thus would not be present in bacterial cell pellets prepared in the laboratory. Alternatively, these two proteins may have been denatured by the Western blotting procedure and rendered unrecognizable to specific antibodies.

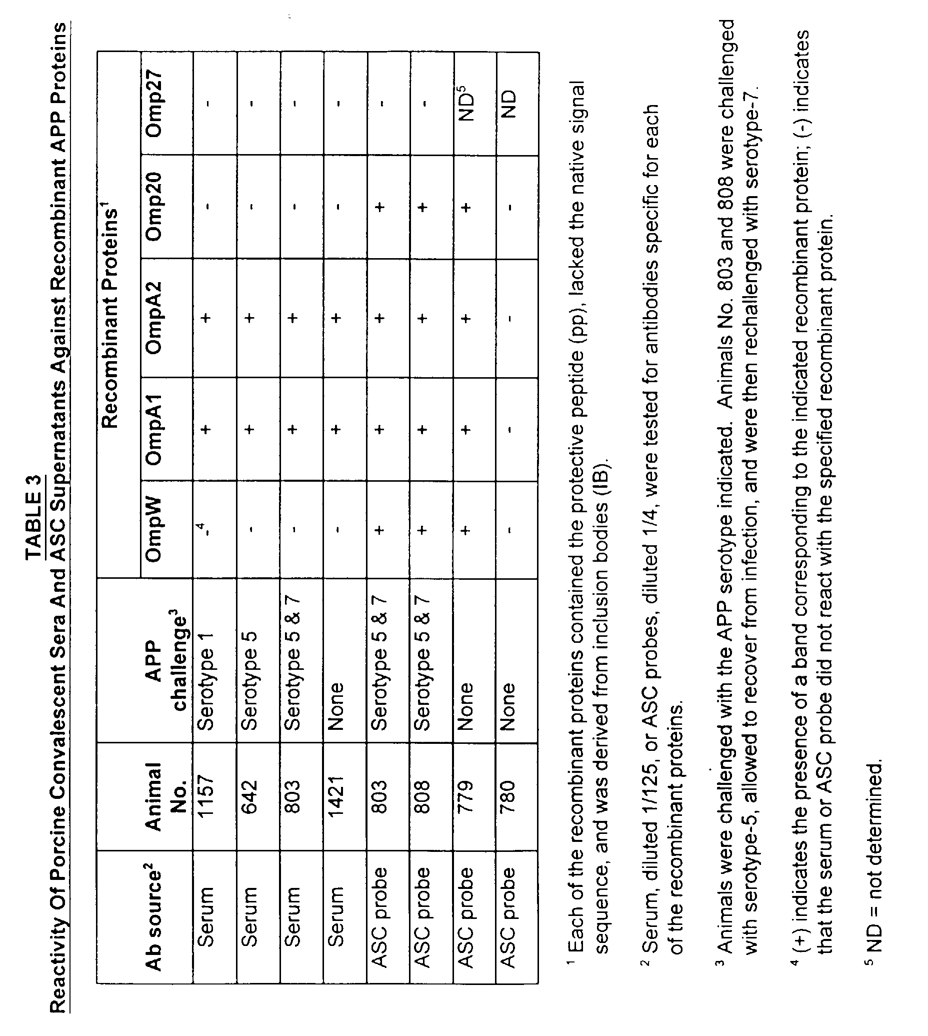

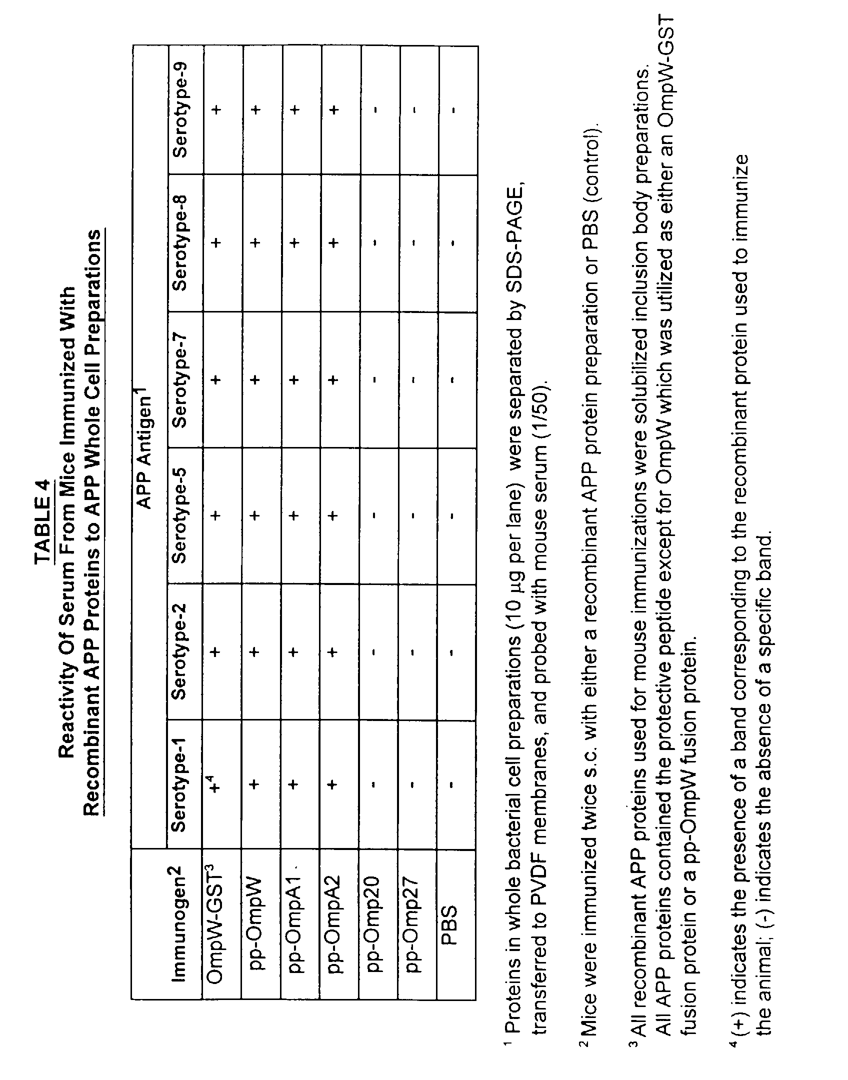

**TABLE 5**

| **Reactivity Of Serum From Mice Immunized With Recombinant APP Proteins Against The Recombinant APP Proteins** | | | | | |
|---|---|---|---|---|---|
| | Antigen¹ | | | | |
| Immunogen² | OmpW³ | OmpA1 | OmpA2 | Omp20 | Omp27 |
| OmpW-GST⁴ | +⁵ | + | + | - | ND⁶ |
| pp-OmpW | + | + | + | - | ND |
| pp-OmpA1 | - | ++ | ++ | - | ND |
| pp-OmpA2 | - | ++ | ++ | - | ND |
| pp-Omp20 | - | + | + | ++ | ND |
| pp-Omp27 | - | + | + | - | - |
| PBS | | +/- | +/- | - | - |

| | | | | | |
|---|---|---|---|---|---|
| ¹ Recombinant proteins separated by SDS-PAGE were transferred to PVDF membranes and probed with mouse serum (1/50). | | | | | |
| ² Mice were immunized twice s.c. with a recombinant protein preparation or PBS (control). | | | | | |
| ³ Each of the recombinant APP proteins in the SDS-PAGE gels contained the protective peptide (pp) and lacked the native signal sequence. | | | | | |
| ⁴ All recombinant APP proteins used for mouse immunizations were from solubilized inclusion body preparations. All proteins contained the protective peptide except for OmpW which was utilized as either an OmpW-GST fusion protein or a pp-OmpW fusion protein, and all proteins lacked the native signal sequence. | | | | | |
| ⁵ (+) indicates that the test serum reacted with the specified recombinant protein; (-) indicates the absence of a specific band; for the PBS control, (+/-) indicates that these bands were visible but very faint as compared to serum from an immunized animal. (++) indicates a very strong immunoreactivity. | | | | | |
| ⁶ ND = not determined. | | | | | |

### EXAMPLE 10: ANIMAL STUDY TO TEST EFFICACY OF VARIOUS ANTIGEN COMBINATIONS

### 10.1 Materials And Methods

Fifty apparently healthy, crossbred pigs (approximately 6.5 weeks of age) were obtained from a herd with no history of APP disease or vaccination. Animals were randomly assigned by litter and by ApxII cytolytic neutralization antibody titer to five groups of 10 pigs (98% of the animals had serum neutralization titers ≤1:200 prior to the initiation of study). Pigs were acclimated for one week prior to initiation of study.

Animals were vaccinated with 2 ml of the appropriate vaccine (APP proteins with pp and without signal sequence) or placebo by the intramuscular route (IM; left neck muscle) on day 0, when pigs were approximately 7.5 weeks of age. After 3 weeks, animals were boosted with a second 2 ml dose (IM; right neck muscle). TABLE 6 identifies the vaccines used for first and second vaccinations of the 5 groups of pigs.

**TABLE 6**

| **Vaccine Group** | **Vaccine Components** |
|---|---|
| A | 75 µg pp-OmpW |
| | 75 µg pp-OmpA1 |
| | 75 µg pp-OmpA2 |
| | 75 µg pp-Omp20 |
| | 75 µg pp-Omp27 |
| | 75 µg rApxl |
| | 75 µg rApxII |
| | 75 µg rOmlA (5) |
| | Adjuvanted with 500 µg |
| | Quil A/200 µg cholesterol |
| B | 75 µg rApxI |
| | 75 µg rApxII |
| | 75 µg rOmIA (5) |
| | Adjuvanted with 500 µg |
| | Quil A/200 µg cholesterol |
| C | 75 µg pp-OmpW |
| | 75 µg pp-OmpA1 |
| | 75 µg pp-OmpA2 |
| | 75 µg pp-Omp20 |
| | 75 µg pp-Omp27 |
| | Adjuvanted with 500 µg |
| | Quil A/200 µg cholesterol |
| D | Commercial Vaccine (whole cell APP bacterin containing serotypes 1, 5, and 7), with Emulsigen® adjuvant |
| E | Phosphate Buffered Saline adjuvanted with 500 µg |
| | Quil A/200 µg cholesterol |

All pigs were observed for approximately 1 hr following vaccinations for vomiting, depression, diarrhea, ataxia-incoordination, increased respiration, and trembling. In addition, daily observations were made for 3 days following first and second vaccinations. Rectal temperatures were recorded one day prior to vaccination, immediately prior to vaccination, 6 hr following vaccination, and 1 day post-vaccination.

Two weeks following the second vaccination, pigs were challenged intranasally with a live virulent culture of APP serotype-1 (ATCC strain 27088) which causes approximately 50% mortality in non-immune pigs. A dose of 1.0 ml (0.5 ml per nostril) of culture containing 1.5 x 10⁷ cfu/ml was used. All animals were anesthetized prior to challenge with an i.m. injection consisting of a combination of 50 mg telazol, 50 mg xylazine, and 50 mg ketamine per ml at the rate of 1.0 ml/ 50 pounds of body weight.

### 10.2. Results

No significant elevations in temperature were seen following first or second vaccinations with the recombinant proteins of the present invention. Significant post-vaccinal site reactions were observed in animals that received the commercial vaccine as compared to all other groups (TABLE 7). None of the animals that received the novel APP proteins alone (Group C), and only 1 animal that received a second vaccination of the novel APP proteins and ApxI/ApxII/OmIA(5) combination (Group A), exhibited post-vaccinal site reactions.

**TABLE 7**

| **Vaccine Group** | **First Vaccination Site Reactions (cm**^{**3**}**)* (% Affected Animals)** | **Second Vaccination Site Reactions (cm**^{**3**}**)*** **(% Affected Animals)** |
|---|---|---|
| A | 0 (0) | 2 (10) |
| B | 2 (10) | 17 (30) |
| C | 0 (0) | 0 (0) |
| D | 16 (30) | 68 (70) |
| E | 0 (0) | 0 (0) |

| | | |
|---|---|---|
| * Numbers represent group average | | |

Group A, which was vaccinated with all of the novel APP proteins plus ApxI/ApxII/OmIA(5), had lower mortality than any other group, including the commercial vaccine (30% vs. 60%) (TABLE 8). The amount of lung damage (% lesions) was also less in Group A as compared to Groups B, C, and controls, but similar to the lung damage seen in animals that received the commercial vaccine (Group D).

**TABLE 8**

| **Vaccine Group** | **Average Lesion (%)** | **Mortality (%)** |
|---|---|---|
| A | 58 | 30 |
| B | 73 | 70 |
| C | 67 | 70 |
| D | 55 | 60 |
| E | 73 | 60 |

These results indicate that a vaccine comprised of the novel APP proteins of the present invention in combination with toxin antigens provides protection against a heterologous APP challenge, which protection is equivalent or superior to that of a commercial vaccine.

### 11. EXAMPLE: PREPARATION OF PLASMIDS AND DEPOSIT MATERIALS

Separate plasmid constructs were prepared encoding each of the APP proteins for deposit with the American Type Culture Collection (ATCC). Each construct contains the total ORF encoding the particular APP protein with its native signal sequence. The ORFs were inserted in the TA cloning site of pCR2.1Topo in the opposite orientation relative to the lactose promoter. The ORFs were obtained by PCR from APP serotype-7 genomic DNA using the primers listed below in TABLE 9. The host cells were E. *coli* Top10. Both host cells and vector are available from Invitrogen (Carlsbad, CA). The 5' primers all begin at the ATG start codon of the respective ORF.

The strains prepared as above, and listed in TABLE 9 below, were deposited with the ATCC at 10801 University Blvd, Manassas, VA, 20110, USA, on October 15, 1998, and were assigned the listed accession numbers.

**TABLE 9**

| **Protein** | **5' Primer (SEQ ID NO)** | **3' Primer (SEQ ID NO)** | **Construct Name** | **Strain Name** | **ATCC Accession No.** |
|---|---|---|---|---|---|
| Omp20 | ER218 (89) | ER73 (51) | pER416 | Pz416 | 98926 |
| Omp27 | ER219 (90) | ER94 (68) | pER417 | Pz417 | 98927 |
| OmpW | ER220 (91) | RA52 (80) | pER418 | Pz418 | 98928 |
| OmpA1 | ER221 (92) | RA71 (87) | pER419 | Pz419 | 98929 |
| OmpA2 | ER222 (93) | RA69 (86) | pER420 | Pz420 | 98930 |

All patents, patent applications, and publications cited above are incorporated herein by reference in their entirety.

The present invention is not to be limited in scope by the specific embodiments described, which are intended as single illustrations of individual aspects of the invention. Functionally equivalent compositions and methods are within the scope of the invention.

## Claims

1. A substantially purified protein which comprises an amino acid sequence selected from the group consisting of about amino acid residue 20 to about amino acid residue 172 of SEQ ID NO:2, about amino acid residue 22 to about amino acid residue 215 of SEQ ID NO:4, about amino acid residue 28 to about amino acid residue 258 of SEQ ID NO:6, about amino acid residue 20 to about amino acid residue 364 of SEQ ID NO:8, and about amino acid residue 20 to about amino acid residue 369 of SEQ ID NO:10.

2. The protein of claim 1, which comprises an amino acid sequence selected from the group consisting of SEQ ID NO:2, SEQ ID NO:4, SEQ ID NO:6, SEQ ID NO:8, and SEQ ID NO:10.

3. A substantially purified polypeptide that is homologous to a protein comprising an amino acid sequence selected from the group consisting of about amino acid residue 20 to about amino acid residue 172 of SEQ ID NO:2, about amino acid residue 22 to about amino acid residue 215 of SEQ ID NO:4, about amino acid residue 28 to about amino acid residue 258 of SEQ ID NO:6, about amino acid residue 20 to about amino acid residue 364 of SEQ ID NO:8, and about amino acid residue 20 to about amino acid residue 369 of SEQ ID NO:10.

4. The polypeptide of claim 3, which is homologous to a polypeptide comprising an amino acid sequence selected from the group consisting of SEQ ID NO:2, SEQ ID NO:4, SEQ ID NO:6, SEQ ID NO:8, and SEQ ID NO:10.

5. A substantially purified polypeptide which is a peptide fragment of: (a) a polypeptide comprising an amino acid sequence selected from the group consisting of about amino acid residue 20 to about amino acid residue 172 of SEQ ID NO:2, about amino acid residue 22 to about amino acid residue 215 of SEQ ID NO:4, about amino acid residue 28 to about amino acid residue 258 of SEQ ID NO:6, about amino acid residue 20 to about amino acid residue 364 of SEQ ID NO:8, and about amino acid residue 20 to about amino acid residue 369 of SEQ ID NO:10; or (b) a polypeptide which is homologous to any of the polypeptides of (a).

6. The polypeptide of claim 5, which is a peptide fragment of a polypeptide comprising an amino acid sequence selected from the group consisting of SEQ ID NO:2, SEQ ID NO:4, SEQ ID NO:6, SEQ ID NO:8, and SEQ ID NO:10.

7. The polypeptide of claim 6, which comprises an amino acid sequence selected from the group consisting of about amino acid residue 1 to about amino acid residue 19 of SEQ ID NO:2, about amino acid residue 1 to about amino acid residue 21 of SEQ ID NO:4, about amino acid residue 1 to about amino acid residue 27 of SEQ ID NO:6, about amino acid residue 1 to about amino acid residue 19 of SEQ ID NO:8, and about amino acid residue 1 to about amino acid residue 19 of SEQ ID NO:10.

8. A fusion protein comprising: (a) a polypeptide comprising an amino acid sequence selected from the group consisting of about amino acid residue 20 to about amino acid residue 172 of SEQ ID NO:2, about amino acid residue 22 to about amino acid residue 215 of SEQ ID NO:4, about amino acid residue 28 to about amino acid residue 258 of SEQ ID NO:6, about amino acid residue 20 to about amino acid residue 364 of SEQ ID NO:8, and about amino acid residue 20 to about amino acid residue 369 of SEQ ID NO:10; (b) a polypeptide that is homologous to a polypeptide of (a); or (c) a peptide fragment of a polypeptide of (a) or (b); joined to a carrier or fusion partner.

9. The fusion protein of claim 8, wherein the fusion partner is selected from the group consisting of a protective peptide, β-galactosidase, trpE, maltose-binding protein, glutathione-S-transferase, and polyhistidine.

10. The fusion protein of claim 9, wherein the fusion partner is a protective peptide that comprises the amino acid sequence Met-Asn-Thr-Thr-Thr-Thr-Thr-Thr-Ser-Arg (SEQ ID NO:96).

11. An analog or derivative of: (a) a polypeptide comprising an amino acid sequence selected from the group consisting of about amino acid residue 20 to about amino acid residue 172 of SEQ ID NO:2, about amino acid residue 22 to about amino acid residue 215 of SEQ ID NO:4, about amino acid residue 28 to about amino acid residue 258 of SEQ ID NO:6, about amino acid residue 20 to about amino acid residue 364 of SEQ ID NO:8, and about amino acid residue 20 to about amino acid residue 369 of SEQ ID NO:10; (b) a polypeptide that is homologous to a polypeptide of (a); (c) a peptide fragment of a polypeptide of (a) or (b); or (d) a fusion protein comprising a polypeptide of (a) or (b), or a peptide fragment of (c), joined to a carrier or fusion partner.

12. An isolated polynucleotide molecule comprising a nucleotide sequence encoding a polypeptide comprising an amino acid sequence selected from the group consisting of about amino acid residue 20 to about amino acid residue 172 of SEQ ID NO:2, about amino acid residue 22 to about amino acid residue 215 of SEQ ID NO:4, about amino acid residue 28 to about amino acid residue 258 of SEQ ID NO:6, about amino acid residue 20 to about amino acid residue 364 of SEQ ID NO:8, and about amino acid residue 20 to about amino acid residue 369 of SEQ ID NO:10.

13. The isolated polynucleotide molecule of claim 12 which encodes the amino acid sequence of about amino acid residue 20 to about amino acid residue 172 of SEQ ID NO:2, comprising the nucleotide sequence of SEQ ID NO:1 from about nt 329 to about nt 790.

14. The isolated polynucleotide molecule of claim 12, encoding the amino acid sequence of SEQ ID NO:2.

15. The isolated polynucleotide molecule of claim 14, comprising the nucleotide sequence of SEQ ID NO:1 from about nt 272 to about nt 790.

16. The isolated polynucleotide molecule of claim 15, comprising the nucleotide sequence of SEQ ID NO:1.

17. The isolated polynucleotide molecule of claim 12 which encodes the amino acid sequence of about amino acid residue 22 to about amino acid residue 215 of SEQ ID NO:4, comprising the nucleotide sequence of SEQ ID NO:3 from about nt 439 to about nt 1023.

18. The isolated polynucleotide molecule of claim 12, encoding the amino acid sequence of SEQ ID NO:4.

19. The isolated polynucleotide molecule of claim 18, comprising the nucleotide sequence of SEQ ID NO:3 from about nt 376 to about nt 1023.

20. The isolated polynucleotide molecule of claim 19, comprising the nucleotide sequence of SEQ ID NO:3.

21. The isolated polynucleotide molecule of claim 12 which encodes the amino acid sequence of about amino acid residue 28 to about amino acid residue 258 of SEQ ID NO:6, comprising the nucleotide sequence of SEQ ID NO:5 from about nt 238 to about nt 933.

22. The isolated polynucleotide molecule of claim 12, encoding the amino acid sequence of SEQ ID NO:6.

23. The isolated polynucleotide molecule of claim 22, comprising the nucleotide sequence of SEQ ID NO:5 from about nt 157 to about nt 933.

24. The isolated polynucleotide molecule of claim 23, comprising the nucleotide sequence of SEQ ID NO:5.

25. The isolated polynucleotide molecule of claim 12 which encodes the amino acid sequence of about amino acid residue 20 to about amino acid residue 364 of SEQ ID NO:8, comprising the nucleotide sequence of SEQ ID NO:7 from about nt 671 to about nt 1708.

26. The isolated polynucleotide molecule of claim 12, encoding the amino acid sequence of SEQ ID NO:8.

27. The isolated polynucleotide molecule of claim 26, comprising the nucleotide sequence of SEQ ID NO:7 from about nt 614 to about nt 1708.

28. The isolated polynucleotide molecule of claim 27, comprising the nucleotide sequence of SEQ ID NO:7.

29. The isolated polynucleotide molecule of claim 12 which encodes the amino acid sequence of about amino acid residue 20 to about amino acid residue 369 of SEQ ID NO:10, comprising the nucleotide sequence of SEQ ID NO:9 from about nt 254 to about nt 1306.

30. The isolated polynucleotide molecule of claim 12, encoding the amino acid sequence of SEQ ID NO:10.

31. The isolated polynucleotide molecule of claim 30, comprising the nucleotide sequence of SEQ ID NO:9 from about nt 197 to about nt 1306.

32. The isolated polynucleotide molecule of claim 31, comprising the nucleotide sequence of SEQ ID NO:9.

33. An isolated polynucleotide molecule that is homologous to a polynucleotide molecule comprising a nucleotide sequence encoding Omp20, OmpW, Omp27, OmpA1 or OmpA2 from APP.

34. An isolated polynucleotide molecule comprising a nucleotide sequence that encodes a polypeptide that is homologous to a protein having an amino acid sequence selected from the group consisting of about amino acid residue 20 to about amino acid residue 172 of SEQ ID NO:2, about amino acid residue 22 to about amino acid residue 215 of SEQ ID NO:4, about amino acid residue 28 to about amino acid residue 258 of SEQ ID NO:6, about amino acid residue 20 to about amino acid residue 364 of SEQ ID NO:8, and about amino acid residue 20 to about amino acid residue 369 of SEQ ID NO:10.

35. An isolated polynucleotide molecule consisting of a nucleotide sequence which is a substantial portion of: (a) a nucleotide sequence encoding a polypeptide comprising an amino acid sequence selected from the group consisting of about amino acid residue 20 to about amino acid residue 172 of SEQ ID NO:2, about amino acid residue 22 to about amino acid residue 215 of SEQ ID NO:4, about amino acid residue 28 to about amino acid residue 258 of SEQ ID NO:6, about amino acid residue 20 to about amino acid residue 364 of SEQ ID NO:8, and about amino acid residue 20 to about amino acid residue 369 of SEQ ID NO:10; (b) a nucleotide sequence that is homologous to a nucleotide sequence of (a); or (c) a nucleotide sequence encoding a polypeptide that is homologous to a polypeptide of (a).

36. An isolated polynucleotide molecule comprising a nucleotide sequence that encodes an amino acid sequence selected from the group consisting of about amino acid residue 1 to about amino acid residue 19 of SEQ ID NO:2, about amino acid residue 1 to about amino acid residue 21 of SEQ ID NO:4, about amino acid residue 1 to about amino acid residue 27 of SEQ ID NO:6, about amino acid residue 1 to about amino acid residue 19 of SEQ ID NO:8, and about amino acid residue 1 to about amino acid residue 19 of SEQ ID NO:10.

37. The isolated polynucleotide molecule of claim 36, wherein the nucleotide sequence is selected from the group consisting of about nt 272 to about nt 328 of SEQ ID NO:1, about nt 376 to about nt 438 of SEQ ID NO:3, about nt 157 to about nt 237 of SEQ ID NO:5, about nt 614 to about nt 670 of SEQ ID NO:7, and about nt 197 to about nt 253 of SEQ ID NO:1.

38. An isolated polynucleotide molecule comprising a nucleotide sequence that encodes a fusion protein comprising: (a) a polypeptide comprising an amino acid sequence selected from the group consisting of about amino acid residue 20 to about amino acid residue 172 of SEQ ID NO:2, about amino acid residue 22 to about amino acid residue 215 of SEQ ID NO:4, about amino acid residue 28 to about amino acid residue 258 of SEQ ID NO:6, about amino acid residue 20 to about amino acid residue 364 of SEQ ID NO:8, and about amino acid residue 20 to about amino acid residue 369 of SEQ ID NO:10; (b) a polypeptide that is homologous to a polypeptide of (a); or (c) a peptide fragment of a polypeptide of (a) or (b); joined to a carrier or fusion partner.

39. The isolated polynucleotide molecule of claim 38, wherein the fusion partner is selected from the group consisting of a protective peptide, β-galactosidase trpE, maltose-binding protein, glutathione-S-transferase, and polyhistidine.

40. The isolated polynucleotide molecule of claim 39, wherein the fusion partner is a protective peptide that comprises the amino acid sequence Met-Asn-Thr-Thr-Thr-Thr-Thr-Thr-Ser-Arg (SEQ ID NO:96).

41. An oligonucleotide molecule that can hybridize under highly stringent conditions to a polynucleotide molecule consisting of a nucleotide sequence selected from SEQ ID NOS:1, 3, 5, 7, or 9, or to a polynucleotide molecule consisting of a nucleotide sequence that is the complement of a nucleotide sequence selected from SEQ ID NOS:1, 3, 5, 7, or 9.

42. The oligonucleotide molecule of claim 41, comprising a nucleotide sequence selected from the group consisting of SEQ ID NOS: 15-47 and 49-93.

43. A recombinant vector, comprising the polynucleotide molecule of claim 12 or a homologous polynucleotide molecule thereof.

44. The recombinant vector of claim 43, comprising a polynucleotide molecule comprising a nucleotide sequence of SEQ ID NO:1 from about nt 329 to about nt 790 or a homologous polynucleotide molecule thereof.

45. The recombinant vector of claim 44, which is a plasmid that is the same as plasmid pER416 present in host cells of strain Pz416 (ATCC 98926).

46. The recombinant vector of claim 43, comprising a polynucleotide molecule comprising a nucleotide sequence of SEQ ID NO:3 from about nt 439 to about nt 1023 or a homologous polynucleotide molecule thereof.

47. The recombinant vector of claim 46, which is a plasmid that is the same as plasmid pER418 present in host cells of strain Pz418 (ATCC 98928).

48. The recombinant vector of claim 43, comprising a polynucleotide molecule comprising a nucleotide sequence of SEQ ID NO:5 from about nt 238 to about nt 933 or a homologous polynucleotide molecule thereof.

49. The recombinant vector of claim 48, which is a plasmid that is the same as plasmid pER417 present in host cells of strain Pz417 (ATCC 98927).

50. The recombinant vector of claim 43, comprising a polynucleotide molecule comprising a nucleotide sequence of SEQ ID NO:7 from about nt 671 to about nt 1708 or a homologous polynucleotide molecule thereof.

51. The recombinant vector of claim 50, which is a plasmid that is the same as plasmid pER419 present in host cells of strain Pz419 (ATCC 98929).

52. The recombinant vector of claim 43, comprising a polynucleotide molecule comprising a nucleotide sequence of SEQ ID NO:9 from about nt 254 to about nt 1306 or a homologous polynucleotide molecule thereof.

53. The recombinant vector of claim 52, which is a plasmid that is the same as plasmid pER420 present in host cells of strain Pz420 (ATCC 98930).

54. A recombinant vector comprising the polynucleotide molecule of claim 34, 35, 36 or 38.

55. A transformed host cell, comprising the recombinant vector of claim 43 or 54.

56. A vaccine against APP, comprising an immunologically effective amount of an antigen of the present invention selected from the group consisting of: (a) a polypeptide comprising an amino acid sequence selected from the group consisting of about amino acid residue 20 to about amino acid residue 172 of SEQ ID NO:2, about amino acid residue 22 to about amino acid residue 215 of SEQ ID NO:4, about amino acid residue 28 to about amino acid residue 258 of SEQ ID NO:6, about amino acid residue 20 to about amino acid residue 364 of SEQ ID NO:8, and about amino acid residue 20 to about amino acid residue 369 of SEQ ID NO:10; (b) a polypeptide which is homologous to the polypeptide of (a); (c) a peptide fragment of the polypeptide of (a) or (b); (d) a fusion protein comprising the polypeptide of (a) or (b) or the peptide fragment of (c) joined to a carrier or fusion partner; (e) an analog or derivative of the polypeptide of (a) or (b), or the peptide fragment of (c), or the fusion protein of (d); or (f) a polynucleotide molecule encoding the polypeptide of (a) or (b), the peptide fragment of (c), the fusion protein of (d), or the analog or derivative of (e); which antigen is capable of inducing, or contributing to the induction of, a protective response against APP in swine; and a veterinarily acceptable carrier or diluent.

57. The vaccine of claim 56, further comprising an immunomodulatory component.

58. The vaccine of claim 57, wherein the immunomodulatory component is an adjuvant.

59. The vaccine of claim 56, further comprising an immunologically effective amount of a different antigen capable of inducing, or contributing to the induction of, a protective response against a disease or pathological condition that can afflict swine.

60. A method of preparing a vaccine that can protect swine against APP, comprising combining an immunologically effective amount of an antigen of the present invention selected from the group consisting of: (a) a polypeptide comprising an amino acid sequence selected from the group consisting of about amino acid residue 20 to about amino acid residue 172 of SEQ ID NO:2, about amino acid residue 22 to about amino acid residue 215 of SEQ ID NO:4, about amino acid residue 28 to about amino acid residue 258 of SEQ ID NO:6, about amino acid residue 20 to about amino acid residue 364 of SEQ ID NO:8, and about amino acid residue 20 to about amino acid residue 369 of SEQ ID NO:10; (b) a polypeptide which is homologous to the polypeptide of (a); (c) a peptide fragment of the polypeptide of (a) or (b); (d) a fusion protein comprising the polypeptide of (a) or (b) or the peptide fragment of (c) joined to a carrier or fusion partner; (e) an analog or derivative of the polypeptide of (a) or (b), or the peptide fragment of (c), or the fusion protein of (d); or (f) a polynucleotide molecule encoding the polypeptide of (a) or (b), the peptide fragment of (c), the fusion protein of (d), or the analog or derivative of (e); which antigen is capable of inducing, or contributing to the induction of, a protective response against APP in swine, with a veterinarily acceptable carrier or diluent in a form suitable for administration to swine.

61. A method of vaccinating swine against APP, comprising administering the vaccine of claim 56 to a pig.

62. A vaccine kit for vaccinating swine against APP, comprising a container comprising an immunologically effective amount of one or more of the antigens of claim 56.

63. The kit of claim 62, further comprising a second container comprising a veterinarily acceptable carrier or diluent.

64. An isolated antibody that specifically binds to a polypeptide comprising an amino acid sequence selected from the group consisting of about amino acid residue 20 to about amino acid residue 172 of SEQ ID NO:2, about amino acid residue 22 to about amino acid residue 215 of SEQ ID NO:4, about amino acid residue 28 to about amino acid residue 258 of SEQ ID NO:6, about amino acid residue 20 to about amino acid residue 364 of SEQ ID NO:8, and about amino acid residue 20 to about amino acid residue 369 of SEQ ID NO:10.

65. A diagnostic kit comprising a first container comprising: (a) a polypeptide comprising an amino acid sequence selected from the group consisting of about amino acid residue 20 to about amino acid residue 172 of SEQ ID NO:2, about amino acid residue 22 to about amino acid residue 215 of SEQ ID NO:4, about amino acid residue 28 to about amino acid residue 258 of SEQ ID NO:6, about amino acid residue 20 to about amino acid residue 364 of SEQ ID NO:8, and about amino acid residue 20 to about amino acid residue 369 of SEQ ID NO:10; (b) a polypeptide which is homologous to the polypeptide of (a); (c) a peptide fragment of the polypeptide of (a) or (b); (d) a fusion protein comprising the polypeptide of (a) or (b) or the peptide fragment of (c) joined to a carrier or fusion partner; or (e) an analog or derivative of the polypeptide of (a) or (b), or the peptide fragment of (c), or the fusion protein of (d), that will specifically bind to porcine antibodies directed against an APP antigen; and a second container comprising a secondary antibody directed against porcine antibodies.

66. A diagnostic kit comprising a first container comprising a primary antibody that binds to an APP protein, said APP protein comprising an amino acid sequence selected from the group consisting of about amino acid residue 20 to about amino acid residue 172 of SEQ ID NO:2, about amino acid residue 22 to about amino acid residue 215 of SEQ ID NO:4, about amino acid residue 28 to about amino acid residue 258 of SEQ ID NO:6, about amino acid residue 20 to about amino acid residue 364 of SEQ ID NO:8, and about amino acid residue 20 to about amino acid residue 369 of SEQ ID NO:10; and a second container comprising a secondary antibody that binds to a different epitope on the APP protein, or that is directed against the primary antibody.

67. A diagnostic kit comprising a container comprising a polynucleotide molecule or oligonucleotide molecule of the present invention that can specifically hybridize to or amplify an APP-specific polynucleotide molecule.


**Annex to the description**

SEQUENCE LISTING

<110> Pfizer Products Inc.

<120> NOVEL PROTEINS FROM ACTINOBACILLUS PLEUROPNEUMONIAE

<130> PC9854A

<140>
<141>

<160> 96

<170> PatentIn Ver. 2.0

<210> 1
<211> 1018
<212> DNA
<213> Actinobacillus pleuropneumoniae

<220>
<221> CDS
<222> (272)..(787)

<400> 1
ggttggaaaa caccttatga agtttacttc aaaaaatcgt tgcacttggt ttgacaattc 60

aagactaaaa atgaccggtc gtgagctaaa accgcatgac cgtactgtgg atgtgacgat 120

tcgtcgtatt cgtaaacact ttgaagatca ccctaataca ccggaaatca ttgtaaccat 180

tcatggtgaa ggttaccgtt tttgcggcga gttagagtag taattaaacg cctataagcg 240

tttagcatct tctttctaaa aaggacattt t atg aaa aat tta aca gtt tta 292
Met Lys Asn Leu Thr Val Leu
1               5

gca tta gca ggt tta ttc tct gcg tcg gca ttt gcc gca ccg gtc gga 340
Ala Leu Ala Gly Leu Phe Ser Ala Ser Ala Phe Ala Ala Pro Val Gly
        10                  15                  20

aat acc ttt acc ggc gta ggc gta ggc gtt gat ctc acc acg gta aaa 388
Asn Thr Phe Thr Gly Val Gly Val Gly Val Asp Leu Thr Thr Val Lys
    25                  30                  35

tat aaa gtg gac ggt gtg aaa ggt aaa caa tca acc ggt cct gcg tta 436
Tyr Lys Val Asp Gly Val Lys Gly Lys Gln Ser Thr Gly Pro Ala Leu
40                  45                  50                  55

gtc gta gat tac ggt atg gat tac ggt gac aat ttt gtc ggt gtt gta 484
Val Val Asp Tyr Gly Met Asp Tyr Gly Asp Asn Phe Val Gly Val Val
                60                  65                  70

caa ggt aaa gta aaa gta ggc agt aca aaa gta ttt agc gat gta aaa 532
Gln Gly Lys Val Lys Val Gly Ser Thr Lys Val Phe Ser Asp Val Lys
            75                  80                  85

caa aaa act aaa tat act gtc gct tat caa caa ggt tat cgt gta gct 580
Gln Lys Thr Lys Tyr Thr Val Ala Tyr Gln Gln Gly Tyr Arg Val Ala
        90                  95                  100

tct gat tta ctt ccg tat gtc aaa gtc gat gtg gcg caa agt aaa gtc 628
Ser Asp Leu Leu Pro Tyr Val Lys Val Asp Val Ala Gln Ser Lys Val
    105                 110                 115

ggc gat acc aat ttc cgt ggt tac ggt tac ggt gcc ggt gct aaa tat 676
Gly Asp Thr Asn Phe Arg Gly Tyr Gly Tyr Gly Ala Gly Ala Lys Tyr
120                 125                 130                 135

gcc gta tca agt aat gta gaa gtg ggt gcg gaa tat acg cgc agc aat 724
Ala Val Ser Ser Asn Val Glu Val Gly Ala Glu Tyr Thr Arg Ser Asn
                140                 145                 150

tta aga aaa agc ggt gct aaa tta aaa ggt aat gaa ttt act gcg aac 772
Leu Arg Lys Ser Gly Ala Lys Leu Lys Gly Asn Glu Phe Thr Ala Asn
            155                 160                 165

cta ggt tac cgt ttc taattatttt tcccttatga caagcggtcg tttcttgcaa 827
Leu Gly Tyr Arg Phe
        170

aaaatttgcg aaaaacgacc gcttattttt ttattaatac tttatttact gagccatttt 887

ttcagctacg gttagaaaac cgtctgcagt cgcatagatt tcttcaaagc cttgcgcttg 947

tagaatacgg tcggacactt cacgaaatgc gccctctcca cctgcctttt ctaatatcca 1007

atccgctttt g 1018

<210> 2
<211> 172
<212> PRT
<213> Actinobacillus pleuropneumoniae

<400> 2
Met Lys Asn Leu Thr Val Leu Ala Leu Ala Gly Leu Phe Ser Ala Ser
1               5                   10                  15

Ala Phe Ala Ala Pro Val Gly Asn Thr Phe Thr Gly Val Gly Val Gly
            20                  25                  30

Val Asp Leu Thr Thr Val Lys Tyr Lys Val Asp Gly Val Lys Gly Lys
        35                  40                  45

Gln Ser Thr Gly Pro Ala Leu Val Val Asp Tyr Gly Met Asp Tyr Gly
    50                  55                  60

Asp Asn Phe Val Gly Val Val Gln Gly Lys Val Lys Val Gly Ser Thr
65                  70                  75                  80

Lys Val Phe Ser Asp Val Lys Gln Lys Thr Lys Tyr Thr Val Ala Tyr
                85                  90                  95

Gln Gln Gly Tyr Arg Val Ala Ser Asp Leu Leu Pro Tyr Val Lys Val
            100                 105                 110

Asp Val Ala Gln Ser Lys Val Gly Asp Thr Asn Phe Arg Gly Tyr Gly
        115                 120                 125

Tyr Gly Ala Gly Ala Lys Tyr Ala Val Ser Ser Asn Val Glu Val Gly
    130                 135                 140

Ala Glu Tyr Thr Arg Ser Asn Leu Arg Lys Ser Gly Ala Lys Leu Lys
145                 150                 155                 160

Gly Asn Glu Phe Thr Ala Asn Leu Gly Tyr Arg Phe
                165                 170


<210> 3
<211> 1188
<212> DNA
<213> Actinobacillus pleuropneumoniae

<220>
<221> CDS
<222> (376)..(1020)

<400> 3
ctaacgcata aagtaaatgt gccggttcaa tgtagttatt atcttttccg atagctaacg 60

attgggcttc tgcaagggct tcttgcaatt tggtagtaaa tttttcgaaa ttcatatttt 120

tactcctaaa tttcattaat ctgtatcgag cagaatttat accgcttcaa cgttttaata 180

aatggagcta gtcgaactta tttcaagtga aaatgtgaaa aagatcgcaa aaaataaatt 240

agtacctcgt tgtaggtact aaaatggcgt atatttgatt cttgtcaata aaagttagcc 300

gaattgttct tagaatgtta ttaacgtaac gaattggtta cttttttatt tttaagaaaa 360

tattaagagg tcaaa atg aaa aaa gca gta tta gcg gca gta tta ggc ggt 411
                 Met Lys Lys Ala Val Leu Ala Ala Val Leu Gly Gly
                 1               5                   10

gcg tta tta gcg ggt tcg gca atg gca cat caa gcg ggc gat gtg att 459
Ala Leu Leu Ala Gly Ser Ala Met Ala His Gln Ala Gly Asp Val Ile
        15                  20                  25

ttc cgt gcc ggt gcg atc ggt gtg att gca aat tca agt tcg gat tat 507
Phe Arg Ala Gly Ala Ile Gly Val Ile Ala Asn Ser Ser Ser Asp Tyr
    30                  35                  40

caa acc ggg gcg gac gta aac tta gat gta aat aat aat att cag ctt 555
Gln Thr Gly Ala Asp Val Asn Leu Asp Val Asn Asn Asn Ile Gln Leu
45                  50                  55                  60

ggt tta acc ggt acc tat atg tta agt gat aat tta ggt ctt gaa tta 603
Gly Leu Thr Gly Thr Tyr Met Leu Ser Asp Asn Leu Gly Leu Glu Leu
                65                  70                  75

tta gcg gca aca ccg ttt tct cac aaa atc acc ggt aag tta ggt gca 651
Leu Ala Ala Thr Pro Phe Ser His Lys Ile Thr Gly Lys Leu Gly Ala
            80                  85                  90

aca gat tta ggc gaa gtg gca aaa gta aaa cat ctt ccg ccg agc ctt 699
Thr Asp Leu Gly Glu Val Ala Lys Val Lys His Leu Pro Pro Ser Leu
        95                  100                 105

tac tta caa tat tat ttc ttt gat tct aat gcg aca gtt cgt cca tac 747
Tyr Leu Gln Tyr Tyr Phe Phe Asp Ser Asn Ala Thr Val Arg Pro Tyr
    110                 115                 120

gtt ggt gcc ggt tta aac tat act cgc ttt ttc agt gct gaa agt tta 795
Val Gly Ala Gly Leu Asn Tyr Thr Arg Phe Phe Ser Ala Glu Ser Leu
125                 130                 135                 140

aaa ccg caa tta gta caa aac tta cgt gtt aaa aaa cat tcc gtc gca 843
Lys Pro Gln Leu Val Gln Asn Leu Arg Val Lys Lys His Ser Val Ala
                145                 150                 155

ccg att gcg aat tta ggt gtt gat gtg aaa tta acg gat aat cta tca 891
Pro Ile Ala Asn Leu Gly Val Asp Val Lys Leu Thr Asp Asn Leu Ser
            160                 165                 170

ttc aat gcg gca gct tgg tac aca cgt att aaa act act gcc gat tat 939
Phe Asn Ala Ala Ala Trp Tyr Thr Arg Ile Lys Thr Thr Ala Asp Tyr
        175                 180                 185

gat gtt ccg gga tta ggt cat gta agt aca ccg att act tta gat cct 987
Asp Val Pro Gly Leu Gly His Val Ser Thr Pro Ile Thr Leu Asp Pro
    190                 195                 200

gtt gta tta ttc tca ggt att agc tac aaa ttc taagtatttt gaaactgtta 1040
Val Val Leu Phe Ser Gly Ile Ser Tyr Lys Phe
205                 210                 215

tgagaaaggg agcgttaatc gctccctttt tgttgtaaaa aatccttgaa aaacgaccgc 1100

ttgttaagca caaaaatgta ggatcatttt agtgagcaat tcacgagtcg gctcaataaa 1160

ttttgtttct aaaaattcat ccggctgg 1188

<210> 4
<211> 215
<212> PRT
<213> Actinobacillus pleuropneumoniae

<400> 4
Met Lys Lys Ala Val Leu Ala Ala Val Leu Gly Gly Ala Leu Leu Ala
1               5                   10                  15

Gly Ser Ala Met Ala His Gln Ala Gly Asp Val Ile Phe Arg Ala Gly
            20                  25                  30

Ala Ile Gly Val Ile Ala Asn Ser Ser Ser Asp Tyr Gln Thr Gly Ala
        35                  40                  45

Asp Val Asn Leu Asp Val Asn Asn Asn Ile Gln Leu Gly Leu Thr Gly
    50                  55                  60

Thr Tyr Met Leu Ser Asp Asn Leu Gly Leu Glu Leu Leu Ala Ala Thr
65                  70                  75                  80

Pro Phe Ser His Lys Ile Thr Gly Lys Leu Gly Ala Thr Asp Leu Gly
                85                  90                  95

Glu Val Ala Lys Val Lys His Leu Pro Pro Ser Leu Tyr Leu Gln Tyr
            100                 105                 110

Tyr Phe Phe Asp Ser Asn Ala Thr Val Arg Pro Tyr Val Gly Ala Gly
        115                 120                 125

Leu Asn Tyr Thr Arg Phe Phe Ser Ala Glu Ser Leu Lys Pro Gln Leu
    130                 135                 140

Val Gln Asn Leu Arg Val Lys Lys His Ser Val Ala Pro Ile Ala Asn
145                 150                 155                 160

Leu Gly Val Asp Val Lys Leu Thr Asp Asn Leu Ser Phe Asn Ala Ala
                165                 170                 175

Ala Trp Tyr Thr Arg Ile Lys Thr Thr Ala Asp Tyr Asp Val Pro Gly
            180                 185                 190

Leu Gly His Val Ser Thr Pro Ile Thr Leu Asp Pro Val Val Leu Phe
        195                 200                 205

Ser Gly Ile Ser Tyr Lys Phe
    210                 215

<210> 5
<211> 1171
<212> DNA
<213> Actinobacillus pleuropneumoniae

<220>
<221> CDS
<222> (157)..(930)

<400> 5
tatttgagct taggctttaa taaagctcga atcctaagcc aggaaatata gaaagtacat 60

taaatataat ttagtattgt attaatagag gataaagcca caaactggca agcaagaatt 120

ggttttactt tttaacctca ctaaaaggag acaact atg aaa cat agc aaa ttc 174
Met Lys His Ser Lys Phe
1               5

aaa tta ttt aaa tat tat tta att agc ttt cct ttt att act ttt gca 222
Lys Leu Phe Lys Tyr Tyr Leu Ile Ser Phe Pro Phe Ile Thr Phe Ala
            10                  15                  20

agt aat gtt aat gga gcc gaa att gga ttg gga gga gcc cgt gag agt 270
Ser Asn Val Asn Gly Ala Glu Ile Gly Leu Gly Gly Ala Arg Glu Ser
        25                  30                  35

agt att tac tat tct aaa cat aaa gta gca aca aat ccc ttt tta gca 318
Ser Ile Tyr Tyr Ser Lys His Lys Val Ala Thr Asn Pro Phe Leu Ala
    40                  45                  50

ctt gat ctt tct tta ggt aat ttt tat atg aga ggg act gca gga att 366
Leu Asp Leu Ser Leu Gly Asn Phe Tyr Met Arg Gly Thr Ala Gly Ile
55                  60                  65                  70

agc gaa ata gga tat gaa caa tct ttc act gac aat ttc agc gta tca 414
Ser Glu Ile Gly Tyr Glu Gln Ser Phe Thr Asp Asn Phe Ser Val Ser
                75                  80                  85

ctg ttt gtt aac cca ttt gat ggt ttt tca att aaa gga aaa gac ttg 462
Leu Phe Val Asn Pro Phe Asp Gly Phe Ser Ile Lys Gly Lys Asp Leu
            90                  95                  100

tta cct gga tat caa agt att caa act cgc aaa act caa ttt gcc ttt 510
Leu Pro Gly Tyr Gln Ser Ile Gln Thr Arg Lys Thr Gln Phe Ala Phe
        105                 110                 115

ggt tgg gga tta aat tat aat ttg gga ggt tta ttc ggc tta aat gat 558
Gly Trp Gly Leu Asn Tyr Asn Leu Gly Gly Leu Phe Gly Leu Asn Asp
    120                 125                 130

act ttt ata tcc ttg gaa gga aaa agc gga aaa cgt ggt gcg agt agt 606
Thr Phe Ile Ser Leu Glu Gly Lys Ser Gly Lys Arg Gly Ala Ser Ser
135                 140                 145                 150

aat gtc agc tta ctt aaa tcg ttt aat atg acg aaa aat tgg aaa gtt 654
Asn Val Ser Leu Leu Lys Ser Phe Asn Met Thr Lys Asn Trp Lys Val
                155                 160                 165

tca cca tat att ggc tca agt tat tat tca tct aaa tat aca gat tat 702
Ser Pro Tyr Ile Gly Ser Ser Tyr Tyr Ser Ser Lys Tyr Thr Asp Tyr
            170                 175                 180

tac ttt ggt att aaa caa tcc gaa tta ggt aat aaa att aca tcc gta 750
Tyr Phe Gly Ile Lys Gln Ser Glu Leu Gly Asn Lys Ile Thr Ser Val
        185                 190                 195

tat aaa cct aaa gca gct tat gca aca cac ata ggt att aat act gat 798
Tyr Lys Pro Lys Ala Ala Tyr Ala Thr His Ile Gly Ile Asn Thr Asp
    200                 205                 210

tat gct ttc acg aac aat ctt ggc atg ggt tta tct gtc ggt tgg aat 846
Tyr Ala Phe Thr Asn Asn Leu Gly Met Gly Leu Ser Val Gly Trp Asn
215                 220                 225                 230

aaa tat tct aaa gaa att aag caa tct cct atc ata aaa cga gac tct 894
Lys Tyr Ser Lys Glu Ile Lys Gln Ser Pro Ile Ile Lys Arg Asp Ser
                235                 240                 245

caa ttt act tca tct ctt agc ctt tat tat aag ttc taaaatagaa 940
Gln Phe Thr Ser Ser Leu Ser Leu Tyr Tyr Lys Phe
            250                 255

tattctaggg agaatactca ttctttatct ttataaagtt aattgtttct ccctgtttct 1000

atattattta gttacttgtt caaaagctac attggttatt ttgtcatttt ataaaagata 1060

ataaggtggt tattttgaaa attaagaaat atattaaata taccctattt actttccttt 1120

taggcatatc atatttatat tttgggggcg aaaacgaaaa ttatcaagag a 1171

<210> 6
<211> 258
<212> PRT
<213> Actinobacillus pleuropneumoniae

<400> 6
Met Lys His Ser Lys Phe Lys Leu Phe Lys Tyr Tyr Leu Ile Ser Phe
1               5                   10                  15

Pro Phe Ile Thr Phe Ala Ser Asn Val Asn Gly Ala Glu Ile Gly Leu
            20                  25                  30

Gly Gly Ala Arg Glu Ser Ser Ile Tyr Tyr Ser Lys His Lys Val Ala
        35                  40                  45

Thr Asn Pro Phe Leu Ala Leu Asp Leu Ser Leu Gly Asn Phe Tyr Met
    50                  55                  60

Arg Gly Thr Ala Gly Ile Ser Glu Ile Gly Tyr Glu Gln Ser Phe Thr
65                  70                  75                  80

Asp Asn Phe Ser Val Ser Leu Phe Val Asn Pro Phe Asp Gly Phe Ser
                85                  90                  95

Ile Lys Gly Lys Asp Leu Leu Pro Gly Tyr Gln Ser Ile Gln Thr Arg
            100                 105                 110

Lys Thr Gln Phe Ala Phe Gly Trp Gly Leu Asn Tyr Asn Leu Gly Gly
        115                 120                 125

Leu Phe Gly Leu Asn Asp Thr Phe Ile Ser Leu Glu Gly Lys Ser Gly
    130                 135                 140

Lys Arg Gly Ala Ser Ser Asn Val Ser Leu Leu Lys Ser Phe Asn Met
145                 150                 155                 160

Thr Lys Asn Trp Lys Val Ser Pro Tyr Ile Gly Ser Ser Tyr Tyr Ser
                165                 170                 175

Ser Lys Tyr Thr Asp Tyr Tyr Phe Gly Ile Lys Gln Ser Glu Leu Gly
            180                 185                 190

Asn Lys Ile Thr Ser Val Tyr Lys Pro Lys Ala Ala Tyr Ala Thr His
        195                 200                 205

Ile Gly Ile Asn Thr Asp Tyr Ala Phe Thr Asn Asn Leu Gly Met Gly
    210                 215                 220

Leu Ser Val Gly Trp Asn Lys Tyr Ser Lys Glu Ile Lys Gln Ser Pro

225                 230                 235                 240

Ile Ile Lys Arg Asp Ser Gln Phe Thr Ser Ser Leu Ser Leu Tyr Tyr
                245                 250                 255

Lys Phe

<210> 7
<211> 1922
<212> DNA
<213> Actinobacillus pleuropneumoniae

<220>
<221> CDS
<222> (614)..(1705)

<400> 7
acggtaacta cttattcttc tcatgttgca acgccaattc ttgcagagaa attaatcccg 60

atgttacaaa aaggcgactt aggggagccg acacctgctg ctgaaatcga caacgtttac 120

ttacgtgata tcaacgatgc aatccgtaac catccggttg aattaatcgg tcaagagtta 180

cgtggttata tgacggatat gaaacgtatt tcatcgcaag gttaattaaa aattaatcaa 240

aagcctactt cgcaagaagt gggctttttg ttattcaagc cgcttaccgc tatcaatggt 300

aagtgatatg cataatagct tataaattat aagttgtttt aagcaaatat atctctatcg 360

gtaagtaaaa aattaattgt agatcattaa aaaacggata aaaaaatcta ttttttgagt 420

gaattacgca taaaaaatga tctaaattat aggtttagtt gtatttttca atttttattt 480

ggtagaatac aactgtaata aaagcttaat tattttgaga tacacataaa ataatttacg 540

gctttattca ttatcccttt taggttaggg atttgtcttt aatagatgac gataaattta 600

gaggatcatc aaa atg aaa aaa tca tta gtt gct tta aca gta tta tcg    649
               Met Lys Lys Ser Leu Val Ala Leu Thr Val Leu Ser
               1               5                   10

gct gca gcg gta gct caa gca gcg cca caa caa aat act ttc tac gca    697
Ala Ala Ala Val Ala Gln Ala Ala Pro Gln Gln Asn Thr Phe Tyr Ala
        15                  20                  25

ggt gcg aaa gca ggt tgg gcg tca ttc cat gat ggt atc gaa caa tta    745
Gly Ala Lys Ala Gly Trp Ala Ser Phe His Asp Gly Ile Glu Gln Leu
    30                  35                  40

gat tca gct aaa aac aca gat cgc ggt aca aaa tac ggt atc aac cgt    793
Asp Ser Ala Lys Asn Thr Asp Arg Gly Thr Lys Tyr Gly Ile Asn Arg
45                  50                  55                  60

aat tca gta act tac ggc gta ttc ggc ggt tac caa att tta aac caa    841
Asn Ser Val Thr Tyr Gly Val Phe Gly Gly Tyr Gln Ile Leu Asn Gln
                65                  70                  75

gac aaa tta ggt tta gcg gct gaa tta ggt tat gac tat ttc ggt cgt    889
Asp Lys Leu Gly Leu Ala Ala Glu Leu Gly Tyr Asp Tyr Phe Gly Arg
            80                  85                  90

gtg cgc ggt tct gaa aaa cca aac ggt aaa gcg gac aag aaa act ttc 937
Val Arg Gly Ser Glu Lys Pro Asn Gly Lys Ala Asp Lys Lys Thr Phe
        95                  100                 105

cgt cac gct gca cac ggt gcg aca atc gca tta aaa cct agc tac gaa 985
Arg His Ala Ala His Gly Ala Thr Ile Ala Leu Lys Pro Ser Tyr Glu
    110                 115                 120

gta tta cct gac tta gac gtt tac ggt aaa gta ggt atc gca tta gta 1033
Val Leu Pro Asp Leu Asp Val Tyr Gly Lys Val Gly Ile Ala Leu Val
125                 130                 135                 140

aac aat aca tat aaa aca ttc aat gca gca caa gag aaa gtg aaa act 1081
Asn Asn Thr Tyr Lys Thr Phe Asn Ala Ala Gln Glu Lys Val Lys Thr
                145                 150                 155

cgt cgt ttc caa agt tct tta att tta ggt gcg ggt gtt gag tac gca 1129
Arg Arg Phe Gln Ser Ser Leu Ile Leu Gly Ala Gly Val Glu Tyr Ala
            160                 165                 170

att ctt cct gaa tta gcg gca cgt gtt gaa tac caa tgg tta aac aac 1177
Ile Leu Pro Glu Leu Ala Ala Arg Val Glu Tyr Gln Trp Leu Asn Asn
        175                 180                 185

gca ggt aaa gca agc tac tct act tta aat cgt atg ggt gca act gac 1225
Ala Gly Lys Ala Ser Tyr Ser Thr Leu Asn Arg Met Gly Ala Thr Asp
    190                 195                 200

tac cgt tcg gat atc agt tcc gta tct gca ggt tta agc tac cgt ttc 1273
Tyr Arg Ser Asp Ile Ser Ser Val Ser Ala Gly Leu Ser Tyr Arg Phe
205                 210                 215                 220

ggt caa ggt gcg gta ccg gtt gca gct ccg gca gtt gaa act aaa aac 1321
Gly Gln Gly Ala Val Pro Val Ala Ala Pro Ala Val Glu Thr Lys Asn
                225                 230                 235

ttc gca ttc agc tct gac gta tta ttc gca ttc ggt aaa tca aac tta 1369
Phe Ala Phe Ser Ser Asp Val Leu Phe Ala Phe Gly Lys Ser Asn Leu
            240                 245                 250

aaa ccg gct gcg gca aca gca tta gat gca atg caa acc gaa atc aat 1417
Lys Pro Ala Ala Ala Thr Ala Leu Asp Ala Met Gln Thr Glu Ile Asn
        255                 260                 265

aac gca ggt tta tca aat gct gcg atc caa gta aac ggt tac acg gac 1465
Asn Ala Gly Leu Ser Asn Ala Ala Ile Gln Val Asn Gly Tyr Thr Asp
    270                 275                 280

cgt atc ggt aaa gaa gct tca aac tta aaa ctt tca caa cgt cgt gcg 1513
Arg Ile Gly Lys Glu Ala Ser Asn Leu Lys Leu Ser Gln Arg Arg Ala
285                 290                 295                 300

gaa aca gta gct aac tac atc gtt tct aaa ggt gct ccg gca gct aac 1561
Glu Thr Val Ala Asn Tyr Ile Val Ser Lys Gly Ala Pro Ala Ala Asn
                305                 310                 315

gta act gca gta ggt tac ggt gaa gca aac cct gta acc ggc gca aca 1609
Val Thr Ala Val Gly Tyr Gly Glu Ala Asn Pro Val Thr Gly Ala Thr
            320                 325                 330

tgt gac aaa gtt aaa ggt cgt aaa gca tta atc gct tgc tta gca ccg 1657
Cys Asp Lys Val Lys Gly Arg Lys Ala Leu Ile Ala Cys Leu Ala Pro
        335                 340                 345

gat cgt cgt gtt gaa gtt caa gtt caa ggt act aaa gaa gta act atg 1705
Asp Arg Arg Val Glu Val Gln Val Gln Gly Thr Lys Glu Val Thr Met
    350                 355                 360

taatttagtt aattttctaa agttaaatta gtaaccctct tgcttattta agcaagaggg 1765

ttattttttt gttccatttt aattagtgct actcttcctg tgtttatatt tgtgtttatg 1825

ataaactctt cataactttt attcacttat agatgaaaat gaaatacagc ttaacccctt 1885

tccatacctt tcatttagcg gcaaatgcaa caaaatc 1922

<210> 8
<211> 364
<212> PRT
<213> Actinobacillus pleuropneumoniae

<400> 8
Met Lys Lys Ser Leu Val Ala Leu Thr Val Leu Ser Ala Ala Ala Val
1               5                   10                  15

Ala Gln Ala Ala Pro Gln Gln Asn Thr Phe Tyr Ala Gly Ala Lys Ala
            20                  25                  30

Gly Trp Ala Ser Phe His Asp Gly Ile Glu Gln Leu Asp Ser Ala Lys
        35                  40                  45

Asn Thr Asp Arg Gly Thr Lys Tyr Gly Ile Asn Arg Asn Ser Val Thr
    50                  55                  60

Tyr Gly Val Phe Gly Gly Tyr Gln Ile Leu Asn Gln Asp Lys Leu Gly
65                  70                  75                  80

Leu Ala Ala Glu Leu Gly Tyr Asp Tyr Phe Gly Arg Val Arg Gly Ser
                85                  90                  95

Glu Lys Pro Asn Gly Lys Ala Asp Lys Lys Thr Phe Arg His Ala Ala
            100                 105                 110

His Gly Ala Thr Ile Ala Leu Lys Pro Ser Tyr Glu Val Leu Pro Asp
        115                 120                 125

Leu Asp Val Tyr Gly Lys Val Gly Ile Ala Leu Val Asn Asn Thr Tyr
    130                 135                 140

Lys Thr Phe Asn Ala Ala Gln Glu Lys Val Lys Thr Arg Arg Phe Gln
145                 150                 155                 160

Ser Ser Leu Ile Leu Gly Ala Gly Val Glu Tyr Ala Ile Leu Pro Glu
                165                 170                 175

Leu Ala Ala Arg Val Glu Tyr Gln Trp Leu Asn Asn Ala Gly Lys Ala
            180                 185                 190

Ser Tyr Ser Thr Leu Asn Arg Met Gly Ala Thr Asp Tyr Arg Ser Asp
        195                 200                 205

Ile Ser Ser Val Ser Ala Gly Leu Ser Tyr Arg Phe Gly Gln Gly Ala
    210                 215                 220

Val Pro Val Ala Ala Pro Ala Val Glu Thr Lys Asn Phe Ala Phe Ser
225                 230                 235                 240

Ser Asp Val Leu Phe Ala Phe Gly Lys Ser Asn Leu Lys Pro Ala Ala
                245                 250                 255

Ala Thr Ala Leu Asp Ala Met Gln Thr Glu Ile Asn Asn Ala Gly Leu
            260                 265                 270

Ser Asn Ala Ala Ile Gln Val Asn Gly Tyr Thr Asp Arg Ile Gly Lys
        275                 280                 285

Glu Ala Ser Asn Leu Lys Leu Ser Gln Arg Arg Ala Glu Thr Val Ala
    290                 295                 300

Asn Tyr Ile Val Ser Lys Gly Ala Pro Ala Ala Asn Val Thr Ala Val
305                 310                 315                 320

Gly Tyr Gly Glu Ala Asn Pro Val Thr Gly Ala Thr Cys Asp Lys Val
                325                 330                 335

Lys Gly Arg Lys Ala Leu Ile Ala Cys Leu Ala Pro Asp Arg Arg Val
            340                 345                 350

Glu Val Gln Val Gln Gly Thr Lys Glu Val Thr Met
        355                 360


<210> 9
<211> 1319
<212> DNA
<213> Actinobacillus pleuropneumoniae

<220>
<221> CDS
<222> (197)..(1303)

<400> 9
atgtaatatt aggactggaa agttcgaaat tacaaattga tattacaaat tgattgtagt 60

tttgcttttt atccttgata attaactctc ttttttctct agtgagacga gagcattaaa 120

tcaaaacttt ggtgccataa gcggtgctga agtgattttg ttttattaat cgatgacaat 180

ttagaggatc atcaaa atg aaa aaa tca tta gtt gct tta gca gta tta tca 232
                  Met Lys Lys Ser Leu Val Ala Leu Ala Val Leu Ser
                  1               5                   10

gct gca gca gta gct caa gca gct cca caa caa aat act ttc tac gca 280
Ala Ala Ala Val Ala Gln Ala Ala Pro Gln Gln Asn Thr Phe Tyr Ala
        15                  20                  25

ggt gcg aaa gtt ggt caa tca tca ttt cac cac ggt gtt aac caa tta 328
Gly Ala Lys Val Gly Gln Ser Ser Phe His His Gly Val Asn Gln Leu
    30                  35                  40

aaa tct ggt cac gat gat cgt tat aat gat aaa aca cgt aag tat ggt 376
Lys Ser Gly His Asp Asp Arg Tyr Asn Asp Lys Thr Arg Lys Tyr Gly
45                  50                  55                  60

atc aac cgt aac tct gta act tac ggt gta ttc ggc ggt tac caa atc 424
Ile Asn Arg Asn Ser Val Thr Tyr Gly Val Phe Gly Gly Tyr Gln Ile
                65                  70                  75

tta aac caa aac aat ttc ggt tta gcg act gaa tta ggt tat gat tac 472
Leu Asn Gln Asn Asn Phe Gly Leu Ala Thr Glu Leu Gly Tyr Asp Tyr
            80                  85                  90

tac ggt cgt gta cgt ggt aac gat ggt gaa ttc cgt gca atg aaa cac 520
Tyr Gly Arg Val Arg Gly Asn Asp Gly Glu Phe Arg Ala Met Lys His
        95                  100                 105

tct gct cac ggt tta aac ttt gcg tta aaa cca agc tac gaa gta tta 568
Ser Ala His Gly Leu Asn Phe Ala Leu Lys Pro Ser Tyr Glu Val Leu
    110                 115                 120

cct gac tta gac gtt tac ggt aaa gta ggt gtt gcg gtt gtt cgt aac 616
Pro Asp Leu Asp Val Tyr Gly Lys Val Gly Val Ala Val Val Arg Asn
125                 130                 135                 140

gac tat aaa tcc tat ggt gca gaa aac act aac gaa cca aca gaa aaa 664
Asp Tyr Lys Ser Tyr Gly Ala Glu Asn Thr Asn Glu Pro Thr Glu Lys
                145                 150                 155

ttc cat aaa tta aaa gca tca act att tta ggt gca ggt gtt gag tac 712
Phe His Lys Leu Lys Ala Ser Thr Ile Leu Gly Ala Gly Val Glu Tyr
            160                 165                 170

gca att ctt cct gaa tta gcg gca cgt gtt gaa tac caa tac tta aac 760
Ala Ile Leu Pro Glu Leu Ala Ala Arg Val Glu Tyr Gln Tyr Leu Asn
        175                 180                 185

aaa gcg ggt aac tta aat aaa gca tta gtt cgt tca ggc aca caa gat 808
Lys Ala Gly Asn Leu Asn Lys Ala Leu Val Arg Ser Gly Thr Gln Asp
    190                 195                 200

gtg gac ttc caa tat gct cct gat atc cac tct gta aca gca ggt tta 856
Val Asp Phe Gln Tyr Ala Pro Asp Ile His Ser Val Thr Ala Gly Leu
205                 210                 215                 220

tca tac cgt ttc ggt caa ggc gct gta gca cca gtt gtt gag cca gaa 904
Ser Tyr Arg Phe Gly Gln Gly Ala Val Ala Pro Val Val Glu Pro Glu
                225                 230                 235

gtt gta act aaa aac ttc gca ttc agc tca gac gtt tta ttt gat ttc 952
Val Val Thr Lys Asn Phe Ala Phe Ser Ser Asp Val Leu Phe Asp Phe
            240                 245                 250

ggt aaa tca agc tta aaa cca gca gca gca aca gct tta gac gca gct     1000
Gly Lys Ser Ser Leu Lys Pro Ala Ala Ala Thr Ala Leu Asp Ala Ala
        255                 260                 265

aac act gaa atc gct aac tta ggt tta gca act cca gct atc caa gtt     1048
Asn Thr Glu Ile Ala Asn Leu Gly Leu Ala Thr Pro Ala Ile Gln Val
    270                 275                 280

aac ggt tat aca gac cgt atc ggt aaa gaa gct tca aac tta aaa ctt     1096
Asn Gly Tyr Thr Asp Arg Ile Gly Lys Glu Ala Ser Asn Leu Lys Leu
285                 290                 295                 300

tca caa cgc cgt gca gaa act gta gct aac tac tta gtt tct aaa ggt     1144
Ser Gln Arg Arg Ala Glu Thr Val Ala Asn Tyr Leu Val Ser Lys Gly
                305                 310                 315

caa aac cct gca aac gta act gca gta ggt tac ggt gaa gca aac cca     1192
Gln Asn Pro Ala Asn Val Thr Ala Val Gly Tyr Gly Glu Ala Asn Pro
            320                 325                 330

gta acc ggc gca aca tgt gac aaa gtt aaa ggt cgt aaa gca tta atc     1240
Val Thr Gly Ala Thr Cys Asp Lys Val Lys Gly Arg Lys Ala Leu Ile
        335                 340                 345

gct tgc tta gca ccg gat cgt cgt gtt gaa gtt caa gta caa ggt gct     1288
Ala Cys Leu Ala Pro Asp Arg Arg Val Glu Val Gln Val Gln Gly Ala
    350                 355                 360

aaa aac gta gct atg taatatagtg ggtttt                               1319
Lys Asn Val Ala Met
365

<210> 10
<211> 369
<212> PRT
<213> Actinobacillus pleuropneumoniae

<400> 10
Met Lys Lys Ser Leu Val Ala Leu Ala Val Leu Ser Ala Ala Ala Val
 1               5                  10                  15

Ala Gln Ala Ala Pro Gln Gln Asn Thr Phe Tyr Ala Gly Ala Lys Val
            20                  25                  30

Gly Gln Ser Ser Phe His His Gly Val Asn Gln Leu Lys Ser Gly His
        35                  40                  45

Asp Asp Arg Tyr Asn Asp Lys Thr Arg Lys Tyr Gly Ile Asn Arg Asn
    50                  55                  60

Ser Val Thr Tyr Gly Val Phe Gly Gly Tyr Gln Ile Leu Asn Gln Asn
65                  70                  75                  80

Asn Phe Gly Leu Ala Thr Glu Leu Gly Tyr Asp Tyr Tyr Gly Arg Val
                85                  90                  95

Arg Gly Asn Asp Gly Glu Phe Arg Ala Met Lys His Ser Ala His Gly
            100                 105                 110

Leu Asn Phe Ala Leu Lys Pro Ser Tyr Glu Val Leu Pro Asp Leu Asp
        115                 120                 125

Val Tyr Gly Lys Val Gly Val Ala Val Val Arg Asn Asp Tyr Lys Ser
    130                 135                 140

Tyr Gly Ala Glu Asn Thr Asn Glu Pro Thr Glu Lys Phe His Lys Leu
145                 150                 155                 160

Lys Ala Ser Thr Ile Leu Gly Ala Gly Val Glu Tyr Ala Ile Leu Pro
                165                 170                 175

Glu Leu Ala Ala Arg Val Glu Tyr Gln Tyr Leu Asn Lys Ala Gly Asn
            180                 185                 190

Leu Asn Lys Ala Leu Val Arg Ser Gly Thr Gln Asp Val Asp Phe Gln
        195                 200                 205

Tyr Ala Pro Asp Ile His Ser Val Thr Ala Gly Leu Ser Tyr Arg Phe
    210                 215                 220

Gly Gln Gly Ala Val Ala Pro Val Val Glu Pro Glu Val Val Thr Lys
225                 230                 235                 240

Asn Phe Ala Phe Ser Ser Asp Val Leu Phe Asp Phe Gly Lys Ser Ser
                245                 250                 255

Leu Lys Pro Ala Ala Ala Thr Ala Leu Asp Ala Ala Asn Thr Glu Ile
            260                 265                 270

Ala Asn Leu Gly Leu Ala Thr Pro Ala Ile Gln Val Asn Gly Tyr Thr
        275                 280                 285

Asp Arg Ile Gly Lys Glu Ala Ser Asn Leu Lys Leu Ser Gln Arg Arg
    290                 295                 300

Ala Glu Thr Val Ala Asn Tyr Leu Val Ser Lys Gly Gln Asn Pro Ala
305                 310                 315                 320

Asn Val Thr Ala Val Gly Tyr Gly Glu Ala Asn Pro Val Thr Gly Ala
                325                 330                 335

Thr Cys Asp Lys Val Lys Gly Arg Lys Ala Leu Ile Ala Cys Leu Ala
            340                 345                 350

Pro Asp Arg Arg Val Glu Val Gln Val Gln Gly Ala Lys Asn Val Ala
        355                 360                 365

Met

<210> 11
<211> 20
<212> PRT
<213> Actinobacillus pleuropneumoniae

<220>
<221> SITE
<222> (18)
<223> Xaa=unknown amino acid

<400> 11
Ala Pro Val Gly Asn Thr Phe Thr Gly Val Lys Val Tyr Val Asp Leu
  1               5                  10                  15

Thr Xaa Val Ala
             20

<210> 12
<211> 35
<212> PRT
<213> Actinobacillus pleuropneumoniae

<220>
<221> SITE
<222> (30)
<223> Xaa=Asn or Val

<400> 12
His Gln Ala Gly Asp Val Ile Phe Arg Ala Gly Ala Ile Gly Val Ile
  1               5                  10                  15

Ala Asn Ser Ser Ser Asp Tyr Gln Thr Gln Ala Asp Val Xaa Leu Asp
             20                  25                  30

Val Asn Asn
         35

<210> 13
<211> 30
<212> PRT
<213> Actinobacillus pleuropneumoniae

<400> 13
Ala Glu Ile Gly Leu Gly Gly Ala Arg Glu Ser Ser Ile Tyr Tyr Ser
  1               5                  10                  15

Lys His Lys Val Ala Thr Asn Pro Phe Leu Ala Leu Asp Leu
             20                  25                  30

<210> 14
<211> 19
<212> PRT
<213> Actinobacillus pleuropneumoniae

<220>
<221> SITE
<222> (2)
<223> Xaa=Asp or Glu

<220>
<221> SITE
<222> (14)
<223> Xaa=unknown amino acid

<220>
<221> SITE
<222> (15)
<223> Xaa=unknown amino acid

<220>
<221> SITE
<222> (17)
<223> Xaa=unknown amino acid

<400> 14
Ala Xaa Pro Glu Asn Thr Phe Tyr Pro Gly Ala Lys Val Xaa Xaa Ser
 1               5                  10                  15

Xaa Phe His



<210> 15
<211> 20
<212> DNA
<213> Artificial Sequence

<220>
<223> Description of Artificial Sequence: Primer

<400> 15
aaaaatttgc gaaaaacgac                                              20


<210> 16
<211> 20
<212> DNA
<213> Artificial Sequence

<220>
<223> Description of Artificial Sequence: Primer

<400> 16
acttctacat tacttgatac                                              20


<210> 17
<211> 20
<212> DNA
<213> Artificial Sequence

<220>
<223> Description of Artificial Sequence: Primer

<400> 17
tccgtatgtc aaagtcgatg                                                    20


<210> 18
<211> 20
<212> DNA
<213> Artificial Sequence

<220>
<223> Description of Artificial Sequence: Primer

<400> 18
taaacaatca accggtcctg                                                    20


<210> 19
<211> 20
<212> DNA
<213> Artificial Sequence

<220>
<223> Description of Artificial Sequence: Primer

<400> 19
ttaccttgta caacaccgac                                                    20


<210> 20
<211> 20
<212> DNA
<213> Artificial Sequence

<220>
<223> Description of Artificial Sequence: Primer

<400> 20
aaaagcagta ttagcggcag                                                    20


<210> 21
<211> 20
<212> DNA
<213> Artificial Sequence

<220>
<223> Description of Artificial Sequence: Primer

<400> 21
ttgatgtgcc attgccgaac                                                    20


<210> 22
<211> 20
<212> DNA
<213> Artificial Sequence

<220>
<223> Description of Artificial Sequence: Primer

<400> 22
gttttaaact ttcagcactg                                                    20


<210> 23
<211> 20
<212> DNA
<213> Artificial Sequence

<220>
<223> Description of Artificial Sequence: Primer

<400> 23
agttcgtcca tacgttggtg                                                    20


<210> 24
<211> 20
<212> DNA
<213> Artificial Sequence

<220>
<223> Description of Artificial Sequence: Primer

<400> 24
aatatatctc tatcggtaag                                                    20


<210> 25
<211> 20
<212> DNA
<213> Artificial Sequence

<220>
<223> Description of Artificial Sequence: Primer

<400> 25
ctaaacctat aatttagatc                                                    20


<210> 26
<211> 20
<212> DNA
<213> Artificial Sequence

<220>
<223> Description of Artificial Sequence: Primer

<400> 26
tgtttccgca cgacgttgtg                                                    20


<210> 27
<211> 20
<212> DNA
<213> Artificial Sequence

<220>
<223> Description of Artificial Sequence: Primer

<400> 27
aagtaaacgg ttacacggac                                                    20

<210> 28
<211> 20
<212> DNA
<213> Artificial Sequence

<220>
<223> Description of Artificial Sequence: Primer

<400> 28
atcaaccgta attcagtaac                                                    20


<210> 29
<211> 20
<212> DNA
<213> Artificial Sequence

<220>
<223> Description of Artificial Sequence: Primer

<400> 29
atagtcataa cctaattcag                                                    20


<210> 30
<211> 20
<212> DNA
<213> Artificial Sequence

<220>
<223> Description of Artificial Sequence: Primer

<400> 30
gtatgggtgc aactgactac                                                    20


<210> 31
<211> 20
<212> DNA
<213> Artificial Sequence

<220>
<223> Description of Artificial Sequence: Primer

<400> 31
aacacgtgcc gctaattcag                                                    20


<210> 32
<211> 20
<212> DNA
<213> Artificial Sequence

<220>
<223> Description of Artificial Sequence: Primer

<400> 32
tgcttgagct accgctgcag                                                    20


<210> 33

<211> 20
<212> DNA
<213> Artificial Sequence

<220>
<223> Description of Artificial Sequence: Primer

<400> 33
gcgaaagttg gtcaatcatc                                                    20


<210> 34
<211> 20
<212> DNA
<213> Artificial Sequence

<220>
<223> Description of Artificial Sequence: Primer

<400> 34
ataacgatca tcgtgaccag                                                    20


<210> 35
<211> 20
<212> DNA
<213> Artificial Sequence

<220>
<223> Description of Artificial Sequence: Primer

<400> 35
tgcgtctaaa gctgttgctg                                                    20


<210> 36
<211> 20
<212> DNA
<213> Artificial Sequence

<220>
<223> Description of Artificial Sequence: Primer

<400> 36
atcaagctta aaaccagcag                                                    20


<210> 37
<211> 20
<212> DNA
<213> Artificial Sequence

<220>
<223> Description of Artificial Sequence: Primer

<400> 37
ctataaatcc tatggtgcag                                                    20


<210> 38
<211> 20
<212> DNA

<213> Artificial Sequence

<220>
<223> Description of Artificial Sequence: Primer

<400> 38
tgcacctaaa atagttgatg                                                20


<210> 39
<211> 34
<212> DNA
<213> Artificial Sequence

<220>
<223> Description of Artificial Sequence: Primer

<400> 39
ggggatccgc dccdgtdggh aatacnttta cngg                                34


<210> 40
<211> 43
<212> DNA
<213> Artificial Sequence

<220>
<223> Description of Artificial Sequence: Primer

<400> 40
ggggatccat ytattatwsw aaacataaag tdgcdacnaa tcc                      43


<210> 41
<211> 30
<212> DNA
<213> Artificial Sequence

<220>
<223> Description of Artificial Sequence: Primer

<400> 41
ggggatccgt accgcgatct gtgtttttag                                     30


<210> 42
<211> 27
<212> DNA
<213> Artificial Sequence

<220>
<223> Description of Artificial Sequence: Primer

<400> 42
ggggatccgg tgctccggca gctaacg                                        27


<210> 43
<211> 35
<212> DNA
<213> Artificial Sequence

<220>
<223> Description of Artificial Sequence: Primer

<400> 43
ggggatccat acttacgtgt tttatcatta taacg                                35


<210> 44
<211> 27
<212> DNA
<213> Artificial Sequence

<220>
<223> Description of Artificial Sequence: Primer

<400> 44
ggggatccgg tcaaaaccct gcaaacg                                         27


<210> 45
<211> 33
<212> DNA
<213> Artificial Sequence

<220>
<223> Description of Artificial Sequence: Primer

<400> 45
tttgcccggg ctcttttatt gatttaagtt act                                  33


<210> 46
<211> 24
<212> DNA
<213> Artificial Sequence

<220>
<223> Description of Artificial Sequence: Primer

<400> 46
gactaacgca ggaccggttg attg                                            24


<210> 47
<211> 31
<212> DNA
<213> Artificial Sequence

<220>
<223> Description of Artificial Sequence: Primer

<400> 47
ggggatccgt gggtgcggaa tatacgcgca g                                    31


<210> 48
<211> 25
<212> DNA
<213> Artificial Sequence

<220>
<223> Description of Artificial Sequence: Primer

<400> 48
caacgtggat ccgaattcaa gcttc                                          25


<210> 49
<211> 24
<212> DNA
<213> Artificial Sequence

<220>
<223> Description of Artificial Sequence: Primer

<400> 49
gtcaccgtaa tccataccgt aatg                                           24


<210> 50
<211> 26
<212> DNA
<213> Artificial Sequence

<220>
<223> Description of Artificial Sequence: Primer

<400> 50
gattgtttac ctttcacacc gtccac                                         26


<210> 51
<211> 27
<212> DNA
<213> Artificial Sequence

<220>
<223> Description of Artificial Sequence: Primer

<400> 51
tcacccggga aaaatatcta gaaacgg                                        27


<210> 52
<211> 28
<212> DNA
<213> Artificial Sequence

<220>
<223> Description of Artificial Sequence: Primer

<400> 52
ccgtggtgag atcaacgcct acgcctac                                       28


<210> 53
<211> 38
<212> DNA
<213> Artificial Sequence

<220>
<223> Description of Artificial Sequence: Primer

<400> 53
cctttcacac cgtccacttt atattttacc gtggtgag                            38

<210> 54
<211> 68
<212> DNA
<213> Artificial Sequence

<220>
<223> Description of Artificial Sequence: Primer

<400> 54
gcgcatatga acaccaccac caccaccacc tctcgtgcac cggtcggaaa tacctttacc 60

ggcgtagg 68


<210> 55
<211> 24
<212> DNA
<213> Artificial Sequence

<220>
<223> Description of Artificial Sequence: Primer

<400> 55
caagactaaa aatgaccggt cgtg 24


<210> 56
<211> 25
<212> DNA
<213> Artificial Sequence

<220>
<223> Description of Artificial Sequence: Primer

<400> 56
tgaatttacg accacgtaaa tgttt 25


<210> 57
<211> 25
<212> DNA
<213> Artificial Sequence

<220>
<223> Description of Artificial Sequence: Primer

<400> 57
ctatgtgaaa gcaaaagcgg attgg 25


<210> 58
<211> 23
<212> DNA
<213> Artificial Sequence

<220>
<223> Description of Artificial Sequence: Primer

<400> 58
ccgtccggtt gtttgactaa cgc 23

<210> 59
<211> 24
<212> DNA
<213> Artificial Sequence

<220>
<223> Description of Artificial Sequence: Primer

<400> 59
tcgaacaagc acaccagccg gatg                                          24


<210> 60
<211> 25
<212> DNA
<213> Artificial Sequence

<220>
<223> Description of Artificial Sequence: Primer

<400> 60
ggcggaatac ggtaactact tattc                                         25


<210> 61
<211> 27
<212> DNA
<213> Artificial Sequence

<220>
<223> Description of Artificial Sequence: Primer

<400> 61
cgcataaaaa atgatctaaa ttatagg                                       27


<210> 62
<211> 24
<212> DNA
<213> Artificial Sequence

<220>
<223> Description of Artificial Sequence: Primer

<400> 62
ggggaattca acgattttgc ttgc                                          24


<210> 63
<211> 26
<212> DNA
<213> Artificial Sequence

<220>
<223> Description of Artificial Sequence: Primer

<400> 63
gaattcttgc tcgtttgaat tagaag                                        26


<210> 64
<211> 25

<212> DNA
<213> Artificial Sequence

<220>
<223> Description of Artificial Sequence: Primer

<400> 64
ggtaattttt atatgagagg gactg                                          25


<210> 65
<211> 25
<212> DNA
<213> Artificial Sequence

<220>
<223> Description of Artificial Sequence: Primer

<400> 65
caaacagtga tacgctgaaa ttgtc                                          25


<210> 66
<211> 25
<212> DNA
<213> Artificial Sequence

<220>
<223> Description of Artificial Sequence: Primer

<400> 66
gcaacacaca taggtattaa tactg                                          25


<210> 67
<211> 66
<212> DNA
<213> Artificial Sequence

<220>
<223> Description of Artificial Sequence: Primer

<400> 67
gcgcatatga acaccaccac caccaccacc tctcgtgccg aaattggatt gggaggagcc 60

cgtgag                                                               66


<210> 68
<211> 28
<212> DNA
<213> Artificial Sequence

<220>
<223> Description of Artificial Sequence: Primer

<400> 68
gtattctctc tagaatattc tattttag                                       28


<210> 69
<211> 27

<212> DNA
<213> Artificial Sequence

<220>
<223> Description of Artificial Sequence: Primer

<400> 69
gccacatgaa gaattattat ttgagct                                          27


<210> 70
<211> 25
<212> DNA
<213> Artificial Sequence

<220>
<223> Description of Artificial Sequence: Primer

<400> 70
acgtgaaaaa taatctcttg ataat                                            25


<210> 71
<211> 60
<212> DNA
<213> Artificial Sequence

<220>
<223> Description of Artificial Sequence: Primer

<400> 71
cgccatatga acaccaccac caccaccacc tctcgtcatc aggcgggaga tgtgattttc 60


<210> 72
<211> 23
<212> DNA
<213> Artificial Sequence

<220>
<223> Description of Artificial Sequence: Primer

<400> 72
caycargcdg ghgatgtdat ytt                                              23


<210> 73
<211> 23
<212> DNA
<213> Artificial Sequence

<220>
<223> Description of Artificial Sequence: Primer

<400> 73
gcdgadccdg araayacdtt yta                                              23


<210> 74
<211> 22
<212> DNA
<213> Artificial Sequence

<220>
<223> Description of Artificial Sequence: Primer

<400> 74
accggtacct atatgttaag tg                                                22


<210> 75
<211> 22
<212> DNA
<213> Artificial Sequence

<220>
<223> Description of Artificial Sequence: Primer

<400> 75
ataggtaccg gttaaaccaa gc                                                22


<210> 76
<211> 23
<212> DNA
<213> Artificial Sequence

<220>
<223> Description of Artificial Sequence: Primer

<400> 76
gttgccgcta ataattcaag acc                                               23


<210> 77
<211> 23
<212> DNA
<213> Artificial Sequence

<220>
<223> Description of Artificial Sequence: Primer

<400> 77
aayacnttyt ayccdggngc naa                                               23


<210> 78
<211> 24
<212> DNA
<213> Artificial Sequence

<220>
<223> Description of Artificial Sequence: Primer

<400> 78
nacdckdckr tcnggngcna rrca                                              24


<210> 79
<211> 21
<212> DNA
<213> Artificial Sequence

<220>
<223> Description of Artificial Sequence: Primer

<400> 79
datytcnacd ckdckrtcng g                                                21

<210> 80
<211> 30
<212> DNA
<213> Artificial Sequence

<220>
<223> Description of Artificial Sequence: Primer

<400> 80
cgctctagag atttttaca acaaaaaggg                                        30

<210> 81
<211> 23
<212> DNA
<213> Artificial Sequence

<220>
<223> Description of Artificial Sequence: Primer

<400> 81
gmnaayacnt tytaygyngg ngc                                              23

<210> 82
<211> 24
<212> DNA
<213> Artificial Sequence

<220>
<223> Description of Artificial Sequence: Primer

<400> 82
aayacnttyt aygynggngc naar                                             24

<210> 83
<211> 23
<212> DNA
<213> Artificial Sequence

<220>
<223> Description of Artificial Sequence: Primer

<400> 83
cargmnaaya cnttytaygy ngg                                              23

<210> 84
<211> 23
<212> DNA
<213> Artificial Sequence

<220>
<223> Description of Artificial Sequence: Primer

<400> 84
gcnccncarg mnaayacntt yta                                              23

<210> 85
<211> 23
<212> DNA
<213> Artificial Sequence

<220>
<223> Description of Artificial Sequence: Primer

<400> 85
cgcgcnccnc argmnaayac ntt                                              23


<210> 86
<211> 29
<212> DNA
<213> Artificial Sequence

<220>
<223> Description of Artificial Sequence: Primer

<400> 86
cggtcgactg atttaagtta ctaaaaccc                                        29


<210> 87
<211> 29
<212> DNA
<213> Artificial Sequence

<220>
<223> Description of Artificial Sequence: Primer

<400> 87
cggtcgacgg gttactaatt taactttag                                        29


<210> 88
<211> 67
<212> DNA
<213> Artificial Sequence

<220>
<223> Description of Artificial Sequence: Primer

<400> 88
gcgtcgacca tatgaacacc accaccacca ccacctctcg tgcgccacaa caaaatactt 60

tytacgc                                                                67


<210> 89
<211> 32
<212> DNA
<213> Artificial Sequence

<220>
<223> Description of Artificial Sequence: Primer

<400> 89
atgaaaaatt taacagtttt agcattagca gg                                    32

<210> 90
<211> 38
<212> DNA
<213> Artificial Sequence

<220>
<223> Description of Artificial Sequence: Primer

<400> 90
atgaaacata gcaaattcaa attatttaaa tattattt                                38


<210> 91
<211> 32
<212> DNA
<213> Artificial Sequence

<220>
<223> Description of Artificial Sequence: Primer

<400> 91
atgaaaaaag cagtattagc ggcagtatta gg                                      32


<210> 92
<211> 36
<212> DNA
<213> Artificial Sequence

<220>
<223> Description of Artificial Sequence: Primer

<400> 92
atgaaaaaat cattagttgc tttaacagta ttatcg                                  36


<210> 93
<211> 36
<212> DNA
<213> Artificial Sequence

<220>
<223> Description of Artificial Sequence: Primer

<400> 93
atgaaaaaat cattagttgc tttagcagta ttatca                                  36


<210> 94
<211> 13
<212> PRT
<213> Artificial Sequence

<220>
<223> Description of Artificial Sequence:N-terminal
      consensus sequence of OmpA-related proteins from
      Pasteurellaceae

<220>
<221> SITE
<222> (4)
<223> Xaa=Ala or Glu

<220>
<221> SITE
<222> (9)
<223> Xaa=Ala or Val

<400> 94
Ala Pro Gln Xaa Asn Thr Phe Tyr Xaa Gly Ala Lys Ala
  1               5                   10


<210> 95
<211> 10
<212> PRT
<213> Artificial Sequence

<220>
<223> Description of Artificial Sequence:C-terminal
      consensus sequence of OmpA-related proteins from
      Pasteurellaceae

<400> 95
Cys Leu Ala Pro Asp Arg Arg Val Glu Ile
  1               5                   10


<210> 96
<211> 10
<212> PRT
<213> Artificial Sequence

<220>
<223> Description of Artificial Sequence:protective
      peptide

<400> 96
Met Asn Thr Thr Thr Thr Thr Thr Ser Arg
  1               5                   10